# EUROPEAN PATENT APPLICATION

(11) **EP 4 614 146 A2**
(43) Date of publication of application: **10.09.2025**
(21) Application number: 25177483.2
(22) Date of filing: 09.08.2022
(51) Int. Cl.: G01N 33/53

(54) **DETECTION OF ANALYTES USING TARGETED EPIGENETIC ASSAYS, PROXIMITY-INDUCED TAGMENTATION, STRAND INVASION, RESTRICTION, OR LIGATION**

(30) Priority: 11.08.2021 US 202163231970 P; 30.09.2021 US 202163250574 P
(62) Divisional of application: 22761331.2
(71) Applicant: ILLUMINA, INC., San Diego, CA 92122 (US)
(72) Inventor: KENNEDY, Andrew, San Diego, 92122 (US); SHULTZABERGER, Sarah, San Diego, 92122 (US); BUSBY, Kayla, San Diego, 92122 (US); BROWN, Colin, San Diego, 92122 (US); PRICE, Andrew, San Diego, 92122 (US); VERMAAS, Eric, San Diego, 92122 (US); PANTOJA, Rigoberto, San Diego, 92122 (US); FEELEY, Matthew, San Diego, 92122 (US); ZOU, Jennifer, San Diego, 92122 (US); LI, Yong, San Diego, 92122 (US); ALMASI, Sepideh, San Diego, 92122 (US); DUTTA, Anindita, San Diego, 92122 (US); ALVAREZ, Michelle, San Diego, 92122 (US)
(74) Representative: Marks & Clerk LLP

(57) **Abstract**

Detecting analytes using proximity-induced tagmentation, strand invasion, restriction, or ligation is provided herein. In some examples, detecting an analyte includes coupling a donor recognition probe to a first portion of the analyte. The donor recognition probe includes a first recognition element specific to the first portion of the analyte, a first oligonucleotide corresponding to the first portion, and a transposase coupled to the first recognition element and the first oligonucleotide. An acceptor recognition probe is coupled to a second portion of the analyte. The acceptor recognition probe includes a second recognition element specific to the second portion of the analyte and a second oligonucleotide coupled to the second recognition element and corresponding to the second portion. The transposase is used to generate a reporter polynucleotide including the first and second oligonucleotides. The analyte is detected based on the reporter including comprising the first and second oligonucleotides.

## Description

### CROSS-REFERENCE TO RELATED APPLICATIONS

This application claims the benefit of the following applications, the entire contents of each of which are incorporated by reference herein:
U.S. Provisional Patent Application No. 63/231,970, filed on August 11, 2021 and entitled "Targeted Epigenetic Assays," and
U.S. Provisional Patent Application No. 63/250,574, filed on September 30, 2021 and entitled "Detection of Analytes Using Proximity-Induced Tagmentation."

### BACKGROUND

The detection of specific nucleic acid sequences present in a biological sample has been used, for example, as a method for identifying and classifying microorganisms, diagnosing infectious diseases, detecting, and characterizing genetic abnormalities, identifying genetic changes associated with cancer, studying genetic susceptibility to diseases, and measuring response to various types of treatment. A common technique for detecting specific nucleic acid sequences in a biological sample is nucleic acid sequencing.

Nucleic acid sequencing methodology has evolved from the chemical degradation methods used by Maxam and Gilbert and the strand elongation methods used by Sanger. Several sequencing methodologies are now in use which allow for the parallel processing of thousands of nucleic acids all on a single chip. Some platforms include bead-based and microarray formats in which silica beads are functionalized with probes depending on the application of such formats in applications including sequencing, genotyping, or gene expression profiling.

Some sequencing systems use fluorescence-based detection, whether for "sequencing-by-synthesis" or for genotyping, in which a given nucleotide is labeled with a fluorescent label, and the nucleotide is identified based on detecting the fluorescence from that label.

There is also an unmet need for methods enabling sensitive characterization of epigenetic changes at targeted DNA loci. Chromatin accessibility (by ATAC-seq) and protein(s) associated with a DNA locus (by ChIP-seq) are examples of epigenetic elements that are difficult to target with existing hybrid capture technology. Commonly, assays that enrich for DNA sequences are associated with an epigenetic feature. However, as these sequences are not known *a priori,* it is challenging to design appropriate hybrid capture oligonucleotides to efficiently enrich the output of the epigenetic assay for a particular genomic region of interest (e.g., a genomic locus).

Prior methods of using deactivated Cas (dCas9) for targeted locus-specific protein isolation to identify histone gene regulators have been presented; see, e.g., Tsui et al., "dCas9-targeted locus-specific protein isolation method identifies histone gene regulators," PNAS 115(2): E2734-E2741 (2018), the entire contents of which are incorporated by reference herein. Such methods demonstrated that dCas9-based locus enrichment can isolate chromatin that can be subsequently assayed by mass spectrometry. However, this method only allows a single chromatin locus to be assayed in each experiment. Furthermore, this prior work provides two separate results, i.e. the sequence of the DNA locus, and mass spectrometry to identify DNA associated proteins. Improved methods for locus-targeted epigenetic analysis are needed.

### SUMMARY

Systems and methods for detecting analytes using targeted epigenetic assays, proximity-induced tagmentation, strand invasion, restriction, or ligation are provided herein.

Some examples herein provide a method for detecting an analyte. The method may include coupling a donor recognition probe to a first portion of the analyte. The donor recognition probe may include a first recognition element specific to the first portion of the analyte, a first oligonucleotide corresponding to the first portion of the analyte, and a transposase coupled to the first recognition element and the first oligonucleotide. The method may include coupling an acceptor recognition probe to a second portion of the analyte. The acceptor recognition probe may include a second recognition element specific to the second portion of the analyte and a second oligonucleotide coupled to the second recognition element and corresponding to the second portion of the analyte. The method may include using the transposase to generate a reporter polynucleotide including the first and second oligonucleotides. The method may include detecting the analyte based on the reporter polynucleotide including the first and second oligonucleotides.

In some examples, the analyte includes a first molecule. In some examples, the first portion of the analyte includes a first portion of the first molecule, and the second portion of the analyte includes a second portion of the first molecule.

In some examples, the first molecule includes a protein or peptide. The first recognition element may include a first antibody or a first aptamer that is specific to a first portion of the protein or peptide. The second recognition element may include a second antibody or a second aptamer that is specific to a second portion of the protein or peptide.

In some examples, the first molecule includes a target polynucleotide. The first recognition element may include a first CRISPR-associated (Cas) protein that is specific to a first subsequence of the target polynucleotide. The second recognition element may include a second Cas protein that is specific to a second subsequence of the target polynucleotide. In some examples, the target polynucleotide includes RNA, and the first and second Cas proteins independently are selected from the group consisting of rCas9 and dCas13.

In some examples, the first molecule includes a carbohydrate. The first recognition element may include a first lectin that is specific to a first portion of the carbohydrate. The second recognition element may include a second lectin that is specific to a second portion of the carbohydrate.

In some examples, the first molecule includes a biomolecule. The biomolecule may be specific for the first and second recognition elements.

In some examples, the analyte further includes a second molecule interacting with the first molecule. In some examples, the first portion of the analyte includes the first molecule, and the second portion of the analyte includes the second molecule.

In some examples, the first molecule may include a first protein or first peptide; and the first recognition element may include a first antibody or a first aptamer that is specific to the first protein or first peptide. Or, for example, the first molecule may include a first target polynucleotide; and the first recognition element may include a first CRISPR-associated (Cas) protein that is specific to the first target polynucleotide. Or, for example, the first molecule may include a first carbohydrate; and the first recognition element may include a first lectin that is specific to the first carbohydrate. Or, for example, the first molecule may include a first biomolecule that is specific for the first recognition element.

It will be appreciated that any suitable second molecules are compatible with any of the aforementioned first molecules. For example, the second molecule may include a second protein or second peptide; and the second recognition element may include a second antibody or a second aptamer that is specific to the second protein or second peptide. Or, the second molecule may include a second target polynucleotide; and the second recognition element may include a second Cas protein that is specific to the second target polynucleotide. Or, the second molecule may include a second carbohydrate; and the second recognition element may include a second lectin that is specific to the second carbohydrate. Or, the second molecule may include a second biomolecule that is specific for the second recognition element.

In some examples, a portion of the second oligonucleotide includes a double-stranded polynucleotide to which the transposase tagments the first oligonucleotide to generate the reporter polynucleotide.

In some examples, the first oligonucleotide includes a first barcode corresponding to the first portion of the analyte, and the second oligonucleotide includes a second barcode corresponding to the second portion of the analyte.

In some examples, the first oligonucleotide includes a mosaic end (ME) transposon end to which the transposase is coupled.

In some examples, the first oligonucleotide has a different sequence than the second oligonucleotide.

In some examples, the first oligonucleotide includes a forward primer, and the second oligonucleotide includes a reverse primer.

In some examples, the method further includes inhibiting activity of the transposase while specifically coupling the donor recognition probe to the first portion of the analyte and while specifically coupling the acceptor recognition probe to the second portion of the analyte. In some examples, the activity of the transposase is inhibited using a first condition of a fluid. In some examples, the first condition of the fluid includes at least one of (i) presence of a sufficient amount of EDTA to inhibit activity of the transposase and (ii) absence of a sufficient amount of magnesium ions for activity of the transposase. In some examples, the activity of the transposase is inhibited using a dsDNA quencher. In some examples, the activity of the transposase is inhibited by associating a blocker with the transposase. In some examples, the activity of the transposase is inhibited by the second oligonucleotide being single stranded. In some examples, the method further includes promoting activity of the transposase before using the transposase to generate the reporter polynucleotide. In some examples, the activity of the transposase is promoted using a second condition of the fluid. In some examples, the second condition of the fluid includes presence of a sufficient amount of magnesium ions for activity of the transposase. In some examples, the activity of the transposase is promoted by degrading the blocker. In some examples, the activity of the transposase is promoted by annealing a third oligonucleotide to the second oligonucleotide to form a double-stranded polynucleotide.

In some examples, detecting the analyte includes sequencing the reporter polynucleotide. In some examples, the sequencing includes performing sequencing-by-synthesis on the reporter polynucleotide.

In some examples, the transposase is coupled to the first recognition element via the first oligonucleotide.

In some examples, the donor recognition probe includes two transposases, two first recognition elements, and two first oligonucleotides, wherein the two transposases form a dimer, each of the transposases being coupled to a corresponding one of the first recognition elements via a corresponding one of the first oligonucleotides.

In some examples, the donor recognition probe includes two transposases, one first recognition element, and two first oligonucleotides. The two transposases may form a dimer, each of the transposases being coupled to the one first recognition element via a corresponding one of the first oligonucleotides.

In some examples, the donor recognition probe includes two transposases, one first recognition element, and two first oligonucleotides. The two transposases may form a dimer, at least one of the transposases being coupled to the one first recognition element via a covalent linkage.

In some examples, the first and second oligonucleotides include DNA.

In some examples, the transposase includes Tn5.

In some examples, the acceptor recognition probe is coupled to a bead before the acceptor recognition probe is coupled to the second portion of the analyte. The method further may include washing the bead after the acceptor recognition probe is coupled to the second portion of the analyte and before the donor recognition probe is coupled to the first portion of the analyte.

In some examples, the first recognition element and the first oligonucleotide are coupled to the first portion of the analyte before the transposase is coupled to the first oligonucleotide and the first recognition element.

Some examples herein provide a method for detecting different analytes in a mixture. The method may include coupling different analytes in a mixture to respective donor recognition probes. Each of the donor recognition probes may include a first recognition element specific to a first portion of the respective analyte, a first oligonucleotide corresponding to the first portion of that analyte, and a transposase coupled to the first recognition element and the first oligonucleotide. The method may include coupling different analytes in the mixture to respective acceptor recognition probes. Each of the acceptor recognition probes may include a second recognition element specific to a second portion of the respective analyte, and a second oligonucleotide corresponding to the second portion of that analyte and coupled to the second recognition element. The method may include, for each of the analytes coupled to the respective donor recognition probe and to the respective acceptor recognition probe, using the transposase of that donor recognition probe to generate a reporter polynucleotide including the first and second oligonucleotides corresponding to that analyte. The method may include detecting the analytes in the mixture based on the reporter polynucleotides including the first and second oligonucleotides corresponding to those analytes.

In some examples, the method further includes determining amounts of the detected analytes in the mixture based on amounts of the reporter polynucleotides corresponding to those analytes.

In some examples, for a first one of the analytes, a first one of the donor recognition probes is specific to a first form of the first portion of that analyte. In some examples, for the first one of the analytes, a second one of the donor recognition probes is specific to a second form of the first portion of that analyte. In some examples, the first and second ones of the donor recognition probes are mixed with the analytes concurrently with one another.

In some examples, for the first one of the analytes, a second one of the donor recognition probes is specific to both the first form and to a second form of the first portion of that analyte. In some examples, the second one of the donor recognition probes is mixed with the analytes after the first one of the donor recognition probes is mixed with the analytes. In some examples, the first form is post-translationally modified (PTM), and the second form is not PTM. In some examples, the first form is phosphorylated, acetylated, methylated, nitrosylated, or glycosylated relative to the second form.

In some examples, the method further includes determining amounts of the first and second forms of the first one of the analytes based on amounts of the reporter polynucleotides corresponding to the first and second ones of the donor recognition probes.

Some examples herein provide a composition. The composition may include an analyte having first and second portions. The composition may include a donor recognition probe coupled to the first portion of the analyte. The donor recognition probe may include a first recognition element specific to the first portion of the analyte, a first oligonucleotide corresponding to the first portion of the analyte, and a transposase coupled to the first recognition element and the first oligonucleotide. The composition may include an acceptor recognition probe coupled to the second portion of the analyte, the acceptor recognition probe including a second recognition element specific to the second portion of the analyte and a second oligonucleotide coupled to the second recognition element and corresponding to the second portion of the analyte.

Some examples herein provide a kit. The kit may include a plurality of donor recognition probes, each including a recognition element specific to a first portion of a respective analyte, a first oligonucleotide corresponding to the first portion of that respective analyte, and a transposase coupled to the first recognition element and the first oligonucleotide. The kit further may include a plurality of acceptor recognition probes, each including a recognition element specific to a second portion of a respective analyte and a second polynucleotide coupled to the second recognition element and corresponding to the second portion of that respective analyte.

Some examples herein provide a method for detecting an analyte. The method may include coupling a first recognition probe to a first portion of the analyte. The first recognition probe may include a first recognition element specific to the first portion of the analyte and a first oligonucleotide corresponding to the first portion of the analyte. The method may include coupling a second recognition probe to a second portion of the analyte. The second recognition probe may include a second recognition element specific for the second portion of the analyte and a second oligonucleotide corresponding to the second portion of the analyte. The method may include coupling the first oligonucleotide to the second oligonucleotide using a splint oligonucleotide that has complementarity to both a portion of the first oligonucleotide and a portion of the second oligonucleotide to form a reporter oligonucleotide coupled to the first and second recognition probes. The method may include performing a sequence analysis of the reporter oligonucleotide. The method may include detecting the analyte based on the sequence analysis of the reporter oligonucleotide.

In some examples, the method further includes generating a double-stranded oligonucleotide including the reporter oligonucleotide coupled to the first and second recognition probes, and a complementary oligonucleotide hybridized to the reporter oligonucleotide. In some examples, the method further includes excising a portion of the double-stranded oligonucleotide, wherein the sequence analysis is performed on the excised portion of the double-stranded oligonucleotide.

In some examples, the sequence analysis that is performed includes any one or more of isothermal bead-based amplification, targeted genome amplification, and whole genome amplification.

In some examples, the first recognition probe or the second recognition probe includes an antibody, a lectin, or an aptamer. In some examples, the first recognition probe includes a first antibody, a first lectin, or a first aptamer. In some examples, the second recognition probe includes a second antibody, a second lectin, or a second aptamer.

In some examples, the first oligonucleotide includes a partial barcode, and the second oligonucleotide comprises a partial barcode, wherein coupling the first oligonucleotide to the second oligonucleotide results in a complete barcode that corresponds to the target analyte.

In some examples, performing the sequence analysis includes performing a polymerase chain reaction (PCR) on the reporter oligonucleotide. In some examples, the reporter oligonucleotide includes a unique molecular identifier (UMI) that is amplified during the PCR.

Some examples herein provide a method for detecting a plurality of analytes in a sample. The method may include incubating the sample with a plurality of pairs of recognition probes. Each pair of recognition probes may include a first recognition probe and a second recognition probe. Each pair of recognition probes may be specific for a respective one of the analytes. Each first recognition probe and each second recognition probe may be coupled to a respective oligonucleotide. The method may include incubating the sample with a plurality of splint oligonucleotides. Each splint oligonucleotide may be complementary to portions of oligonucleotides that respectively are coupled to a first recognition probe and a second recognition probe of a pair of recognition probes which is specific to a respective one of the analytes. Complementary binding of each splint oligonucleotide to oligonucleotides that are coupled to first recognition probes and second recognition probes may result in formation of reporter oligonucleotides. The method may include washing the sample to remove any unbound recognition probes and any unbound splint oligonucleotides. The method may include performing a sequence analysis of the reporter oligonucleotides. The method may include detecting the plurality of analytes based on the sequence analysis.

In some examples, incubating the sample further includes incubation with a ligase.

In some examples, performing the sequence analysis includes using any one or more of a microarray, a bead array, library preparation, or PCR.

Some examples herein provide a composition. The composition may include a plurality of analytes. The composition may include a plurality of pairs of recognition probes. Each pair of recognition probes may include a first recognition probe and a second recognition probe. Each pair of recognition probes may be specific for a respective one of the analytes. Each first recognition probe and each second recognition probed may be coupled to a respective oligonucleotide. The composition may include a plurality of splint oligonucleotides. Each splint oligonucleotide may be complementary to portions of oligonucleotides that respectively are coupled to a first recognition probe and a second recognition probe of a pair of recognition probes which is specific to a respective one of the analytes.

Some examples herein provide a kit. The kit may include a plurality of pairs of recognition probes. Each pair of recognition probes may include a first recognition probe and a second recognition probe. Each pair of recognition probes may be specific for a respective one of the analytes. Each first recognition probe and each second recognition probe may be coupled to a respective oligonucleotide. The kit may include a plurality of splint oligonucleotides. Each splint oligonucleotide may be complementary to portions of oligonucleotides that respectively are coupled to a first recognition probe and a second recognition probe of a pair of recognition probes which is specific to a respective one of the analytes.

Some examples herein provide a method for detecting an analyte. The method may include coupling a first recognition probe to a first portion of the analyte. The first recognition probe may include a first recognition element specific to the first portion of the analyte and a double-stranded oligonucleotide that includes a first barcode corresponding to the first portion of the analyte. The method may include coupling a second recognition probe to a second portion of the analyte. the second recognition probe may include a second recognition element specific for the second portion of the analyte and a single-stranded oligonucleotide that includes a second barcode corresponding to the second portion of the analyte. The method may include hybridizing the single-stranded oligonucleotide with a single oligonucleotide strand of the double-stranded oligonucleotide to form a reporter oligonucleotide that includes the first barcode and the second barcode. The method may include performing a sequence analysis of the reporter oligonucleotide. The method may include detecting the analyte based on the sequence analysis of the reporter oligonucleotide.

In some examples, the hybridizing step includes strand invasion of the double-stranded oligonucleotide by the single-stranded oligonucleotide.

In some examples, the sequence analysis that is performed includes any one or more of isothermal bead-based amplification, targeted genome amplification, and whole genome amplification.

In some examples, detecting the analyte comprises performing quantitative detection of the reporter oligonucleotide.

Some examples herein provide a method for detecting an analyte. The method may include coupling a first recognition probe to a first portion of the analyte, the first recognition probe comprising a first recognition element specific to the first portion of the analyte and a first oligonucleotide corresponding to the first portion of the analyte. The first oligonucleotide may include a first restriction endonuclease site. The method may include coupling a second recognition probe to a second portion of the analyte, the second recognition probe comprising a second recognition element specific for the second portion of the analyte and a second oligonucleotide corresponding to the second portion of the analyte. The second oligonucleotide may include a second restriction endonuclease site. The method may include coupling the first oligonucleotide to the second oligonucleotide. The method may include cutting the first oligonucleotide and the second oligonucleotide at the first and second restriction endonuclease sites to form a reporter oligonucleotide. The method may include performing a sequence analysis of the reporter oligonucleotide. The method may include detecting the analyte based on the sequence analysis of the reporter oligonucleotide.

In some examples, the cutting step comprises using one or more restriction endonucleases.

In some examples, the sequence analysis that is performed includes any one or more of isothermal bead-based amplification, targeted genome amplification, and whole genome amplification.

In some examples, detecting the analyte includes performing quantitative detection of the reporter oligonucleotide.

Some examples herein provide a method of performing a targeted epigenetic assay. The method may include contacting a polynucleotide with a mixture of first complexes that are specific to different types of proteins coupled to respective loci of the polynucleotide. Each of the first complexes may include a first antibody that is specific to a corresponding type of protein, and a first transposome coupled to the first antibody and including a first oligonucleotide corresponding to that type of protein. The method may include respectively coupling the first complexes to proteins for which the first antibodies are specific. The method may include generating fragments of the polynucleotide, including activating the first transposomes to make first cuts in the polynucleotide and to couple the first oligonucleotides to the first cuts. The method may include removing the proteins and first complexes from the fragments. The method may include subsequently sequencing the fragments and the first oligonucleotides coupled thereto. The method may include identifying the proteins that had been coupled to the fragments using the sequences of the first oligonucleotides coupled to those fragments.

In some examples, each of the first complexes includes a plurality of first transposomes. For example, each of the first complexes may include two first transposomes.

Additionally, or alternatively, in some examples, the first transposomes may be deactivated using a first condition of a fluid. In some examples, the first condition of the fluid may include at least one of (i) presence of a sufficient amount of EDTA to inhibit activity of the first transposomes and (ii) absence of a sufficient amount of magnesium ions for activity of the first transposomes. Additionally, or alternatively, in some examples, the first transposomes are activated using a second condition of the fluid. In some examples, the second condition of the fluid may include presence of a sufficient amount of magnesium ions for activity of the first transposomes.

Additionally, or alternatively, in some examples, the sequencing includes performing sequencing-by-synthesis on the fragments and the oligonucleotides coupled thereto.

Additionally, or alternatively, in some examples, the method includes using respective locations in the fragments of the first oligonucleotides to identify the respective loci of the proteins.

Additionally, or alternatively, in some examples, the first oligonucleotides include primers.

Additionally, or alternatively, in some examples, the first oligonucleotides include unique molecular identifiers (UMIs).

Additionally, or alternatively, in some examples, the first oligonucleotides include barcodes corresponding to the proteins.

Additionally, or alternatively, in some examples, the first oligonucleotides include mosaic end (ME) transposon ends.

Additionally, or alternatively, in some examples, the first transposomes are coupled to the first antibodies via covalent linkages.

Additionally, or alternatively, in some examples, the first transposomes are coupled to the first antibodies via non-covalent linkages. For example, the first transposomes may be coupled to protein A, and active sites of the first antibodies may be coupled to the protein A.

Additionally, or alternatively, in some examples, the first transposomes include Tn5.

Additionally, or alternatively, in some examples, each of the first complexes includes a fusion protein including the first antibody and the first transposome.

Additionally, or alternatively, in some examples, the first antibody is coupled to the first oligonucleotide, and wherein the first transposome is coupled to the first antibody via the first oligonucleotide.

Additionally, or alternatively, in some examples, the method further includes contacting the polynucleotide with a mixture of second complexes that are specific to the first complexes. Each of the second complexes may include a second antibody that is specific to the first antibodies, and a second transposome coupled to the second antibody and including a second oligonucleotide. The method may include respectively coupling the second complexes to the first complexes. Generating fragments of the polynucleotide further may include activating the second transposomes to make second cuts in the polynucleotide and to couple the second oligonucleotides to the second cuts. The second oligonucleotides may be used to amplify the fragments prior to sequencing.

Additionally, or alternatively, in some examples, the polynucleotide includes double-stranded DNA.

Some examples herein provide a composition. The composition may include a polynucleotide, having different types of proteins coupled to respective loci thereof. The composition may include a mixture of first complexes that are specific to different types of the proteins. Each of the first complexes may include a first antibody selective for a type of protein, and a first transposome coupled to the first antibody and including a first oligonucleotide corresponding to that type of protein.

**In** some examples, each of the first complexes includes a plurality of first transposomes. For example, each of the first complexes may include two first transposomes.

Additionally, or alternatively, in some examples, the first transposomes are deactivated using a condition of a fluid. For example, the condition of the fluid may include at least one of (i) presence of a sufficient amount of EDTA to inhibit activity of the first transposomes and (ii) absence of a sufficient amount of magnesium ions for activity of the first transposomes.

Additionally, or alternatively, in some examples, the first transposomes are activatible to cut the polynucleotide and add the first oligonucleotides to the cuts. In some examples, the first transposomes are activatible using a condition of a fluid. In some examples, the condition of the fluid may include presence of a sufficient amount of magnesium ions for activity of the first transposomes.

Additionally, or alternatively, in some examples, the first oligonucleotides include primers.

Additionally, or alternatively, in some examples, the first oligonucleotides include unique molecular identifiers (UMIs).

Additionally, or alternatively, in some examples, the first oligonucleotides include barcodes corresponding to the proteins.

Additionally, or alternatively, in some examples, the first oligonucleotides include mosaic end (ME) transposon ends.

Additionally, or alternatively, in some examples, the first transposomes are coupled to the antibodies via covalent linkages.

Additionally, or alternatively, in some examples, the first transposomes are coupled to the antibodies via non-covalent linkages.

Additionally, or alternatively, in some examples, the first transposomes are coupled to protein A, and active sites of the first antibodies are coupled to the protein A.

Additionally, or alternatively, in some examples, the first transposomes include Tn5.

Additionally, or alternatively, in some examples, each of the first complexes includes a fusion protein including the first antibody and the first transposome.

Additionally, or alternatively, in some examples, the first antibody is coupled to the first oligonucleotide, and the first transposome is coupled to the first antibody via the first oligonucleotide.

Additionally, or alternatively, in some examples, the composition further includes a mixture of second complexes that are specific to the first complexes. Each of the second complexes may include a second antibody that is coupled to one of the first antibodies, and a second transposome including a second oligonucleotide.

Additionally, or alternatively, in some examples, the polynucleotide includes double-stranded DNA.

It is to be understood that any respective features/examples of each of the aspects of the disclosure as described herein may be implemented together in any appropriate combination, and that any features/examples from any one or more of these aspects may be implemented together with any of the features of the other aspect(s) as described herein in any appropriate combination to achieve the benefits as described herein.

### BRIEF DESCRIPTION OF THE DRAWINGS

FIG. 1 schematically illustrates example operations and compositions in a process flow for detecting analytes using proximity-induced tagmentation.
FIG. 2 schematically illustrates example donor recognition probes for use in detecting analytes using proximity-induced tagmentation.
FIG. 3 schematically illustrates example acceptor recognition probes for use in detecting analytes using proximity-induced tagmentation.
FIGS. 4A-4G schematically illustrate further details of operations and compositions in the process flow of FIG. 1, according to some examples.
FIG. 5 schematically illustrates example operations and compositions in a process flow for detecting post-translational modifications (PTMs) using donor-recognition probes.
FIG. 6 schematically illustrates example operations and compositions in a process flow for detecting post-translational modifications (PTMs) using a PTM-specific donor-recognition probe and a non-PTM specific donor-recognition probe.
FIGS. 7A-7C schematically illustrate example operations and compositions in a process flow for detecting molecular interactions using proximity-induced tagmentation.
FIGS. 8A-8C schematically illustrate example process flows for preparing donor recognition probes.
FIGS. 9A-9E schematically illustrate example compositions and operations for reducing background tagmentation during proximity-induced tagmentation.
FIGS. 10A-10D schematically illustrate additional example compositions and operations for reducing background tagmentation during proximity-induced tagmentation.
FIGS. 11A-11C schematically illustrate additional example compositions and operations for reducing background tagmentation during proximity-induced tagmentation.
FIG. 12 schematically illustrates example compositions and operations for reducing contaminants during proximity-induced tagmentation.
FIG. 13 illustrates an example flow of operations in a method for detecting analytes using proximity-induced tagmentation.
FIG. 14 schematically illustrates example operations and compositions in a process flow for detecting molecular interactions using proximity-induced tagmentation.
FIGS. 15A-15C schematically illustrate example operations and compositions in a process flow. FIG. 15A illustrates the detection of RNA modifications on a particular RNA target. FIGS. 15B and 15C illustrate the detection of molecular interactions using proximity-induced tagmentation.
FIG. 16 schematically illustrates example operations and compositions in a process flow for detecting nucleic acid modifications using donor-recognition probes that are specific for nucleic acid modifications.
FIG. 17 schematically illustrates example operations and compositions in a process flow for detecting nucleic acid modifications using donor-recognition probes that can detect the modification specifically, and donor-recognition probes that are specific to the target but not specific to the modification.
FIG. 18 schematically illustrates example operations and compositions in a process flow for detecting background tagmentation during proximity-induced tagmentation.
FIG. 19 schematically illustrates example process flows for adding adapters to reporter polynucleotides.
FIGS. 20A and 20B schematically illustrate example operations and compositions for detecting analytes using a bead array.
FIGS. 21A-21B schematically illustrate additional example operations and compositions for detecting analytes using a bead array.
FIG. 22 schematically illustrates additional example operations and compositions for detecting analytes using a bead array.
FIGS. 23A and 23B schematically illustrate example process flows for adding unique molecular identifiers to donor and acceptor recognition probes.
FIGS. 24A-24D schematically illustrate an example process of a proximity induced ligation assay, using a splint oligonucleotide.
FIGS. 25A-25C schematically illustrate examples of ways of differentiating between ligated and un-ligated oligonucleotides.
FIGS. 26A-26C schematically illustrate another example process of a proximity induced ligation assay, using a splint oligonucleotide.
FIGS. 27A-27B illustrate flows of operations in example methods for detecting an analyte using a splint oligonucleotide, according to some examples herein.
FIGS. 28A-28D schematically illustrate an example process of a proximity induced strand invasion assay.
FIG. 29 illustrates a flow of operations in an example method for detecting an analyte using proximity induced strand invasion, according to some examples herein.
FIGS. 30A-30D schematically illustrate an example process of a proximity induced restriction assay.
FIG. 31 illustrates a flow of operations in an example method for detecting an analyte using proximity induced restriction, according to some examples herein.
FIGS. 32A-32C schematically illustrate example operations and compositions for use in whole genome amplification using random-primed, isothermal multiple displacement amplification (MDA).
FIGS. 33A-33C schematically illustrate example synthetic oligonucleotide sequences.
FIG. 33D is a table with the corresponding number of targets synthesized for each probe class.
FIG. 34 schematically illustrates an example synthetic model system that was used to evaluate detection of synthetic oligonucleotides.
FIGS. 35A-35C schematically illustrate an example synthetic model system that was used to evaluate detection of synthetic oligonucleotides.
FIG. 36 illustrates fluorescence measured during use of the example synthetic model system of FIGS. 34 and 35A-35C.
FIG. 37 illustrates the results of additional measurements made during use of the example synthetic model system of FIGS. 34 and 35A-35C.
FIGS. 38A-38E schematically illustrate example compositions and operations in a process flow for targeted epigenetic assays.
FIG. 39A schematically illustrates example oligonucleotides that may be used in the process flow of FIGS. 38A-38E.
FIG. 39B schematically illustrates fragments coupled to example oligonucleotides of FIG. 39A.
FIGS. 40A-40C schematically illustrate further details of a complex such as may be used in the process flow of FIGS. 38A-38E.
FIG. 41 schematically illustrates an example flow of operations for generating complexes respectively including a transposome coupled to an antibody.
FIG. 42 schematically illustrates an example flow of operations for generating complexes respectively including multiple transposomes coupled to an antibody.
FIG. 43 schematically illustrates an operation in which the antibody of one of the complexes of FIG. 5 selectively binds to a protein at a locus of a polynucleotide.
FIG. 44 schematically illustrates an example flow of operations for amplifying a fragment of a polynucleotide following tagmentation by transposomes of a complex.
FIG. 45 schematically illustrates another example flow of operations for generating complexes respectively including a transposome coupled to multiple antibodies.
FIGS. 46A-46B schematically illustrate example flows of operations for generating complexes respectively including a transposome coupled to an antibody.
FIGS. 47A and 47C schematically illustrates an example flow of operations in which proteins at respective loci of a polynucleotide are sequentially bound by antibodies of primary and secondary complexes.
FIG. 47B schematically illustrates example fragments of the polynucleotide of FIG. 47A or 47C following tagmentation.
FIG. 48 illustrates an example flow of operations in a method for targeted epigenetic assays.

### DETAILED DESCRIPTION

Targeted epigenetic assays, proximity-induced tagmentation, strand invasion, restriction, and ligation, and their uses to detect analytes, are provided herein.

For example, the present examples may be used to detect analytes, such as biomolecules, by using analyte recognition elements (e.g., antibodies, aptamers, or lectins) that are specific to respective analytes, to generate reporter polynucleotides having sequences that correspond to those analytes. The reporter polynucleotides then may be sequenced, and from those sequences the respective analytes may be detected. In some examples provided herein, the reporter polynucleotides are generated using a proximity-induced tagmentation reaction between two analyte-bound recognition elements that respectively are coupled to: 1) a donor recognition probe that includes an active barcoded transposome, and 2) an acceptor DNA handle with a second barcode. In other examples provided herein, the reporter polynucleotides are generated using a proximity-induced strand invasion between analyte-bound recognition elements that are respectively coupled to 1) a double-stranded oligonucleotide and 2) a single-stranded oligonucleotide that invades the double-stranded oligonucleotide. In still other examples provided herein, the reporter polynucleotides are generated using a proximity-induced ligation reaction between analyte-bound recognition elements that are respectively coupled to single-stranded oligonucleotides that become coupled to one another when brought into proximity to one another and to a splint oligonucleotide that hybridizes to both of the single-stranded oligonucleotides. In yet other examples provided herein, the reporter polynucleotides are generated using proximity-induced restriction in which analyte-bound recognition elements are respectively coupled to single-stranded oligonucleotides that hybridize to one another when brought into proximity of one another to form a double-stranded oligonucleotide that includes one or more targets for a restriction enzyme, and a restriction enzyme is used to cut the double-stranded oligonucleotide. As will be apparent from the present description, the present approaches provide for highly scalable, multiplexed detection, quantitation, and/or characterization of analytes.

Some of the present examples may use antibody-transposome complexes that selectively couple oligonucleotides to a polynucleotide near loci to which proteins are coupled. Those oligonucleotides then may be sequenced to identify the proteins, and to identify their respective loci, along that polynucleotide. Each of the complexes may include an antibody that selectively couples to a corresponding protein along the polynucleotide, an oligonucleotide, and one or more transposomes that respectively (i) cut the polynucleotide at a location adjacent to (e.g., within about 1-20 base pairs of) that protein and (ii) couples the oligonucleotide to that cut end of the polynucleotide. Each of the oligonucleotides may include a barcode that corresponds to the protein for which the antibody of the respective complex is selective, and also may include a unique molecular identifier (UMI) that corresponds to the particular polynucleotide molecule that is cut. The location at which the oligonucleotide is coupled to the polynucleotide corresponds to the location of the protein. As such, the sequence of the oligonucleotide and the location of the oligonucleotide together may be used to identify the particular protein that was coupled to the particular locus of a particular polynucleotide molecule. The UMI may be used to accurately quantify if there is a lot of overlap in sequence; for example, if the same loci are cut at substantially the same place in 50 separate copies of the polynucleotide (each of which copies has its own UMI), then it can be determined that there were 50 original pieces of the polynucleotide. Such operations may be performed along any desired portion of the polynucleotide, and indeed may be performed on an entire chromosome or even on a whole genome (WG) sample, thus generating a collection of fragment molecules each labeled with an oligonucleotide indicating the protein(s) that were coupled to that particular fragment molecule. The fragments (with oligonucleotides coupled thereto) readily may be sequenced in a multiplexed manner, e.g., using existing commercially available sequencing-by-synthesis systems. The sequences thus obtained may be correlated to the proteins that were coupled to those fragments. As such, the present examples provide a powerful and highly multiplexed platform for assaying which proteins are coupled to which specific loci of any desired polynucleotide or collection of polynucleotides.

Accordingly, it will be appreciated that some examples herein relate to enriching DNA regions (small or large) retaining epigenetic features (e.g., proteins), which are subsequently processed in an epigenetic-NGS assay. This approach enables ultra-deep epigenetic assays, improving resolution of fine epigenetic changes (e.g., as compared to chromatin immunoprecipitation with sequencing (ChIP-seq)) and complex networks (e.g., locus-associated proteomics) which may facilitate a better understanding of epigenetic mechanisms such as may be important for research or clinical development.

First, some terms used herein will be briefly explained. Then, some example compositions and example methods for targeted epigenetic assays, or for using proximity-induced tagmentation, strand invasion, restriction, or ligation will be described.

### Terms

Unless defined otherwise, all technical and scientific terms used herein have the same meaning as is commonly understood by one of ordinary skill in the art. The use of the term "including" as well as other forms, such as "include," "includes," and "included," is not limiting. The use of the term "having" as well as other forms, such as "have," "has," and "had," is not limiting. As used in this specification, whether in a transitional phrase or in the body of the claim, the terms "comprise(s)" and "comprising" are to be interpreted as having an open-ended meaning. That is, the above terms are to be interpreted synonymously with the phrases "having at least" or "including at least." For example, when used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

As used herein, the singular forms "a", "an" and "the" include plural referents unless the content clearly dictates otherwise.

The terms "substantially," "approximately," and "about" used throughout this specification are used to describe and account for small fluctuations, such as due to variations in processing. For example, they may refer to less than or equal to ±10%, such as less than or equal to ±5%, such as less than or equal to ±2%, such as less than or equal to ±1%, such as less than or equal to ±0.5%, such as less than or equal to ±0.2%, such as less than or equal to ±0.1%, such as less than or equal to ±0.05%.

As used herein, terms such as "hybridize" and "hybridization" are intended to mean noncovalently associating a polynucleotides to one another along the lengths of those polynucleotides to form a double-stranded "duplex," a three-stranded "triplex," or higher-order structure. For example, two DNA polynucleotide strands may associate through complementary base pairing to form a duplex. The primary interaction between polynucleotide strands typically is nucleotide base specific, e.g., A:T, A:U, and G:C, by Watson-Crick and Hoogsteen-type hydrogen bonding. Base-stacking and hydrophobic interactions also may contribute to duplex stability. Hybridization conditions may include salt concentrations of less than about 1 M, more usually less than about 500 mM, or less than about 200 mM. A hybridization buffer may include a buffered salt solution such as 5% SSPE or another suitable buffer known in the art. Hybridization temperatures may be as low as 5° C, but are typically greater than 22° C, and more typically greater than about 30° C, and typically in excess of 37° C. The strength of the association between the first and second polynucleotides increases with the complementarity between the sequences of nucleotides within those polynucleotides. The strength of hybridization between polynucleotides may be characterized by a temperature of melting (Tm) at which 50% of the duplexes have polynucleotide strands that disassociate from one another.

As used herein, the term "nucleotide" is intended to mean a molecule that includes a sugar and at least one phosphate group, and in some examples also includes a nucleobase. A nucleotide that lacks a nucleobase may be referred to as "abasic." Nucleotides include deoxyribonucleotides, modified deoxyribonucleotides, ribonucleotides, modified ribonucleotides, peptide nucleotides, modified peptide nucleotides, modified phosphate sugar backbone nucleotides, and mixtures thereof. Examples of nucleotides include adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP), cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (dUMP), deoxyuridine diphosphate (dUDP), and deoxyuridine triphosphate (dUTP).

As used herein, the term "nucleotide" also is intended to encompass any nucleotide analogue which is a type of nucleotide that includes a modified nucleobase, sugar, backbone, and/or phosphate moiety compared to naturally occurring nucleotides. Nucleotide analogues also may be referred to as "modified nucleic acids." Example modified nucleobases include inosine, xathanine, hypoxathanine, isocytosine, isoguanine, 2-aminopurine, 5-methylcytosine, 5-hydroxymethyl cytosine, 2-aminoadenine, 6-methyl adenine, 6-methyl guanine, 2-propyl guanine, 2-propyl adenine, 2-thiouracil, 2-thiothymine, 2-thiocytosine, 15-halouracil, 15-halocytosine, 5-propynyl uracil, 5-propynyl cytosine, 6-azo uracil, 6-azo cytosine, 6-azo thymine, 5-uracil, 4-thiouracil, 8-halo adenine or guanine, 8-amino adenine or guanine, 8-thiol adenine or guanine, 8-thioalkyl adenine or guanine, 8-hydroxyl adenine or guanine, 5-halo substituted uracil or cytosine, 7-methylguanine, 7-methyladenine, 8-azaguanine, 8-azaadenine, 7-deazaguanine, 7-deazaadenine, 3-deazaguanine, 3-deazaadenine or the like. As is known in the art, certain nucleotide analogues cannot become incorporated into a polynucleotide, for example, nucleotide analogues such as adenosine 5'-phosphosulfate. Nucleotides may include any suitable number of phosphates, e.g., three, four, five, six, or more than six phosphates. Nucleotide analogues also include locked nucleic acids (LNA), peptide nucleic acids (PNA), and 5-hydroxylbutynl-2'-deoxyuridine ("super T").

As used herein, the term "polynucleotide" refers to a molecule that includes a sequence of nucleotides that are bonded to one another. A polynucleotide is one nonlimiting example of a polymer. Examples of polynucleotides include deoxyribonucleic acid (DNA), ribonucleic acid (RNA), and analogues thereof such as locked nucleic acids (LNA) and peptide nucleic acids (PNA). A polynucleotide may be a single stranded sequence of nucleotides, such as RNA or single stranded DNA, a double stranded sequence of nucleotides, such as double stranded DNA, or may include a mixture of a single stranded and double stranded sequences of nucleotides. Double stranded DNA (dsDNA) includes genomic DNA, and PCR and amplification products. Single stranded DNA (ssDNA) can be converted to dsDNA and vice-versa. Polynucleotides may include non-naturally occurring DNA, such as enantiomeric DNA, LNA, or PNA. The precise sequence of nucleotides in a polynucleotide may be known or unknown. The following are examples of polynucleotides: a gene or gene fragment (for example, a probe, primer, expressed sequence tag (EST) or serial analysis of gene expression (SAGE) tag), genomic DNA, genomic DNA fragment, exon, intron, messenger RNA (mRNA), transfer RNA, ribosomal RNA, ribozyme, cDNA, recombinant polynucleotide, synthetic polynucleotide, branched polynucleotide, plasmid, vector, isolated DNA of any sequence, isolated RNA of any sequence, nucleic acid probe, primer or amplified copy of any of the foregoing.

As used herein, a "polymerase" is intended to mean an enzyme having an active site that assembles polynucleotides by polymerizing nucleotides into polynucleotides. A polymerase can bind a primed single stranded target polynucleotide, and can sequentially add nucleotides to the growing primer to form a "complementary copy" polynucleotide having a sequence that is complementary to that of the target polynucleotide. Another polymerase, or the same polymerase, then can form a copy of the target nucleotide by forming a complementary copy of that complementary copy polynucleotide. Any of such copies may be referred to herein as "amplicons." DNA polymerases may bind to the target polynucleotide and then move down the target polynucleotide sequentially adding nucleotides to the free hydroxyl group at the 3' end of a growing polynucleotide strand (growing amplicon). DNA polymerases may synthesize complementary DNA molecules from DNA templates and RNA polymerases may synthesize RNA molecules from DNA templates (transcription). Polymerases may use a short RNA or DNA strand (primer), to begin strand growth. Some polymerases may displace the strand upstream of the site where they are adding bases to a chain. Such polymerases may be said to be strand displacing, meaning they have an activity that removes a complementary strand from a template strand being read by the polymerase.

Example polymerases include Bst DNA polymerase, 9° Nm DNA polymerase, Phi29 DNA polymerase, DNA polymerase I (*E. coli*), DNA polymerase I (Large), (Klenow) fragment, Klenow fragment (3'-5' exo-), T4 DNA polymerase, T7 DNA polymerase, Deep VentR^{™} (exo-) DNA polymerase, Deep VentR^{™} DNA polymerase, DyNAzyme^{™} EXT DNA, DyNAzyme^{™} II Hot Start DNA Polymerase, Phusion^{™} High-Fidelity DNA Polymerase, Therminator^{™} DNA Polymerase, Therminator^{™} II DNA Polymerase, VentR^{®} DNA Polymerase, VentR^{®} (exo-) DNA Polymerase, RepliPHI^{™} Phi29 DNA Polymerase, rBst DNA Polymerase, rBst DNA Polymerase (Large), Fragment (IsoTherm^{™} DNA Polymerase), MasterAmp^{™} AmpliTherm^{™}, DNA Polymerase, Taq DNA polymerase, Tth DNA polymerase, Tfl DNA polymerase, Tgo DNA polymerase, SP6 DNA polymerase, Tbr DNA polymerase, DNA polymerase Beta, and ThermoPhi DNA polymerase. In specific, nonlimiting examples, the polymerase is selected from a group consisting of Bst, Bsu, and Phi29. As the polymerase extends the hybridized strand, it can be beneficial to include single-stranded binding protein (SSB). SSB may stabilize the displaced (non-template) strand. Example polymerases having strand displacing activity include, without limitation, the large fragment of Bst (Bacillus stearothermophilus) polymerase, exo-Klenow polymerase or sequencing grade T7 exo-polymerase. Some polymerases degrade the strand in front of them, effectively replacing it with the growing chain behind (5' exonuclease activity). Some polymerases have an activity that degrades the strand behind them (3' exonuclease activity). Some useful polymerases have been modified, either by mutation or otherwise, to reduce or eliminate 3' and/or 5' exonuclease activity.

As used herein, the term "primer" is defined as a polynucleotide to which nucleotides may be added via a free 3' OH group. A primer may include a 3' block inhibiting polymerization until the block is removed. A primer may include a modification at the 5' terminus to allow a coupling reaction or to couple the primer to another moiety. A primer may include one or more moieties, such as 8-oxo-G, which may be cleaved under suitable conditions, such as UV light, chemistry, enzyme, or the like. The primer length may be any suitable number of bases long and may include any suitable combination of natural and non-natural nucleotides. A target polynucleotide may include an "amplification adapter" or, more simply, an "adapter," that hybridizes to (has a sequence that is complementary to) a primer, and may be amplified so as to generate a complementary copy polynucleotide (amplicon) by adding nucleotides to the free 3' OH group of the primer.

As used herein, the term "plurality" is intended to mean a population of two or more different members. Pluralities may range in size from small, medium, large, to very large. The size of small plurality may range, for example, from a few members to tens of members. Medium sized pluralities may range, for example, from tens of members to about 100 members or hundreds of members. Large pluralities may range, for example, from about hundreds of members to about 1000 members, to thousands of members and up to tens of thousands of members. Very large pluralities may range, for example, from tens of thousands of members to about hundreds of thousands, a million, millions, tens of millions and up to or greater than hundreds of millions of members. Therefore, a plurality may range in size from two to well over one hundred million members as well as all sizes, as measured by the number of members, in between and greater than the above example ranges. Example polynucleotide pluralities include, for example, populations of about 1×10⁵ or more, 5×10⁵ or more, or 1×10⁶ or more different polynucleotides. Accordingly, the definition of the term is intended to include all integer values greater than two. An upper limit of a plurality may be set, for example, by the theoretical diversity of polynucleotide sequences in a sample.

As used herein, the term "double-stranded," when used in reference to a polynucleotide, is intended to mean that all or substantially all of the nucleotides in the polynucleotide are hydrogen bonded to respective nucleotides in a complementary polynucleotide. A double-stranded polynucleotide also may be referred to as a "duplex." As used herein, the term "single-stranded," when used in reference to a polynucleotide, means that essentially none of the nucleotides in the polynucleotide are hydrogen bonded to a respective nucleotide in a complementary polynucleotide.

As used herein, the term "target polynucleotide" is intended to mean a polynucleotide that is the object of an analysis or action, and may also be referred to using terms such as "library polynucleotide," "template polynucleotide," or "library template." The analysis or action includes subjecting the polynucleotide to capture, amplification, sequencing and/or other procedure. A target polynucleotide may include nucleotide sequences additional to a target sequence to be analyzed. For example, a target polynucleotide may include one or more adapters, including an amplification adapter that functions as a primer binding site, that flank(s) a target polynucleotide sequence that is to be analyzed. A target polynucleotide hybridized to a capture primer may include nucleotides that extend beyond the 5' or 3' end of the capture oligonucleotide in such a way that not all of the target polynucleotide is amenable to extension. In particular examples, target polynucleotides may have different sequences than one another but may have first and second adapters that are the same as one another. The two adapters that may flank a particular target polynucleotide sequence may have the same sequence as one another, or complementary sequences to one another, or the two adapters may have different sequences. Thus, species in a plurality of target polynucleotides may include regions of known sequence that flank regions of unknown sequence that are to be evaluated by, for example, sequencing (e.g., SBS). In some examples, target polynucleotides carry an amplification adapter at a single end, and such adapter may be located at either the 3' end or the 5' end the target polynucleotide. Target polynucleotides may be used without any adapter, in which case a primer binding sequence may come directly from a sequence found in the target polynucleotide.

The terms "polynucleotide" and "oligonucleotide" are used interchangeably herein. The different terms are not intended to denote any particular difference in size, sequence, or other property unless specifically indicated otherwise. For clarity of description, the terms may be used to distinguish one species of polynucleotide from another when describing a particular method or composition that includes several polynucleotide species.

The terms "sequence" and "subsequence" may in some cases be used interchangeably herein. For example, a sequence may include one or more subsequences therein. Each of such subsequences also may be referred to as a sequence.

As used herein, the term "amplicon," when used in reference to a polynucleotide, is intended to mean a product of copying the polynucleotide, wherein the product has a nucleotide sequence that is substantially the same as, or is substantially complementary to, at least a portion of the nucleotide sequence of the polynucleotide. "Amplification" and "amplifying" refer to the process of making an amplicon of a polynucleotide. A first amplicon of a target polynucleotide may be a complementary copy. Additional amplicons are copies that are created, after generation of the first amplicon, from the target polynucleotide or from the first amplicon. A subsequent amplicon may have a sequence that is substantially complementary to the target polynucleotide or is substantially identical to the target polynucleotide. It will be understood that a small number of mutations (e.g., due to amplification artifacts) of a polynucleotide may occur when generating an amplicon of that polynucleotide.

As used herein, the term "complex" is intended to mean an element that includes two or more elements with different functional properties than one another.

As used herein, the terms "fusion protein" and "chimeric protein" are intended to mean an element that includes two or more polypeptide domains with different functional properties (such as different enzymatic activities) than one another. The domains may be coupled to one another covalently or non-covalently. Fusion proteins may optionally include a third, fourth or fifth or other polypeptide domains operatively linked to one or more other of the polypeptide domains. Fusion proteins may include multiple copies of the same polypeptide domain. Fusion proteins may also or alternatively include one or more mutations in one or more of the polypeptides. A fusion protein may include one or more non-protein elements, such as a polynucleotide and/or a linker that couples the domains to one another. A fusion protein may be formed by combining the gene sequences from different proteins into a single gene that encodes those proteins. In one nonlimiting, purely illustrative example, Tn5 with Protein A is a fusion protein when both domains are expressed together from a single gene.

As used herein, terms such as "CRISPR-Cas system," "Cas-gRNA ribonucleoprotein," and Cas-gRNA RNP refer to an enzyme system including a guide RNA (gRNA) sequence that includes an oligonucleotide sequence that is complementary or substantially complementary to a sequence within a target polynucleotide, and a Cas protein. CRISPR-Cas systems may generally be categorized into three major types which are further subdivided into ten subtypes, based on core element content and sequences; see, e.g., Makarova et al., "Evolution and classification of the CRISPR-Cas systems," Nat Rev Microbiol. 9(6): 467-477 (2011). Cas proteins may have various activities, e.g., nuclease activity. Thus, CRISPR-Cas systems provide mechanisms for targeting a specific sequence (e.g., via the gRNA) as well as certain enzyme activities upon the sequence (e.g., via the Cas protein).

A Type I CRISPR-Cas system may include Cas3 protein with separate helicase and DNase activities. For example, in the Type 1-E system, crRNAs are incorporated into a multisubunit effector complex called Cascade (CRISPR-associated complex for antiviral defense), which binds to the target DNA and triggers degradation by the Cas3 protein; see, e.g., Brouns et al., "Small CRISPR RNAs guide antiviral defense in prokaryotes," Science 321(5891): 960-964 (2008); Sinkunas et al., "Cas3 is a single-stranded DNA nuclease and ATP-dependent helicase in the CRISPR-Cas immune system," EMBO J 30:1335-1342 (2011); and Beloglazova et al., "Structure and activity of the Cas3 HD nuclease MJ0384, an effector enzyme of the CRISPR interference, EMBO J 30:4616-4627 (2011). Type II CRISPR-Cas systems include the signature Cas9 protein, a single protein (about 160 KDa) capable of generating crRNA and cleaving the target DNA. The Cas9 protein typically includes two nuclease domains, a RuvC-like nuclease domain near the amino terminus and the HNH (or McrA-like) nuclease domain near the middle of the protein. Each nuclease domain of the Cas9 protein is specialized for cutting one strand of the double helix; see, e.g., Jinek et al., "A programmable dual-RNA-guided DNA endonuclease in adaptive bacterial immunity, Science 337(6096): 816-821 (2012). Type III CRISPR-Cas systems include polymerase and RAMP modules. Type III systems can be further divided into sub-types III-A and III-B. Type III-A CRISPR-Cas systems have been shown to target plasmids, and the polymerase-like proteins of Type III-A systems are involved in the cleavage of target DNA; see, e.g., Marraffini et al., "CRISPR interference limits horizontal gene transfer in Staphylococci by targeting DNA," Science 322(5909):1843-1845 (2008). Type III-B CRISPR-Cas systems have also been shown to target RNA; see, e.g., Hale et al., "RNA-guided RNA cleavage by a CRISPR-RNA-Cas protein complex," Cell 139(5): 945-956 (2009). CRISPR-Cas systems include engineered and/or programmed nuclease systems derived from naturally accruing CRISPR-Cas systems. CRISPR-Cas systems may include engineered and/or mutated Cas proteins. CRISPR-Cas systems may include engineered and/or programmed guide RNA.

In some specific examples, the Cas protein in one of the present Cas-gRNA RNPs may include Cas9 or other suitable Cas that may cut the target polynucleotide at the sequence to which the gRNA is complementary, in a manner such as described in the following references, the entire contents of each of which are incorporated by reference herein: Nachmanson et al., "Targeted genome fragmentation with CRISPR/Cas9 enables fast and efficient enrichment of small genomic regions and ultra-accurate sequencing with low DNA input (CRISPR-DS)," Genome Res. 28(10): 1589-1599 (2018); Vakulskas et al., "A high-fidelity Cas9 mutant delivered as a ribonucleoprotein complex enables efficient gene editing in human hematopoietic stem and progenitor cells," Nature Medicine 24: 1216-1224 (2018); Chatterjee et al., "Minimal PAM specificity of a highly similar SpCas9 ortholog," Science Advances 4(10): eaau0766, 1-10 (2018); Lee et al., "CRISPR-Cap: multiplexed double-stranded DNA enrichment based on the CRISPR system," Nucleic Acids Research 47(1): 1-13 (2019). Isolated Cas9-crRNA complex from the *S. thermophilus* CRISPR-Cas system as well as complex assembled in vitro from separate components demonstrate that it binds to both synthetic oligodeoxynucleotide and plasmid DNA bearing a nucleotide sequence complementary to the crRNA. It has been shown that Cas9 has two nuclease domains-RuvC- and HNH-active sites/nuclease domains, and these two nuclease domains are responsible for the cleavage of opposite DNA strands. In some examples, the Cas9 protein is derived from Cas9 protein of *S. thermophilus* CRISPR-Cas system. In some examples, the Cas9 protein is a multi-domain protein having about 1,409 amino acids residues. Some Cas9 proteins may be used to target single-stranded DNA in a manner such as described in Ma et al., "Single-stranded DNA Cleavage by Divergent CRISPR-Cas9 Enzymes," Molecular Cell 60(3): 398-407 (2016), the entire contents of which are incorporated by reference herein.

In other examples, the Cas may be engineered so as not to cut the target polynucleotide at the sequence to which the gRNA is complementary, e.g., in a manner such as described in the following references, the entire contents of each of which are incorporated by reference herein: Guilinger et al., "Fusion of catalytically inactive Cas9 to Fokl nuclease improves the specificity of genome modification," Nature Biotechnology 32: 577-582 (2014); Bhatt et al., "Targeted DNA transposition using a dCas9-transposase fusion protein," https://doi.org/10.1101/571653, pages 1-89 (2019); Xu et al., "CRISPR-assisted targeted enrichment-sequencing (CATE-seq)," available at URL www.biorxiv.org/content/10.1101/672816v1, 1-30 (2019); and Tijan et al., "dCas9-targeted locus-specific protein isolation method identifies histone gene regulators," PNAS 115(12): E2734-E2741 (2018). Cas that lacks nuclease activity may be referred to as deactivated Cas (dCas). In some examples, the dCas may include a nuclease-null variant of the Cas9 protein, in which both RuvC- and HNH-active sites/nuclease domains are mutated. A nuclease-null variant of the Cas9 protein (dCas9) binds to double-stranded DNA, but does not cleave the DNA. Another variant of the Cas9 protein has two inactivated nuclease domains with a first mutation in the domain that cleaves the strand complementary to the crRNA and a second mutation in the domain that cleaves the strand non-complementary to the crRNA. In some examples, the Cas9 protein has a first mutation D10A and a second mutation H840A. In examples in which the target polynucleotide is RNA, dCas13 or rCas9, which lack nuclease activity, may be used to bind the target polynucleotide at the sequence to which the gRNA is complementary. For further details regarding dCas13, see Yang et al., "Dynamic imaging of RNA in living cells by CRISPR-Cas13 systems," Molecular Cell 76(6): P981-997.E7 (2019), the entire contents of which are incorporated by reference herein. For further details regarding rCas9, see Nelles et al., "Programmable RNA tracking in live cells with CRISPR/Cas9," Cell 165: 488-496 (2016), the entire contents of which are incorporated by reference herein.

In still other examples, the Cas protein includes a Cascade protein. Cascade complex in *E. coli* recognizes double-stranded DNA (dsDNA) targets in a sequence-specific manner. *E. coli* Cascade complex is a 405-kDa complex including five functionally essential CRISPR-associated (Cas) proteins (CasA1B2C6D1E1, also called Cascade protein) and a 61-nucleotide crRNA. The crRNA guides Cascade complex to dsDNA target sequences by forming base pairs with the complementary DNA strand while displacing the noncomplementary strand to form an R-loop. Cascade recognizes target DNA without consuming ATP, which suggests that continuous invader DNA surveillance takes place without energy investment; see, e.g., Matthijs et al., "Structural basis for CRISPR RNA-guided DNA recognition by Cascade," Nature Structural & Molecular Biology 18(5): 529-536 (2011). In still other examples, the Cas protein includes a Cas3 protein. Illustratively, *E*. *coli* Cas3 may catalyze ATP-independent annealing of RNA with DNA forming R-loops, and hybrid of RNA base-paired into duplex DNA. Cas3 protein may use gRNA that is longer than that for Cas9; see, e.g., Howard et al., "Helicase disassociation and annealing of RNA-DNA hybrids by Escherichia coli Cas3 protein," Biochem J. 439(1): 85-95 (2011). Such longer gRNA may permit easier access of other elements to the target DNA, e.g., access of a primer to be extended by polymerase. Another feature provided by Cas3 protein is that Cas3 protein does not require a PAM sequence as may Cas9, and thus provides more flexibility for targeting desired sequence. R-loop formation by Cas3 may utilize magnesium as a co-factor; see, e.g., Howard et al., "Helicase disassociation and annealing of RNA-DNA hybrids by Escherichia coli Cas3 protein," Biochem J. 439(1): 85-95 (2011). Cas9 variants also have been developed that reduce or avoid the need for PAM sequences; see, e.g., Walton et al., "Unconstrained genome targeting with near-PAMless engineered CRISPR-Cas9 variants," Science 368(6488): 290-296 (2020), the entire contents of which are incorporated by reference herein. It will be appreciated that any suitable cofactors, such as cations, may be used together with the Cas proteins used in the present compositions and methods.

It also should be appreciated that any CRISPR-Cas systems capable of disrupting the double stranded polynucleotide and creating a loop structure may be used. For example, the Cas proteins may include, but not limited to, Cas proteins such as described in the following references, the entire contents of each of which are incorporated by reference herein: Haft et al., "A guild of 45 CRISPR-associated (Cas) protein families and multiple CRISPR/Cas subtypes exist in prokaryotic genomes," PLoS Comput Biol. 1(6): e60, 1-10 (2005); Zhang et al., "Expanding the catalog of cas genes with metagenomes," Nucl. Acids Res. 42(4): 2448-2459 (2013); and Strecker et al., "RNA-guided DNA insertion with CRISPR-associated transposases," Science 365(6448): 48-53 (2019) in which the Cas protein may include Cas12k. Some these CRISPR-Cas systems may utilize a specific sequence to recognize and bind to the target sequence. For example, Cas9 may utilize the presence of a 5'-NGG protospacer-adjacent motif (PAM).

CRISPR-Cas systems may also include engineered and/or programmed guide RNA (gRNA). As used herein, the terms "guide RNA" and "gRNA" (and sometimes referred to in the art as single guide RNA, or sgRNA) is intended to mean RNA including a sequence that is complementary or substantially complementary to a region of a target DNA sequence and that guides a Cas protein to that region. A guide RNA may include nucleotide sequences in addition to that which is complementary or substantially complementary to the region of a target DNA sequence. Methods for designing gRNA are well known in the art, and nonlimiting examples are provided in the following references, the entire contents of each of which are incorporated by reference herein: Stevens et al., "A novel CRISPR/Cas9 associated technology for sequence-specific nucleic acid enrichment," PLoS ONE 14(4): e0215441, pages 1-7 (2019); Fu et al., "Improving CRISPR-Cas nuclease specificity using truncated guide RNAs, Nature Biotechnology 32(3): 279-284 (2014); Kocak et al., "Increasing the specificity of CRISPR systems with engineered RNA secondary structures," Nature Biotechnology 37: 657-666 (2019); Lee et al., "CRISPR-Cap: multiplexed double-stranded DNA enrichment based on the CRISPR system," Nucleic Acids Research 47(1): e1, 1-13 (2019); Quan et al., "FLASH: a next-generation CRISPR diagnostic for multiplexed detection of antimicrobial resistance sequences," Nucleic Acids Research 47(14): e83, 1-9 (2019); and Xu et al., "CRISPR-assisted targeted enrichment-sequencing (CATE-seq)," https://doi.org/10.1101/672816, 1-30 (2019).

In some examples, gRNA includes a chimera, e.g., CRISPR RNA (crRNA) fused to trans-activating CRISPR RNA (tracrRNA). Such a chimeric single-guided RNA (sgRNA) is described in Jinek et al., "A programmable dual-RNA-guided endonuclease in adaptive bacterial immunity," Science 337 (6096): 816-821 (2012). The Cas protein may be directed by a chimeric sgRNA to any genomic locus followed by a 5'-NGG protospacer-adjacent motif (PAM). In one nonlimiting example, crRNA and tracrRNA may be synthesized by *in vitro* transcription, using a synthetic double stranded DNA template including the T7 promoter. The tracrRNA may have a fixed sequence, whereas the target sequence may dictate part of the crRNA's sequence. Equal molarities of crRNA and tracrRNA may be mixed and heated at 55° C for 30 seconds. Cas9 may be added at the same molarity at 37° C and incubated for 10 minutes with the RNA mix. A 10-20 fold molar excess of the resulting Cas9-gRNA RNP then may be added to the target DNA. The binding reaction may occur within 15 minutes. Other suitable reaction conditions readily may be used.

As used herein, the term "transposase" is intended to mean an enzyme that, under certain conditions, is capable of coupling an oligonucleotide to a double-stranded polynucleotide. The oligonucleotide includes at least a mosaic end (ME) sequence, which also may be referred to as a transposition end (TE). A "transposome" or "transposition system" is intended to refer to a transposase that is coupled to a respective oligonucleotide including at least an ME sequence. For example, the combination of a transposase and transposon end may be referred to as a "transposome." A transposome may be activated, under certain conditions, to cut a double-stranded polynucleotide and to couple the oligonucleotide to the cut end. For example, the transposome and the double-stranded polynucleotide may form a "transposition complex" wherein the transposome inserts the oligonucleotide into the double-stranded polynucleotide. In some examples, a transposome may perform a process that may be referred to as "tagmentation" or "transposition" that results in fragmentation of the target polynucleotide and ligation of adapters to the 5' end of both strands of double-stranded DNA fragments, or to the 5' and 3' ends, e.g., in a manner such as described in U.S. 2010/0120098 or in WO 2010/048605, the entire contents of each of which are incorporated by reference herein.

One nonlimiting example of a transposase is Tn5. Another nonlimiting example of a transposase is Tn3. Another nonlimiting example of a transposase is Mu. In still further examples, transposases may include integrases from retrotransposons or retroviruses. Other examples of known transposition complexes (or components thereof) that may be used in the present methods include, but are not limited to, *Staphylococcus aureus* Tn552, Tyl, Transposon Tn7, Tn/O and IS10, Mariner transposase, Tel, P Element, Tn3, bacterial insertion sequences, retroviruses, and retrotransposon of yeast (see, e.g., Colegio et al., 2001, J. Bacteriol. 183: 2384-8; Kirby et al., 2002, Mol. Microbiol. 43: 173-86; Devine and Boeke, 1994, Nucleic Acids Res., 22: 3765-72; International Patent Application No. WO 95/23875; Craig, 1996, Science 271: 1512; Craig, 1996, Review in: Curr Top Microbiol Immunol. 204: 27-48; Kleckner et al., 1996, Curr Top Microbiol Immunol. 204: 49-82; Lampe et al., 1996, EMBO J. 15: 5470-9; Plasterk, 1996, Curr Top Microbiol Immunol 204: 125-43; Gloor, 2004, Methods Mol. Biol. 260: 97-114; Ichikawa and Ohtsubo, 1990, J Biol. Chem. 265: 18829-32; Ohtsubo and Sekine, 1996, Curr. Top. Microbiol. Immunol. 204: 1-26; Brown et al., 1989, Proc Natl Acad Sci USA 86: 2525-9; and Boeke and Corces, 1989, Annu Rev Microbiol. 43: 403-34). Still other example transposition systems include, but are not limited to, those formed by a hyperactive Tn5 transposase and a Tn5-type transposon end or by a MuA transposase and a Mu transposon end including R1 and R2 end sequences; see, e.g., the following references, the entire contents of each of which are incorporated by reference herein: Goryshin et al., "Tn5 in vitro transposition," J. Biol. Chem. 273: 7367-7394 (1998); Mizuuchi, "In vitro transposition of bacteriophage Mu: a biochemical approach to a novel replication reaction," Cell 35(3 pt 2): 785-794 (1983); and Savilahti et al., "The phage Mu transposomes core: DNA requirements for assembly and function," EMBO J. 14(19): 4893-4903 (1995). Transposases may be mutated to modulate their activity and/or the ME sequence may be changed to modulate the transposome' s activity in a manner such as described in Reznikoff, "Tn5 as a model for understanding DNA transposition," Mol. Microbiol. 47(5): 1199-1206 (2003), the entire contents of which are incorporated by reference herein.

Still further examples of transposases and other suitable transposition systems include *Staphylococcus aureus* Tn552 (see, e.g., Colegio et al., "In vitro transposition system for efficient generation of random mutants of Campylobacter jejuni," J Bacteriol. 183: 2384-2388 (2001) and Kirby et al., "Cryptic plasmids of Mycobacterium avium: Tn552 to the rescue," Mol Microbiol., 43(1): 173-186 (2002)); TyI (Devine et al., "Efficient integration of artificial transposons into plasmid targets in vitro: a useful tool for DNA mapping, sequencing and genetic analysis," Nucleic Acids Res. 22(18): 3765-3772 (1994) and International Patent Application No. WO 95/23875); Transposon Tn7 (Craig, "V(D)J recombination and transposition: Closer than expected," Science 271(5255): 1512 (1996) and Craig, Review in: Curr Top Microbiol Immunol, 204: 27-48 (1996)); TnIO and ISlO (Kleckner et al., Curr Top Microbiol Immunol, 204: 49-82 (1996)); Mariner transposase (Lampe et al., "A purified mariner transposase is sufficient to mediate transposition in vitro," EMBO J. 15(19): 5470-5479 (1996)); Tci (Plasterk, Curr Top Microbiol Immunol, 204: 125-143 (1996)), P Element (Gloor, "Gene targeting in Drosophila," Methods Mol Biol 260: 97-114 (2004)); TnJ (Ichikawa et al., "In vitro transposition of transposon Tn3," J Biol Chem. 265(31): 18829-18832 (1990)); bacterial insertion sequences (Ohtsubo et al., "Bacterial insertion sequences," Curr. Top. Microbiol. Immunol. 204:1-26 (1996)); retroviruses (Brown et al., "Retroviral integration: Structure of the initial covalent product and its precursor, and a role for the viral IN protein," Proc Natl Acad Sci USA, 86: 2525-2529 (1989)); and retrotransposon of yeast (Boeke et al., "Transcription and reverse transcription of retrotransposons," Annu Rev Microbiol. 43: 403-434 (1989). Transposases, transposomes, ME sequences, transposons and transposition systems and complexes are generally known to those of skill in the art, as exemplified by the disclosure of US 2010/0120098, the entire contents of which are incorporated by reference herein.

Some transposomes may include transposase monomers. For example, a single unit (monomeric) Tn3 transposase may bind two target sequences simultaneously and change conformation to form the transposome, e.g., in a manner such as described in Nicolas et al., "Unlocking Tn3-family transposase activity in vitro unveils an asymetric pathway for transposome assembly," PMAS 114(5): E669-E678 (2017), the entire contents of which are incorporated by reference herein. Some transposomes may include transposase dimers. For example, Tn5 transposases may dimerize in a manner such as described in Naumann et al., "Trans catalysis in Tn5 transposition," PNAS 97(16): 8944-8949 (2000), the entire contents of which are incorporated by reference herein. Some transposomes may include transposase tetramers. For example, Mu transposases may form tetramers in a manner such as described in Harshey, "Transposable phase Mu," Microbiol Spectr. 2(5): MDNA3-0007-2014 doi:10.1128/microbiolspec.MDNA3-0007-2014 (22 pages) (2014), and in Lamberg et al., "Efficient insertion mutagenesis strategy for bacterial genomes involving electroporation of in vitro-assembled DNA transposition complexes of bacteriophage Mu," Appl Environ Microbiol. 68(2): 705-712 (2002), the entire contents of each of which are incorporated by reference herein.

In the context of a polypeptide, the terms "variant" and "derivative" as used herein refer to a polypeptide that includes an amino acid sequence of a polypeptide or a fragment of a polypeptide, which has been altered by the introduction of amino acid residue substitutions, deletions, or additions. A variant or a derivative of a polypeptide can be a fusion protein which contains part of the amino acid sequence of a polypeptide. The term "variant" or "derivative" as used herein also refers to a polypeptide or a fragment of a polypeptide, which has been chemically modified, e.g., by the covalent attachment of any type of molecule to the polypeptide. For example, but not by way of limitation, a polypeptide or a fragment of a polypeptide can be chemically modified, e.g., by glycosylation, acetylation, pegylation, phosphorylation, methylation, nitrosylation, amidation, derivatization by known protecting/blocking groups, proteolytic cleavage, linkage to a cellular ligand or other protein, etc. The variants or derivatives are modified in a manner that is different from naturally occurring or starting peptide or polypeptides, either in the type or location of the molecules attached. Variants or derivatives further include deletion of one or more chemical groups which are naturally present on the peptide or polypeptide. A variant or a derivative of a polypeptide or a fragment of a polypeptide can be chemically modified by chemical modifications using techniques known to those of skill in the art, including, but not limited to specific chemical cleavage, acetylation, formulation, metabolic synthesis of tunicamycin, etc. Further, a variant or a derivative of a polypeptide or a fragment of a polypeptide can contain one or more non-classical amino acids. A polypeptide variant or derivative may possess a similar or identical function as a polypeptide, or a fragment of a polypeptide described herein. A polypeptide variant or derivative may possess an additional or different function compared with a polypeptide or a fragment of a polypeptide described herein.

As used herein, the term "sequencing" is intended to mean determining the sequence of a polynucleotide. Sequencing may include one or more of sequencing-by-synthesis, bridge PCR, chain termination sequencing, sequencing by hybridization, nanopore sequencing, and sequencing by ligation.

As used herein, to be "selective" for an element is intended to mean to couple to that target and not to couple to a different element. For example, an antibody that is selective for a protein may couple to that protein and not to a different protein.

As used herein, the terms "unique molecular identifier" and "UMI" are intended to mean an oligonucleotide that may be coupled to a polynucleotide and via which the polynucleotide may be identified. For example, a set of different UMIs may be coupled to a plurality of different polynucleotides, and each of those polynucleotides may be identified using the particular UMI coupled to that polynucleotide. One example of a UMI is a "barcode".

As used herein, the term "whole genome" or "WG" of a species is intended to mean a set of one or more polynucleotides that, together, provide the majority of polynucleotides used by the cellular processes of that species. The whole genome of a species may include any suitable combination of the species' chromosomal DNA and/or mitochondrial DNA, and in the case of a plant species may include the DNA contained in the chloroplast. The set of one or more polynucleotides together may provide at least about 50%, or at least about 60%, or at least about 70%, or at least about 80%, or at least about 90%, or at least about 95%, or at least about 98%, or at least about 99% of the polynucleotides used by the cellular processes of that species.

As used herein, the term "fragment" is intended to mean a portion of a polynucleotide. For example, a polynucleotide may be a total number of bases long, and a fragment of that polynucleotide may be less than the total number of bases long.

As used herein, the term "sample" is intended to mean a volume of fluid that includes one or more polynucleotides. The polynucleotide(s) in sample may include a whole genome, or may include only a portion of a whole genome. A sample may include polynucleotides from a single species, or from multiple species.

The term "antibody" as used herein encompasses monoclonal antibodies (including full length monoclonal antibodies), polyclonal antibodies, multi-specific antibodies (e.g., bi-specific antibodies), and antibody fragments so long as they exhibit the desired biological activity of binding to a target antigenic site and its isoforms of interest. For example, an antibody may selectively bind to a target protein, such as a protein at a locus of a polynucleotide, and may not bind to any other target proteins. As another example, a first antibody may selectively bind to a portion of a second antibody. A set of different antibodies also may include that portion, and as such, the first antibody may selectively bond to that portion of each of those antibodies, and may not bind to any other portions of those antibodies or to any other proteins. The term "antibody fragments" include a portion of a full-length antibody, generally the antigen binding or variable region thereof. The term "antibody" as used herein encompasses any antibodies derived from any species and resources, including but not limited to, human antibody, rat antibody, mouse antibody, rabbit antibody, and so on, and can be synthetically made or naturally occurring.

The term "monoclonal antibody" as used herein refers to an antibody obtained from a population of substantially homogeneous antibodies. That is, the individual antibodies including the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The "monoclonal antibodies" may also be isolated from phage antibody libraries using the techniques known in the art. Monoclonal antibodies, as the term is used herein, may include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity.

As used herein, terms such as "target specific" and "selective," when used in reference to a polynucleotide, are intended to mean a polynucleotide that includes a sequence that is specific to (substantially complementary to and may hybridize to) a sequence within another polynucleotide. As used herein, terms such as "target specific" and "selective," when used in reference to an antibody, are intended to mean an antibody that includes a features that is specific to (couples to) a particular type of target protein and that does not couple to any another type of protein.

As used herein, the terms "complementary" and "substantially complementary," when used in reference to a polynucleotide, are intended to mean that the polynucleotide includes a sequence capable of selectively hybridizing to a sequence in another polynucleotide under certain conditions.

As used therein, terms such as "amplification" and "amplify" refer to the use of any suitable amplification method to generate amplicons of a polynucleotide. Polymerase chain reaction (PCR) is one nonlimiting amplification method. Other suitable amplification methods known in the art include, but are not limited to, rolling circle amplification; riboprimer amplification (e.g., as described in U.S. Pat. No. 7,413,857); ICAN; UCAN; ribospia; terminal tagging (e.g., as described in U.S. 2005/0153333); and Eberwine-type aRNA amplification or strand-displacement amplification. Additional, nonlimiting examples of amplification methods are described in WO 02/16639; WO 00/56877; AU 00/29742; U.S. 5,523,204; U.S. 5,536,649; U.S. 5,624,825; U.S. 5,631,147; U.S. 5,648,211; U.S. 5,733,752; U.S. 5,744,311; U.S. 5,756,702; U.S. 5,916,779; U.S. 6,238,868; U.S. 6,309,833; U.S. 6,326,173; U.S. 5,849,547; U.S. 5,874,260; U.S. 6,218,151; U.S. 5,786,183; U.S. 6,087,133; U.S. 6,214,587; U.S. 6,063,604; U.S. 6,251,639; U.S. 6,410,278; WO 00/28082; U.S. 5,591,609; U.S. 5,614,389; U.S. 5,773,733; U.S. 5,834,202; U.S. 6,448,017; U.S. 6,124,120; and U.S. 6,280,949.

The terms "polymerase chain reaction" and "PCR," as used herein, refer to a procedure wherein small amounts of a polynucleotide, e.g., RNA and/or DNA, are amplified. Generally, amplification primers are coupled to the polynucleotide for use during the PCR. See, e.g., the following references, the entire contents of which are incorporated by reference herein: U.S. 4,683,195 to Mullis; Mullis et al., Cold Spring Harbor Symp. Quant. Biol., 51: 263 (1987); and Erlich, ed., PCR Technology, (Stockton Press, NY, 1989). A wide variety of enzymes and kits are available for performing PCR as known by those skilled in the art. For example, in some examples, the PCR amplification is performed using either the F AILSAFE^{™} PCR System or the MASTERAMP^{™} Extra-Long PCR System from EPICENTRE Biotechnologies, Madison, Wis., as described by the manufacturer.

As used herein, the term "chromatin" is intended to refer to a structure in which DNA and one or more proteins (such as histones) are condensed together into a chromosome. More tightly condensed chromatin may be referred to as heterochromatin, while more loosely condensed chromatin may be referred to as euchromatin.

As used herein, the term "protein" is intended to refer to a polypeptide chain that is folded into a tertiary structure. Proteins that are coupled to DNA may be referred to as "epigenetic" or "epigenomic" modifications to the DNA, and as such an "epigenetic assay" or "epigenomic" assay may refer herein to an assay to identify which proteins are bound to respective DNA loci. It may be desirable to determine which proteins are coupled to DNA, such as the proteins of euchromatin, and the respective loci of such proteins, because such proteins may be transcriptionally active and thus of interest

As used herein, the terms "locus" and "loci" refer to the locations along a polynucleotide at which a respective element, such as a protein, is present.

As used herein, the term "substrate" refers to a material used as a support for compositions described herein. Example substrate materials may include glass, silica, plastic, quartz, metal, metal oxide, organo-silicate (e.g., polyhedral organic silsesquioxanes (POSS)), polyacrylates, tantalum oxide, complementary metal oxide semiconductor (CMOS), or combinations thereof. An example of POSS can be that described in Kehagias et al., Microelectronic Engineering 86 (2009), pp. 776-778, which is incorporated by reference in its entirety. In some examples, substrates used in the present application include silica-based substrates, such as glass, fused silica, or other silica-containing material. In some examples, silica-based substrates can include silicon, silicon dioxide, silicon nitride, or silicone hydride. In some examples, substrates used in the present application include plastic materials or components such as polyethylene, polystyrene, poly(vinyl chloride), polypropylene, nylons, polyesters, polycarbonates, and poly(methyl methacrylate). Example plastics materials include poly(methyl methacrylate), polystyrene, and cyclic olefin polymer substrates. In some examples, the substrate is or includes a silica-based material or plastic material or a combination thereof. In particular examples, the substrate has at least one surface including glass or a silicon-based polymer. In some examples, the substrates can include a metal. In some such examples, the metal is gold. In some examples, the substrate has at least one surface including a metal oxide. In one example, the surface includes a tantalum oxide or tin oxide. Acrylamides, enones, or acrylates may also be utilized as a substrate material or component. Other substrate materials can include, but are not limited to gallium arsenide, indium phosphide, aluminum, ceramics, polyimide, quartz, resins, polymers, and copolymers. In some examples, the substrate and/or the substrate surface can be, or include quartz. In some other examples, the substrate and/or the substrate surface can be, or include, semiconductor, such as GaAs or ITO. The foregoing lists are intended to be illustrative of, but not limiting to the present application. Substrates can include a single material or a plurality of different materials. Substrates can be composites or laminates. In some examples, the substrate includes an organo-silicate material.

Substrates can be flat, round, spherical, rod-shaped, or any other suitable shape. Substrates may be rigid or flexible. In some examples, a substrate is a bead or a flow cell.

Substrates can be non-patterned, textured, or patterned on one or more surfaces of the substrate. In some examples, the substrate is patterned. Such patterns may include posts, pads, wells, ridges, channels, or other three-dimensional concave or convex structures. Patterns may be regular or irregular across the surface of the substrate. Patterns can be formed, for example, by nanoimprint lithography or by use of metal pads that form features on non-metallic surfaces, for example.

In some examples, a substrate described herein forms at least part of a flow cell or is located in or coupled to a flow cell. Flow cells may include a flow chamber that is divided into a plurality of lanes or a plurality of sectors. Example flow cells and substrates for manufacture of flow cells that can be used in methods and compositions set forth herein include, but are not limited to, those commercially available from Illumina, Inc. (San Diego, CA).

As used herein, the term "post translational modification" (PTM) refers to a modification of a protein following biosynthesis of that protein. Nonlimiting examples of PTMs include phosphorylation, methylation, nitrosylation, acetylation, and glycosylation. For a given protein, one of its forms may not be post translationally modified, while one or more other of its forms may be post translationally modified, e.g., by an enzyme.

As used herein, "analyte" is intended to mean a chemical or biological element that is desired to be detected. An analyte may be referred to as a "target." Analytes may include nucleotide analytes and non-nucleotide analytes. Nucleotide analytes may include one or more nucleotides. Non-nucleotide analytes may include chemical entities that are not nucleotides. An example nucleotide analyte is a DNA analyte, which includes a deoxyribonucleotide or modified deoxyribonucleotide. DNA analytes may include any DNA sequence or feature that may be of interest for detection, such as single nucleotide polymorphisms or DNA methylation. Another example nucleotide analyte is an RNA analyte, which includes a ribonucleotide or modified ribonucleotide. RNA analytes may include any RNA sequence or feature that may be of interest for detection, such as the presence or amount of mRNA or of cDNA. An example non-nucleotide analyte is a protein analyte. A protein includes a sequence of polypeptides that are folded into a structure. Another example non-nucleotide analyte is a metabolite analyte. A metabolite analyte is a chemical element that is formed or used during metabolism. Additional example analytes include, but are not limited to, carbohydrates, fatty acids, sugars (such as glucose), amino acids, nucleosides, neurotransmitters, phospholipids, and heavy metals. In the present disclosure, analytes may be detected in the context of any suitable application(s), such as analyzing a disease state, analyzing metabolic health, analyzing a microbiome, analyzing drug interaction, analyzing drug response, analyzing toxicity, or analyzing infectious disease. Illustratively, metabolites can include chemical elements that are upregulated or downregulated in response to disease. Nonlimiting examples of analytes include lipids, kinases, serine hydrolases, metalloproteases, disease-specific biomarkers such as antigens for specific diseases, and glucose.

As used herein, an "aptamer" is intended to mean an oligonucleotide that has a tertiary structure causing that oligonucleotide selective for a target, such as an analyte. To be "selective" for a target is intended to mean to couple to that target and not to couple to a different target. Aptamers may include any suitable type of oligonucleotide, e.g., DNA, RNA, and/or nucleic acid analogues such as exemplified elsewhere herein. An aptamer may become coupled to a target through any suitable combination of interactions, e.g., through any suitable combination of electrostatic interactions, hydrophobic interactions, and formation of a tertiary structure.

As used herein, "lectin" is intended to mean a protein that selectively binds a particular sugar or sugars, and as such does not bind any other sugars. "Monovalent" lectins may bind a single sugar at a given time, while "divalent" lectins may bind two sugars at once, and "multivalent" lectins may bind two or more sugars at once. Lectins may be naturally occurring, or non-naturally occurring. Naturally occurring lectins may include plant lectins and animal lectins.

As used herein, "sugar" is intended to mean a water-soluble carbohydrate. Sugars may include monosaccharides, disaccharides, and polysaccharides.

As used herein, "splint oligonucleotide" is intended to mean any oligonucleotide capable of connecting two other oligonucleotides together through complementary binding by the "splint oligonucleotide" to respective portions of each of the two other oligonucleotides. In some examples, the "splint oligonucleotide" connects the two other oligonucleotides together through ligating the two other oligonucleotides together.

As used herein, "probe" is intended to mean any biological or synthetic molecule capable of interacting with a target of interest, and capable of detecting the target of interest. Detection of the target of interest can occur through direct detection of the probe's interaction with the target or through indirect detection of amino acids or nucleotide sequences that are connected to the probe. In some examples, detection of the target of interest occurs after amino acids or nucleotide sequences are detached from the probe.

As used herein, "reporter oligonucleotide" is intended to mean any oligonucleotide that can be analyzed to determine the identity of a target of interest or an analyte of interest. In some examples, a "reporter oligonucleotide" is connected to a "probe." In some examples, a "reporter oligonucleotide" is detached from a "probe."

### Compositions and methods for detecting analytes using proximity-induced tagmentation

Some examples herein provide for detecting analytes using proximity-induced tagmentation.

For example, the proteome presents a significant opportunity for discovery in biological systems. The enzyme-linked immunosorbent assay (ELISA) is a standard method for detecting and quantifying a specific protein in a complex mixture. This approach relies on specific immobilization of the target of interest, usually via antibodies or other target recognition elements, followed by detection and quantitation with a second antibody coupled to a reporter molecule. This approach is well-established, but it is difficult to assess multiple targets simultaneously due to the limited variety of available reporter molecules. A robust and simplified method for converting multiplexed protein detection into a polynucleotide readout would be expected to help advance the field of proteomics and increase the utility of next generation sequencing (NGS) technology.

As provided herein, proximity-induced tagmentation is used to address the problem of detecting analytes, such as proteins or other biomolecules, in a multiplexed manner by generating reporter polynucleotides that may be sequenced, and from which sequences the analytes may be detected. In a manner such as described herein, proximity-induced tagmentation may be performed using a donor recognition probe and an acceptor recognition probe. The donor recognition probe includes a first analyte-specific recognition element and a transposome which includes a barcode (sequence) corresponding to the target analyte. The acceptor recognition probe includes a second analyte-specific recognition element and an oligonucleotide. Responsive to the recognition elements of the respective donor recognition probe and the acceptor recognition probe selectively binding to the same analyte as one another, the barcoded transposome is brought into sufficient proximity to the oligonucleotide as to tagment that oligonucleotide with the barcode - hence the term "proximity-induced tagmentation." The polynucleotide resulting from such tagmentation includes both the barcode from the donor recognition probe and the oligonucleotide from the acceptor recognition probe. As such, the sequence of this "reporter" polynucleotide reflects that it was formed responsive to proximity of two probes that were specific for the same analyte. Accordingly, it may be understood that the present assay is highly specific and readily may be read out by sequencing the reporter polynucleotide.

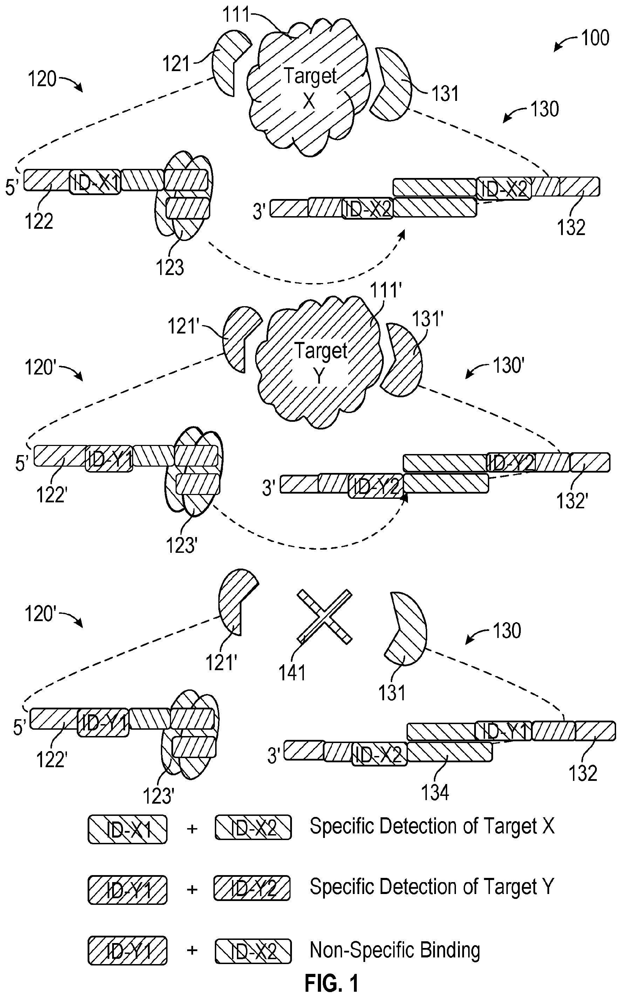

FIG. 1 schematically illustrates example operations and compositions in a process flow for detecting analytes using proximity-induced tagmentation. Composition 100 illustrated in FIG. 1 includes a plurality of analytes 111, 111' each having a first portion and a second portion, a plurality of donor recognition probes 120, 120', and a plurality of acceptor recognition probes 130, 130'. Each of the donor recognition probes 120, 120' may include a first recognition element 121, 121' specific to a first portion of a respective analyte 111, 111', a first oligonucleotide 122, 122' corresponding to the first portion of that respective analyte, and a transposase 123, 123' coupled to the first recognition element and the first oligonucleotide. Each of the acceptor recognition probes 130, 130' may include a second recognition element 131, 131' specific to a second portion of a respective analyte 111, 111' and a second polynucleotide 132, 132' coupled to the second recognition element and corresponding to the second portion of that respective analyte. In some examples, the first and second oligonucleotides 122, 122', 132, 132' may include DNA. Note that the donor recognition probes 120, 120' and acceptor recognition probes 130, 130' may be provided in a kit that includes, for each analyte that it may be desired to assay (which may number, for example, in the tens, hundreds, thousands, or millions), a plurality of donor recognition probes and a plurality of acceptor recognition probes that include recognition elements that are specific for that analyte. In the simplified example illustrated in FIG. 1, the kit may include a plurality of donor recognition probes 120 and a plurality of acceptor recognition probes 130 that are specific to analyte 111, and a plurality of donor recognition probes 120' and a plurality of acceptor recognition probes 130' that are specific to analyte 111'.

At the particular time illustrated in FIG. 1, the first recognition element 121 of first donor recognition probe 120 is specifically coupled to the first portion of the analyte 111, and the second recognition element 131 of first acceptor recognition probe 130 is specifically coupled to the second portion of the analyte 111. Responsive to such coupling of recognition elements 121, 131 to respective portions of analyte 111, transposase 123 tagments second oligonucleotide 132, resulting in first oligonucleotide 122 becoming covalently coupled to second oligonucleotide 132. First oligonucleotide 122 may include a sequence that corresponds to the first portion of analyte 111, e.g., the barcode "ID-X1," and second oligonucleotide 132 may include a sequence that corresponds to the second portion of analyte 111, e.g., the barcode "ID-X2." Accordingly, it may be understood that transposase 123 generates a "reporter" polynucleotide that includes both the sequences ID-X1 and ID-X2, from which it may be determined that analyte 111 was present and was coupled to both first recognition element 121 and second recognition element 131, resulting in proximity-induced tagmentation of second oligonucleotide 132 by transposase 123. Because the sequences ID-X1 and ID-X2 correspond to the same analyte as one another, it may be determined that both first recognition element 121 and second recognition element 131 were specifically coupled to such analyte.

Similarly, the first recognition element 121' of second donor recognition probe 120' is specifically coupled to the first portion of the analyte 111', and the second recognition element 131' of second acceptor probe 130' is specifically coupled to the second portion of the analyte 111'. Responsive to such coupling of recognition elements 121', 131' to respective portions of analyte 111', transposase 123' tagments second oligonucleotide 132', resulting in first oligonucleotide 122' becoming covalently coupled to second oligonucleotide 132'. First oligonucleotide 122' may include a sequence that corresponds to the first portion of analyte 111', e.g., the barcode "ID-Y1," and second oligonucleotide 132' may include a sequence that corresponds to the second portion of analyte 111', e.g., the barcode "ID-Y2." Accordingly, it may be understood that transposase 123' generates a "reporter" polynucleotide that includes both the sequences ID-Y1 and ID-Y2, from which it may be determined that analyte 111' was present and was coupled to both first recognition element 121' and second recognition element 131', resulting in proximity-induced tagmentation of first oligonucleotide 132' by transposase 123'. Because the sequences ID-Y1 and ID-Y2 correspond to the same analyte as one another, it may be determined that both first recognition element 121' and second recognition element 131' were specifically coupled to such analyte.

In comparison, any tagmentation resulting from non-specific binding of recognition elements to contamination or other elements in the sample may be expected to generate reporter polynucleotides that include mismatched barcodes. In an illustrative example of non-specific binding, first recognition element 121' of a second donor probe 120' is non-specifically coupled to a first portion of analyte 141, and the second recognition element 131 of a first acceptor probe 130 is non-specifically coupled to a second portion of the analyte 141. Responsive to such coupling of recognition elements 121', 131 to respective portions of analyte 141, transposase 123' tagments oligonucleotide 132, resulting in oligonucleotide 122' becoming covalently coupled to oligonucleotide 132. As described above, oligonucleotide 122' may include a sequence that corresponds to the first portion of analyte 111', e.g., the barcode "ID-Y1," and oligonucleotide 132 may include a sequence that corresponds to the first portion of analyte 111, e.g., the barcode "ID-X2." Accordingly, it may be understood that transposase 123' generates a "reporter" polynucleotide that includes both the sequences ID-Y1 and ID-X2, from which it may be determined that analyte 141 was present and coupled to both first recognition element 121' and second recognition element 131, resulting in proximity-induced tagmentation of oligonucleotide 132 by transposase 123'. Because the sequences ID-Y1 and ID-X2 do not correspond to the same analyte as one another, it may be determined that either or both first recognition element 121' and second recognition element 131 were non-specifically coupled to such analyte.

It will be appreciated that any suitable analytes may be assayed using proximity-induced tagmentation, and that any suitable recognition elements may be used to specifically bind to such analytes. In some examples, the analytes may include a first molecule. For example, the first portion of the analyte (to which the first recognition element may specifically bind) may include a first portion of the first molecule, and the second portion of the analyte (to which the second recognition element may specifically bind) may include a second portion of the first molecule. Illustratively, the first molecule may include a protein or peptide, the first recognition element 121, 121' may include a first antibody or a first aptamer that is specific to a first portion of the protein or peptide, and the second recognition element 131, 131' may include a second antibody or a second aptamer that is specific to a second portion of the protein or peptide. Or, for example, the first molecule may include a target polynucleotide, the first recognition element 121, 121' may include a first CRISPR-associated (Cas) protein that is specific to a first subsequence of the target polynucleotide, and the second recognition element 131, 131' may include a second Cas protein that is specific to a second subsequence of the target polynucleotide. In some examples, the target polynucleotide may include RNA, and the first and second Cas proteins independently are selected from the group consisting of rCas9 and dCas13. Or, for example, the first molecule may include a carbohydrate, the first recognition element 121, 121' may include a first lectin that is specific to a first portion of the carbohydrate, and the second recognition element 131, 131' may include a second lectin that is specific to a second portion of the carbohydrate. Or, for example, the first molecule may include a biomolecule, and the biomolecule may be specific for the first and second recognition elements 121, 131 or 121', 131'. However, it will be appreciated that recognition elements 121, 121', 131, 131' may have any suitable configuration that specifically recognizes and becomes coupled to an analyte of interest or that the analyte specifically recognizes and becomes coupled to, e.g., a specific binding protein.

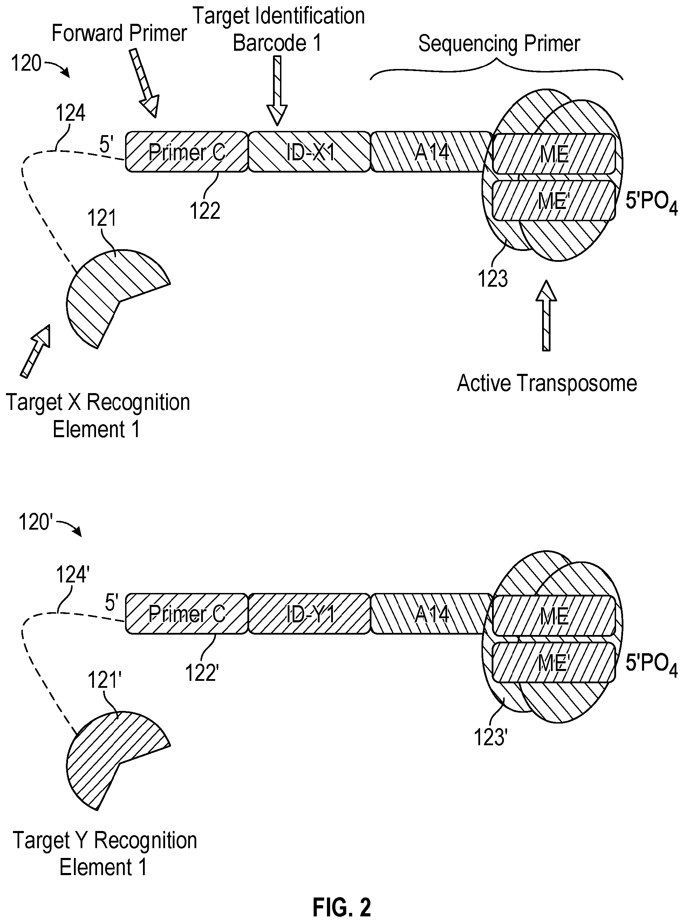

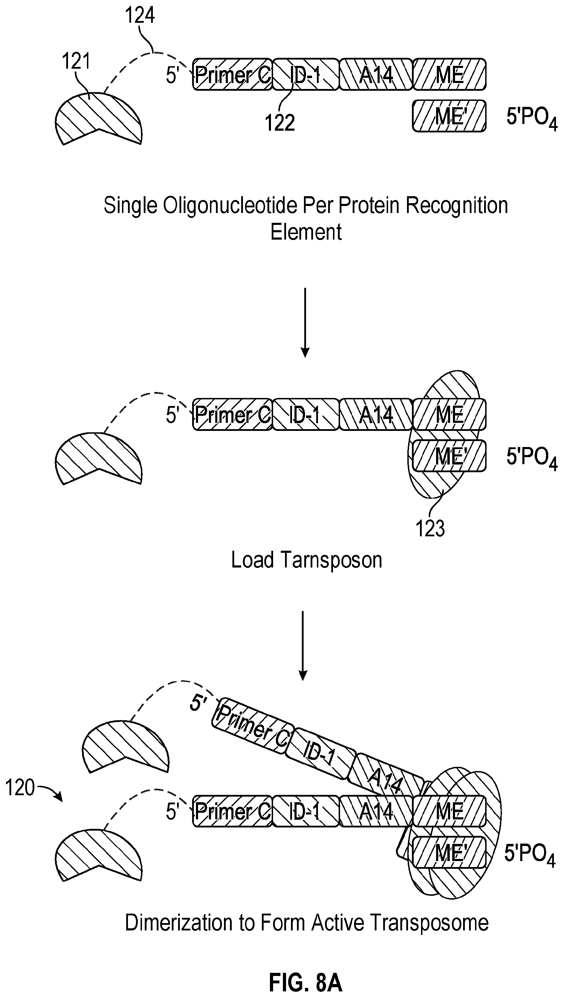

The oligonucleotides 122, 122' of donor recognition probes 120, 120' may include any suitable sequence for use in binding transposases 123, 123' for tagmenting oligonucleotides 132, 132', and being subsequently amplified and sequenced. FIG. 2 schematically illustrates example donor recognition probes for use in detecting analytes using proximity-induced tagmentation. First oligonucleotide 122 may be synthetic and may include annealed mosaic end (ME, ME') transposon end sequences, a sequencing primer (e.g., A14), a unique barcode that identifies the recognition element 121 (e.g., ID-X1), and a primer binding site (e.g., Primer C). Similarly, first oligonucleotide 122' may be synthetic and may include annealed mosaic end (ME, ME') transposon end sequences, a sequencing primer (e.g., A14), a unique barcode that identifies the recognition element 121' (e.g., ID-Y1), and a forward primer binding site (e.g., Primer C). Transposase 123, e.g., Tn5, may be coupled to the annealed mosaic end transposon end sequences (ME, ME') at the 3' end of oligonucleotide 122 to form an active transposome. Transposase 123', e.g., Tn5, may be coupled to the annealed mosaic end transposon end sequences (ME, ME') at the 3' end of oligonucleotide 122' to form an active transposome. The 5' end of oligonucleotide 122 may be coupled to recognition element 121 via linker 124, and the 5' end of oligonucleotide 122' may be coupled to recognition element 121' via linker 124'. Aptamers, antibodies, proteins, and the like that are coupled to custom-designed oligonucleotides are commercially available, or methods of preparing such couplings are otherwise known in the art. Further options for preparing donor recognition probes are provided further below with reference to FIGS. 8A-8C. In this regard, although the depictions of the transposases in donor recognition probes 120, 120' may be simplified in FIGS. 1, 2, 4A, 7A-7C, 9A-9E, 10A-10D, 11A-11C, and 12 by illustrating only a single oligonucleotide coupled to those transposases, it should be appreciated that the present donor recognition probes may include pairs of oligonucleotides reflecting that the transposases may be dimerized in a manner such as described below with reference to FIGS. 8A-8C.

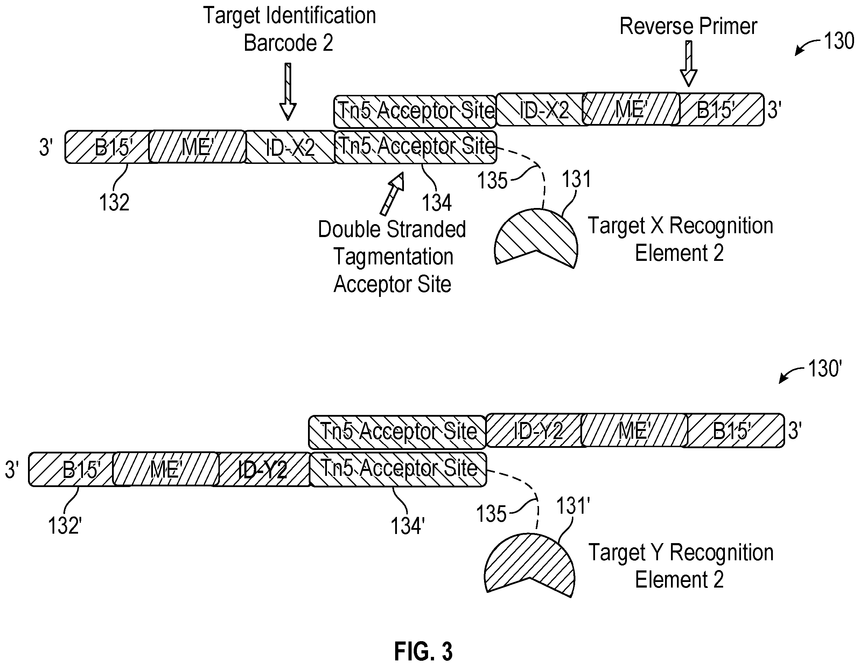

The oligonucleotides 132, 132' of acceptor recognition probes 130, 130' may include any suitable sequence for use in being tagmented by transposases 123, 123' to be coupled to oligonucleotides 122, 122' and subsequently amplified and sequenced. FIG. 3 schematically illustrates example acceptor recognition probes for use in detecting analytes using proximity-induced tagmentation. Second oligonucleotide 132 may be synthetic and may include a reverse mosaic end transposon end sequence (ME'), a reverse sequencing primer (e.g., B15'), a unique barcode that identifies the recognition element 131 (e.g., ID-X2), and double-stranded tagmentation acceptor site (e.g., TN5 acceptor site) 134. Similarly, oligonucleotide 132' may be synthetic and may include a reverse annealed mosaic end transposon end sequence (ME'), a reverse sequencing primer (e.g., B15'), a unique barcode that identifies the recognition element 131' (e.g., ID-Y2), and double-stranded tagmentation acceptor site (e.g., TN5 acceptor site) 134'. Furthermore, the acceptor recognition probe may include two 3' overhangs each of which may include the unique barcode that identifies the recognition element. The 5' end of oligonucleotide 132 may be coupled to recognition element 131 via linker 135, and the 5' end of oligonucleotide 132' may be coupled to recognition element 131' via linker 135'. Aptamers, antibodies, proteins, and the like that are coupled to custom-designed oligonucleotides are commercially available, or methods of preparing such couplings are otherwise known in the art.

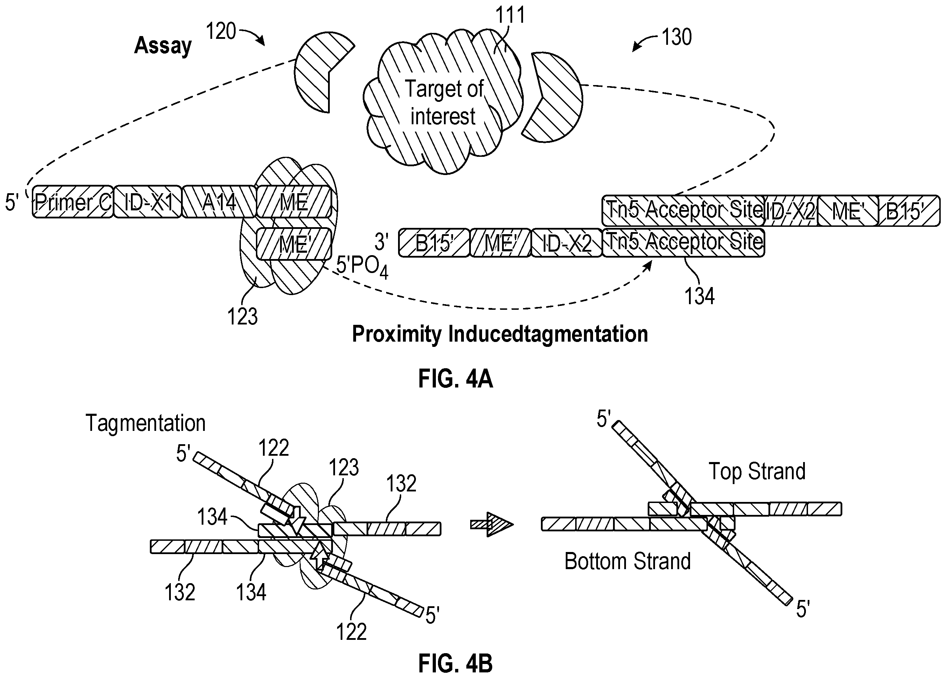

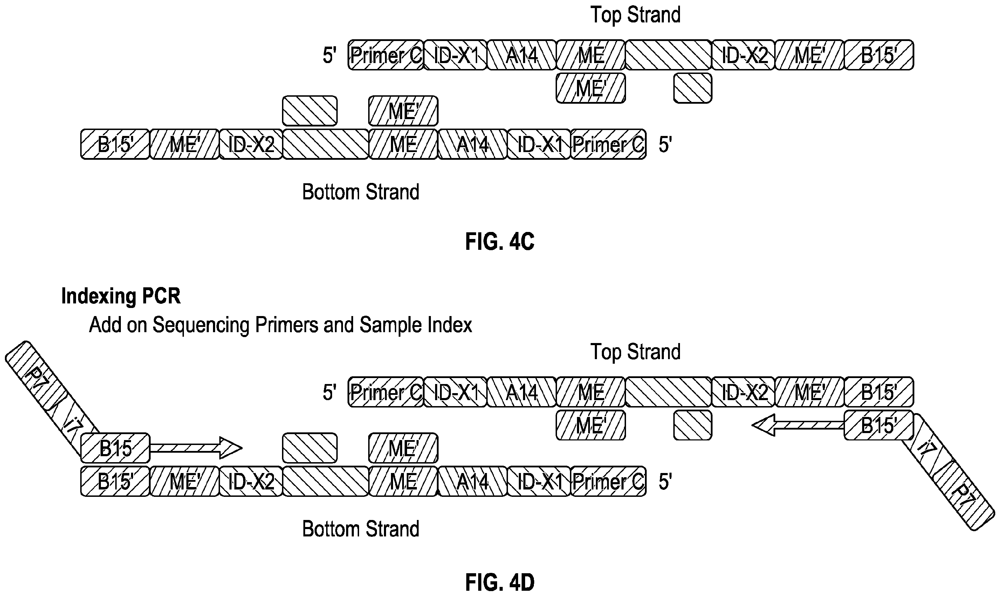

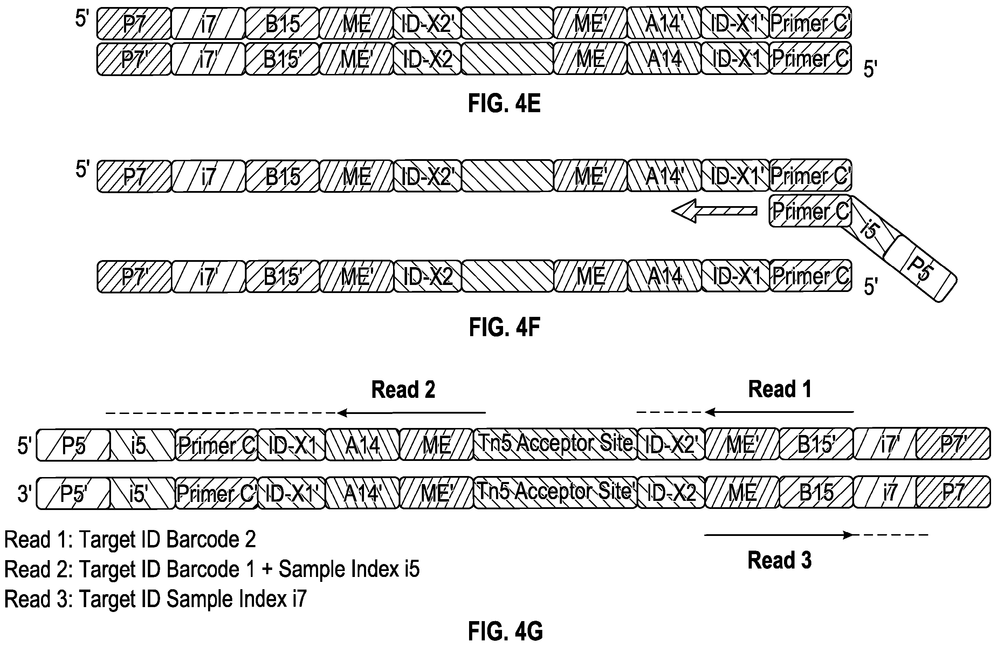

FIGS. 4A-4G schematically illustrate further details of operations and compositions in the process flow of FIG. 1, according to some examples. For example, FIG. 4A illustrates an assay in which donor recognition probe 120 (described with reference to FIGS. 1 and 2) and acceptor recognition probe 130 (described with reference to FIGS. 1 and 3) perform proximity-induced tagmentation responsive to those probes' recognition elements specifically binding analyte 111. More specifically, transposase 123 tagments double-stranded tagmentation acceptor site 134 responsive to such specific binding of analyte 111. The tagmentation reaction may be initiated by adding any suitable cofactor for transposome cleavage and insertion activity, e.g., magnesium ions (Mg++). FIG. 4B illustrates further details of the tagmentation reaction, in which transposase 123 inserts first oligonucleotide 122 into the double-stranded tagmentation acceptor site 134 of second oligonucleotide 132. In this regard, as noted elsewhere herein, the present donor recognition probes 120 may include pairs of oligonucleotides reflecting that the transposases may be dimerized in a manner such as described below with reference to FIGS. 8A-8C, and accordingly the tagmentation reaction may generate top and bottom strands having sequences such as illustrated schematically in FIG. 4B and FIG. 4C. Furthermore, as noted elsewhere herein, the acceptor recognition probe may include two 3' overhangs each of which may include the unique barcode that identifies the recognition element, which may provide increased redundancy by producing two template strands per tagmentation event as illustrated in FIGS. 4B and 4C. As illustrated in FIG. 4D, sample indexes (i7 and i5) may be added to the template strands using primers and extended to form a duplex such as illustrated in FIG. 4E. As illustrated in FIG. 4F, primers (e.g., primer C') may be annealed to the complementary strand and extended to form an elongated reporter polynucleotide that is then PCR amplified and that includes both sample indexes and the barcodes corresponding to recognition elements 121, 131. As illustrated in FIG. 4G, sequencing then is used to determine donor and recognition probe identities and sample indexes. For example, a first read ("Read 1") may be performed by annealing a suitable primer to the B15' primer on the top strand to read the sequence ID-X2' corresponding to recognition element 131. Additionally, a second read ("Read 2") may be performed by annealing a suitable primer to the ME and A14 primer on the top strand to read the sequence ID-X1 corresponding to recognition element 121 and the i5 sample index. Additionally, a third read ("Read 3") may be performed by annealing a suitable primer to the ME and B 15 primer on the bottom strand to read the i7 sample index. It will be appreciated, however, that any suitable sequencing method may be used to read the two barcode sequences within the reporter oligonucleotides, and that the use of sample indexes is optional.

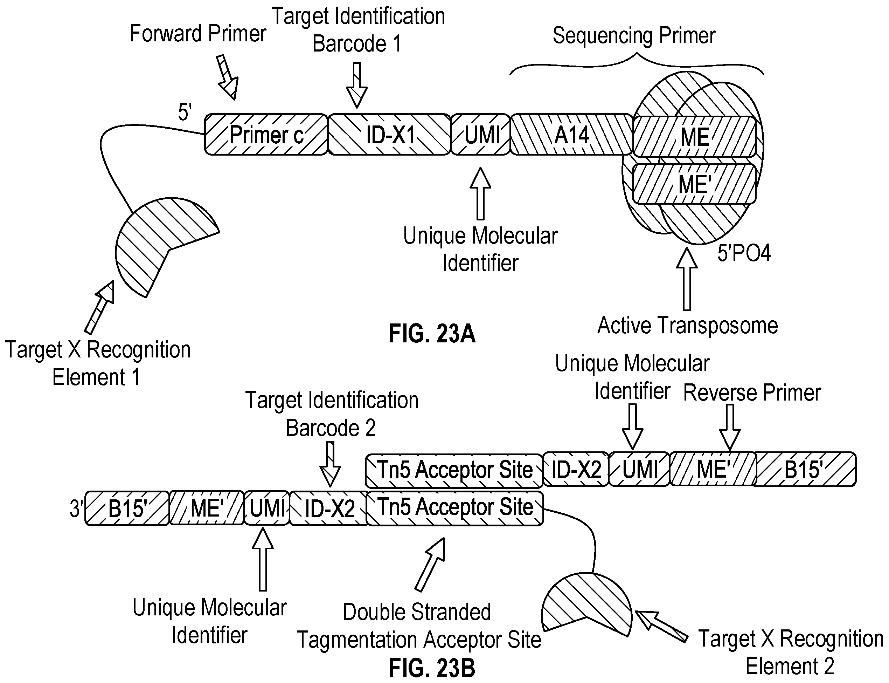

The precision of PCR quantitation of the tagmentation products may be impacted by the amplification of PCR duplicates. In order to distinguish duplicates from distinct detection events, unique molecular identifiers (UMIs) may be added to the donor recognition probe, as illustrated in FIG. 23A, the acceptor recognition probe, as illustrated in FIG. 23B, or both. The UMI sequence may be a random sequence of nucleotides. Alternatively, the UMI may be a sequence randomly selected from a set of known sequences, which enables error correction and avoidance of undesired secondary structure, e.g. dsDNA that would be a target for the transposase Tn5. Note that the two UMIs illustrated in the acceptor recognition probe illustrated in FIG. 23B may be the same as one another, or may be different than one another.

It will further be appreciated that the examples of analytes provided with reference to FIG. 1 are purely illustrative. Another nonlimiting example of an analyte that may be assayed using the present proximity-induced tagmentation is a post-translational modification (PTM) of a protein. Proteins, for example, frequently exhibit PTMs due to phosphorylation, acetylation, methylation, nitrosylation, glycosylation, and many other mechanisms. To distinguish between these different target forms, and to determine the fraction of total target that is modified with a PTM of interest, a system may be used that includes three recognition elements: a donor (PTM) recognition probe, with a recognition element that binds to the target in a PTM-specific manner; a donor (no PTM) recognition probe, with a recognition element that is either (1) specific to the opposite form of the target than the donor (PTM) probe or that (2) can bind either form of the target; and an acceptor recognition probe, with a recognition element that binds to either form of the target. Depending on the specificity of the donor recognition probes, different incubation strategies may be used.

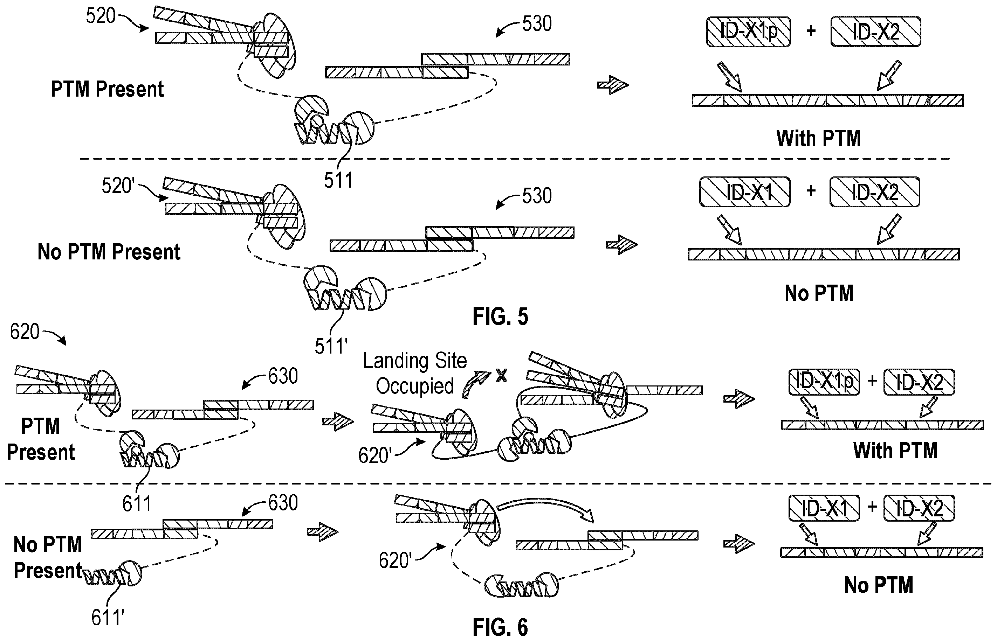

For example, if the donor recognition probes are exclusive and specific to each PTM form, they can be incubated in the same reaction and distinguished bioinformatically by unique combinations of acceptor and donor barcodes. For example, FIG. 5 schematically illustrates example operations and compositions in a process flow for detecting PTMs. In some examples, this strategy uses two donor recognition probes that bind a similar site on the protein (with the difference being +/- PTM at that site).

In FIG. 5, a first form 511 of a protein is post-translationally modified (PTM), and a second form 511' is not PTM or has a different PTM. Illustratively, the first form 511 may be phosphorylated, acetylated, methylated, nitrosylated, or glycosylated relative to the second form 511', although the first form may include any other suitable modifications, and optionally the second form may be modified differently than the first form. The donor recognition probes (e.g., in a kit) include a first donor recognition probe 520 which is specific to the first form (e.g., includes a recognition element specific to the first form) and includes an oligonucleotide with a barcode corresponding to the first form (e.g., ID-X1p), and a second donor recognition probe 520' which is specific to the second form (e.g., includes a recognition element specific to the second form) and includes an oligonucleotide with a barcode corresponding to the second form (e.g., ID-X1). The acceptor recognition probes (e.g., in the kit) 530 may be specific to the protein, but need not necessarily be specific to the first or second form, and include an oligonucleotide with a barcode corresponding to either form (e.g., ID-X2). It will be appreciated that use of acceptor recognition probes that respectively are specific to particular forms may provide even further specificity.

As illustrated in FIG. 5, first donor recognition probe 520 and acceptor recognition probe 530 specifically bind to first form 511, responsive to which proximity-induced tagmentation occurs resulting in generation of a reporter polynucleotide including ID-X1p and ID-X2. Second donor recognition probe 520' and acceptor recognition probe 530 specifically bind to second form 511', responsive to which proximity-induced tagmentation occurs resulting in generation of a reporter polynucleotide including ID-X1 and ID-X2. Because first and second donor recognition probes 520, 520' are specific to their respective forms 511, 511', they may be co-incubated. Accordingly, it may be determined from the sequences of the reporter polynucleotides that the protein had the first form 511 in the first instance, and the second form 511' in the second instance. Optionally, amounts of the first and second forms of the first one of the analytes may be determined based on amounts of the reporter polynucleotides corresponding to the first and second ones of the donor recognition probes. For example, the amounts of the respective reporter polynucleotides illustrated in FIG. 5 correlate to the amounts of the first and second forms 511, 511' that were assayed.

Alternatively, if one of the donor recognition probes is not specific for the PTM but is specific for the analyte, the two forms of the analyte may be distinguished using a sequential reaction. For example, FIG. 6 schematically illustrates example operations and compositions in a process flow for detecting post-translational modifications (PTMs) using a PTM-specific donor-recognition probe and a non-PTM specific donor-recognition probe. In FIG. 6, a first form 611 of a protein is post-translationally modified (PTM), and a second form 611' is not PTM or has a different PTM. Illustratively, the first form 611 may be phosphorylated, acetylated, methylated, nitrosylated, or glycosylated relative to the second form 611', although the first form may include any other suitable modifications, and optionally the second form may be modified differently than the first form. The donor recognition probes (e.g., in a kit) include a first donor recognition probe 620 which is specific to the first form (e.g., includes a recognition element specific to the first form) and includes an oligonucleotide with a barcode corresponding to the first form (e.g., ID-X1p), and a second donor recognition probe 620' which is specific to the protein but is not specific to either the first or second form (e.g., includes a recognition element specific to the protein) and includes an oligonucleotide with a barcode corresponding to the protein (e.g., ID-X1). The acceptor recognition probes (e.g., in the kit) 630 may be specific to the protein, but need not necessarily be specific to the first or second form, and include an oligonucleotide with a barcode corresponding to either form (e.g., ID-X2).

Because second donor recognition probe 620' may non-specifically bind either to first form 611 or to second form 611', if probe 620' were incubated at the same time as probe 620, then probe 620' may bind to first form 611, thus inhibiting probe 620 from binding to first form 611 and making it appear (via the sequencing readout) as though the first form was not present. So as to provide enhanced differentiation between the first form 611 and second form 611', a sequential reaction may be used as illustrated in FIG. 6, in which first donor recognition probe 620 specifically binds to first form 611 and not to second form 611', and in which acceptor recognition probes 630 bind both to first form and to second form 611'. At first form 611, the transposase of donor recognition probe 620 performs proximity-induced tagmentation using the oligonucleotide of acceptor recognition probe 630, generating a reporter polynucleotide including ID-X1p and ID-X2. At second form 611', acceptor recognition probe 630 is bound but lacks an acceptor recognition probe with which to perform proximity-induced tagmentation. Donor recognition probe 620' then is added and incubated, as illustrated in FIG. 6. During such incubation, donor recognition probe 620' may attempt to bind with first form 611, but is inhibited from participating in proximity-induced tagmentation because the acceptor recognition probe 630 has already reacted with probe 620 and/or because the recognition element of probe 620 at least partially occupies the landing site for probe 620'. However, donor recognition probe 620' readily may bind to second form 611', responsive to which the transposase of probe 620' performs proximity-induced tagmentation using the oligonucleotide of acceptor recognition probe 630, generating a reporter polynucleotide including ID-X1 and ID-X2. Accordingly, it may be determined from the sequences of the reporter polynucleotides that the protein had the first form 611 in the first instance, and the second form 611' in the second instance. Optionally, amounts of the first and second forms of the first one of the analytes may be determined based on amounts of the reporter polynucleotides corresponding to the first and second ones of the donor recognition probes. For example, the amounts of the respective reporter polynucleotides illustrated in FIG. 6 correlate to the amounts of the first and second forms 611, 611' that were assayed.

Note that in examples in which different probes may compete with one another to bind to analytes, e.g., such as described with reference to FIG. 6, the concentration of each probe may be calibrated for enhanced specificity. For example, more specific donor recognition probes may be used at higher concentrations to drive rapid, accurate binding before non-specific binding of other recognition probes.

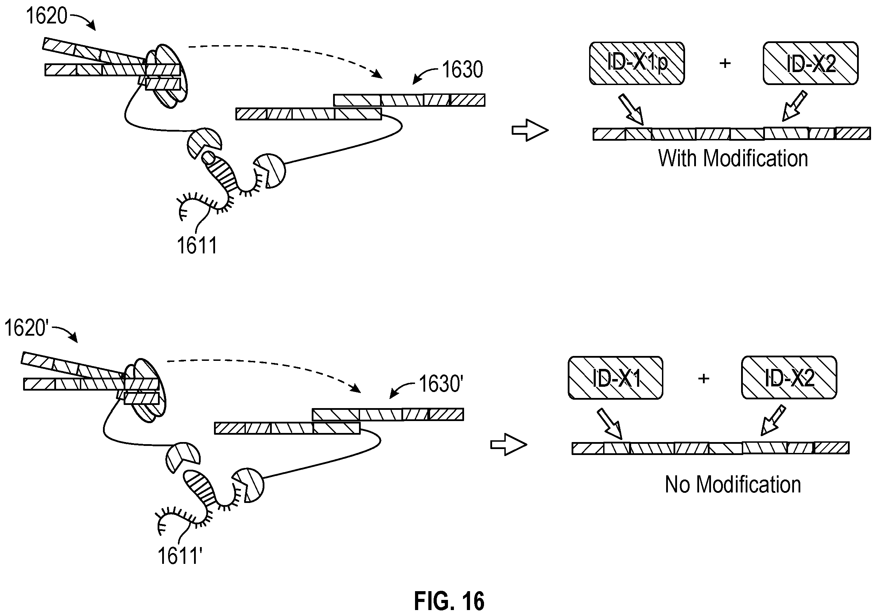

Similar to assays for detecting PTMs, proximity-induced tagmentation may be used to detect nucleic acid modifications, e.g. N⁶-methyladenosine RNA modifications, 5-methylcytosine DNA modifications, etc. For example, as illustrated in the top panel of FIG. 16, donor recognition probe 1620 and acceptor recognition probe 1630 specifically bind to a modified oligonucleotide target 1611, and proximity-induced tagmentation occurs resulting in generation of a reporter polynucleotide including ID-X1p and ID-X2.

Proximity-induced tagmentation may also be used to distinguish between different target forms, such as a modified form of an oligonucleotide and a non-modified form of the same oligonucleotide, and to determine the fraction of total target that is modified. Three recognition elements may be used: a first donor recognition probe, with a recognition element that binds to the target in a modification-specific manner; a second donor recognition probe, with a recognition element that is either (1) specific to the opposite form of the target than the first donor recognition probe or that (2) can bind either form of the target; and an acceptor recognition probe, with a recognition element that binds to either form of the target. Depending on the specificity of the donor recognition probes, different incubation strategies may be used.

For example, if the donor recognition probes are exclusive and specific to each form of the target, they can be incubated in the same reaction and distinguished bioinformatically by unique combinations of acceptor and donor barcodes. FIG. 16 schematically illustrates example operations and compositions in a process flow for detecting modifications using donor-recognition probes that are specific for nucleotide modifications. In FIG. 16, a first form 1611 of an oligonucleotide is modified, and a second form 1611' is not modified or has a different modification. Illustratively, the first form 1611 may include methylated adenosines relative to the second form 1611', although the first form may include any other suitable modifications, and optionally the second form may be modified differently than the first form. The donor recognition probes (e.g., in a kit) include a first donor recognition probe 1620 which is specific to the first form (e.g., includes a recognition element specific to the first form) and includes an oligonucleotide with a barcode corresponding to the first form (e.g., ID-X1p), and a second donor recognition probe 1620' which is specific to the second form (e.g., includes a recognition element specific to the second form) and includes an oligonucleotide with a barcode corresponding to the second form (e.g., ID-X1). The acceptor recognition probes (e.g., in the kit) 1630 may be specific to the oligonucleotide, but need not necessarily be specific to the first or second form, and include an oligonucleotide with a barcode corresponding to either form (e.g., ID-X2). It will be appreciated that use of acceptor recognition probes that respectively are specific to particular forms may provide even further specificity.

As illustrated in FIG. 16, first donor recognition probe 1620 and acceptor recognition probe 1630 specifically bind to first form 1611, responsive to which proximity-induced tagmentation occurs resulting in generation of a reporter polynucleotide including ID-X1p and ID-X2. Second donor recognition probe 1620' and acceptor recognition probe 1630 specifically bind to second form 1611', responsive to which proximity-induced tagmentation occurs resulting in generation of a reporter polynucleotide including ID-X1 and ID-X2. Because first and second donor recognition probes 1620, 1620' are specific to their respective forms 1611, 1611', they may be co-incubated. Accordingly, it may be determined from the sequences of the reporter polynucleotides that the oligonucleotide was of the first form 1611 in the first instance, and the second form 1611' in the second instance. Optionally, amounts of the first and second forms of the analytes may be determined based on amounts of the reporter polynucleotides corresponding to the first and second donor recognition probes. For example, the amounts of the respective reporter polynucleotides illustrated in FIG. 16 correlate to the amounts of the first and second forms 1611, 1611' that were assayed.

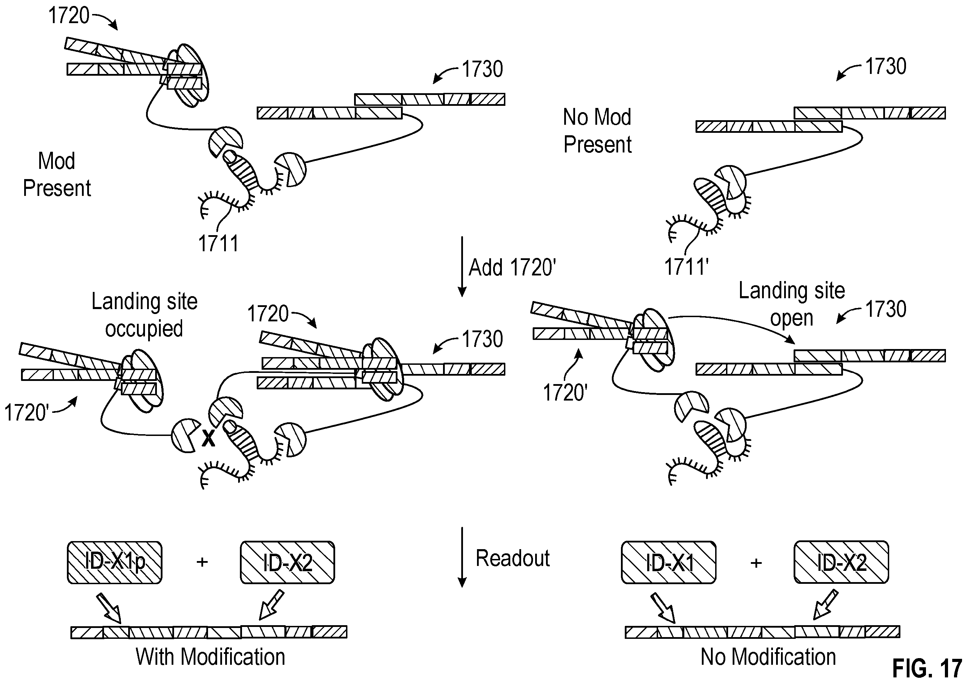

Alternatively, if one of the donor recognition probes is non-specific, the two forms may be distinguished using a sequential reaction. For example, FIG. 17 schematically illustrates example operations and compositions in a process flow for detecting nucleic acid modifications using donor-recognition probes that can detect the modification specifically, and donor-recognition probes that are specific to the target but not specific to the modification. In FIG. 17, a first form 1711 of a target oligonucleotide includes nucleotide modifications, and a second form 1711' is not modified or has a different modification. The donor recognition probes (e.g., in a kit) include a first donor recognition probe 1720 which is specific to the first form (e.g., includes a recognition element specific to the first form) and includes an oligonucleotide with a barcode corresponding to the first form (e.g., ID-X1p), and a second donor recognition probe 1720' which is specific to the target oligonucleotide but is not specific to either the first or second form (e.g., includes a recognition element specific to the oligonucleotide) and includes an oligonucleotide with a barcode corresponding to the target oligonucleotide (e.g., ID-X1). The acceptor recognition probes (e.g., in the kit) 1730 may be specific to the target oligonucleotide, but need not necessarily be specific to the first or second form, and include an oligonucleotide with a barcode corresponding to either form (e.g., ID-X2).

Because second donor recognition probe 1720' may non-specifically bind either to first form 1711 or to second form 1711', if probe 1720' were incubated at the same time as probe 1720, then probe 1720' may bind to first form 1711, thus inhibiting probe 1720 from binding to first form 1711 and making it appear (via the sequencing readout) as though the first form was not present. So as to provide enhanced differentiation between the first form 1711 and second form 1711', a sequential reaction may be used as illustrated in FIG. 17, in which first donor recognition probe 1720 specifically binds to first form 1711 and not to second form 1711', and in which acceptor recognition probes 1730 bind both to first form 1711 and to second form 1711'. At first form 1711, the transposase of donor recognition probe 1720 performs proximity-induced tagmentation using the oligonucleotide of acceptor recognition probe 1730, generating a reporter polynucleotide including ID-X1p and ID-X2. At second form 1711', acceptor recognition probe 1730 is bound but lacks an acceptor recognition probe with which to perform proximity-induced tagmentation. Donor recognition probe 1720' then is added and incubated. During such incubation, donor recognition probe 1720' may attempt to bind with first form 1711, but is inhibited from participating in proximity-induced tagmentation because the acceptor recognition probe 1730 has already reacted with probe 1720 and/or because the recognition element of probe 1720 at least partially occupies the landing site for probe 1720'. However, donor recognition probe 1720' readily may bind to second form 1711', responsive to which the transposase of probe 1720' performs proximity-induced tagmentation using the oligonucleotide of acceptor recognition probe 1730, generating a reporter polynucleotide including ID-X1 and ID-X2. Accordingly, it may be determined from the sequences of the reporter polynucleotides that the target oligonucleotide had the first form 1711 in the first instance, and the second form 1711' in the second instance. Optionally, amounts of the first and second forms of the analytes may be determined based on amounts of the reporter polynucleotides corresponding to the first and second donor recognition probes. For example, the amounts of the respective reporter polynucleotides illustrated in FIG. 17 correlate to the amounts of the first and second forms 1711, 1711' that were assayed.

In examples in which different probes may compete with one another to bind to analytes, e.g., such as described with reference to FIG. 17, the concentration of each probe may be calibrated for enhanced specificity. For example, more specific donor recognition probes may be used at higher concentrations to drive rapid, accurate binding before non-specific binding of other recognition probes.

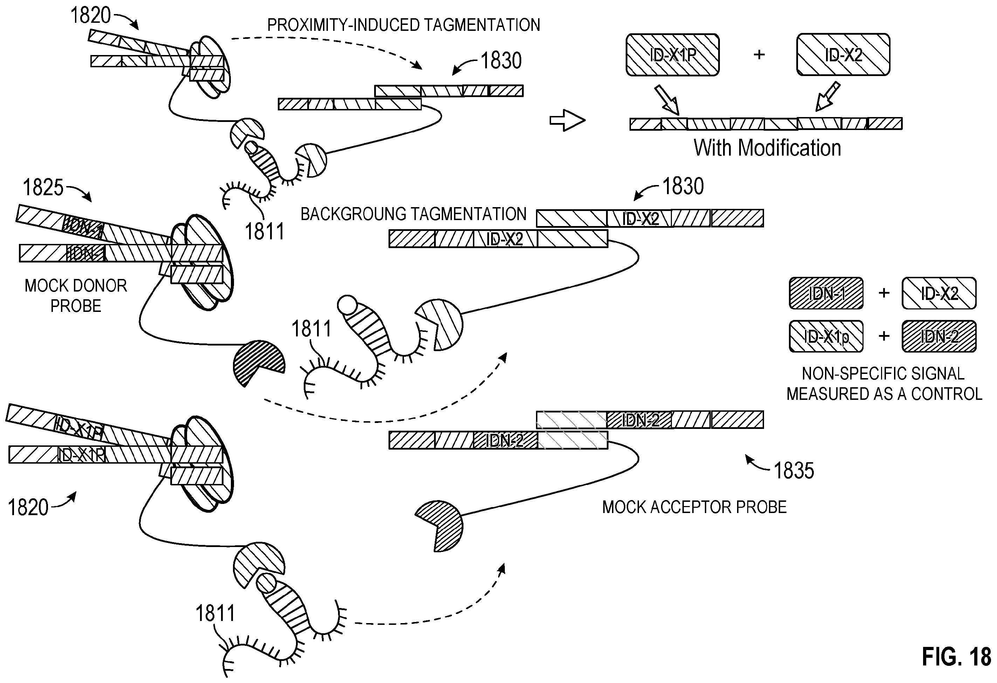

As illustrated in FIG. 18, when assaying a modified oligonucleotide target, an amount of background activity may be quantified to determine how much of the signal observed in the assay is due to true proximity-induced tagmentation. For example, a sample may be incubated with a mixture of mock donor recognition probe 1825, which does not specifically bind to modified oligonucleotide 1811 and which includes distinguishable barcodes "IDN-1", and acceptor recognition probe 1830, which includes distinguishable barcode "ID-X2". Acceptor recognition probe 1830 may specifically bind to molecule 1811. As a result of non-specific binding, mock donor recognition probe 1825 may come into sufficient proximity to acceptor recognition probe 1830 to perform background tagmentation, generating a background reporter polynucleotide that includes the barcodes IDN-1 and ID-X2. The sample (or another sample) may also be incubated with a mixture of mock acceptor recognition probe 1835, which does not specifically bind to molecule 1811 and which includes a distinguishable barcode "IDN-2", and donor recognition probe 1820, which includes barcode "ID-X1p". Donor recognition probe 1820 may specifically bind to molecule 1811. As a result of non-specific binding, mock acceptor recognition probe 1835 may come into sufficient proximity to donor recognition probe 1820 to perform background tagmentation, generating a background reporter polynucleotide that includes the barcodes ID-X1p and IDN-2. All reporter nucleotides in the sample may be sequenced and quantified, and the amount of the two background reporter polynucleotides, representative of background tagmentation events, may be sequenced and quantified, and the amount may be compared with the amount of the reporter polynucleotide that includes the barcodes ID-X1p and ID-X2, representing true proximity-induced tagmentation events.

In some examples, proximity-induced tagmentation may be used to detect molecular interactions, in which the analyte includes at least two molecules that are interacting with one another. For example, biomolecular interactions, such as protein-protein interactions and RNA-protein interactions, play an important role in cellular biology and are increasingly targeted for pharmaceutical development; see, e.g., Lu et al., "Recent advances in the development of protein-protein interactions modulators: Mechanisms and clinical trials," Signal Transduction and Targeted Therapy 5(1): article no. 213 (2020), the entire contents of which are incorporated by reference herein. However, existing methods for detecting biomolecular interactions are complex and typically require affinity purification of a biomolecule of interest, followed by characterizing bound material through techniques such as mass spectrometry (proteins) or sequencing (RNA). The present proximity-induced tagmentation assay may be used to detect such interactions without the need for affinity purification, and instead using simple sequencing readout similar to that described with reference to FIG. 1. The assay for detecting molecular interactions may include a donor recognition probe with a recognition element that binds a target molecule (e.g., biomolecule) X; an acceptor recognition probe with a recognition element that binds a target molecule (e.g., biomolecule) Y, although optionally the target molecule may be bound to the oligonucleotide 132 thus eliminating the need for a recognition element; and a mock donor probe and a mock acceptor probe that both include either a non-specific recognition element or lack a recognition element/target. These mock probes provide for the measurement of non-specific tagmentation events that may be used as a control in analysis.

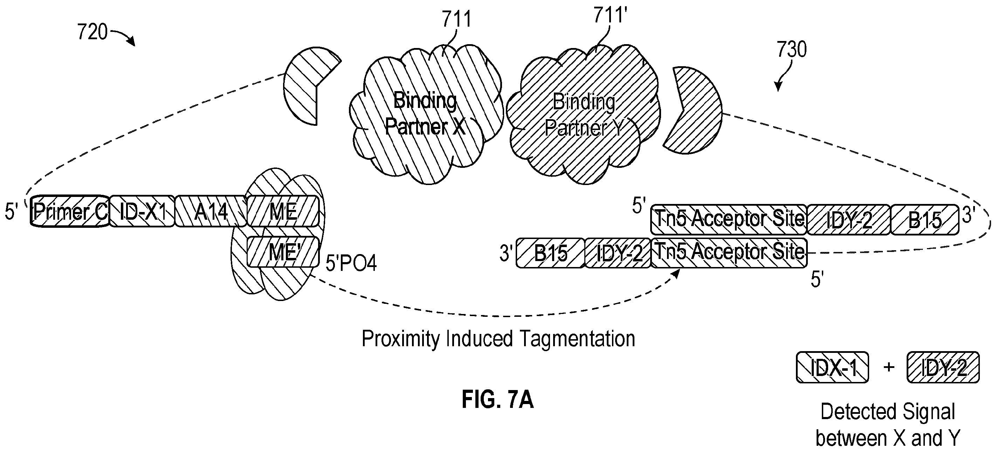

For example, FIGS. 7A-7C schematically illustrate example operations and compositions in a process flow for detecting molecular interactions using proximity-induced tagmentation. As illustrated in FIG. 7A, molecules 711 (X) and 711' (Y) in a sample are interacting with each other, e.g., are covalently or noncovalently coupled to one another. Donor recognition probe 720 specifically binds to molecule 711 and acceptor recognition probe 730 specifically binds to molecule 711', bringing the transposase of probe 720 into sufficient proximity to probe 730 to perform proximity-induced tagmentation in a manner such as described elsewhere herein. The reporter polynucleotide that is generated includes the barcode corresponding to molecule 711 (e.g., IDX-1) and the barcode corresponding to molecule 711' (e.g., IDY-1). Accordingly, the sequence of that polynucleotide indicates that molecules 711 and 711' were interacting with one another in sufficient proximity to bind both donor recognition probe and acceptor recognition probe 730.

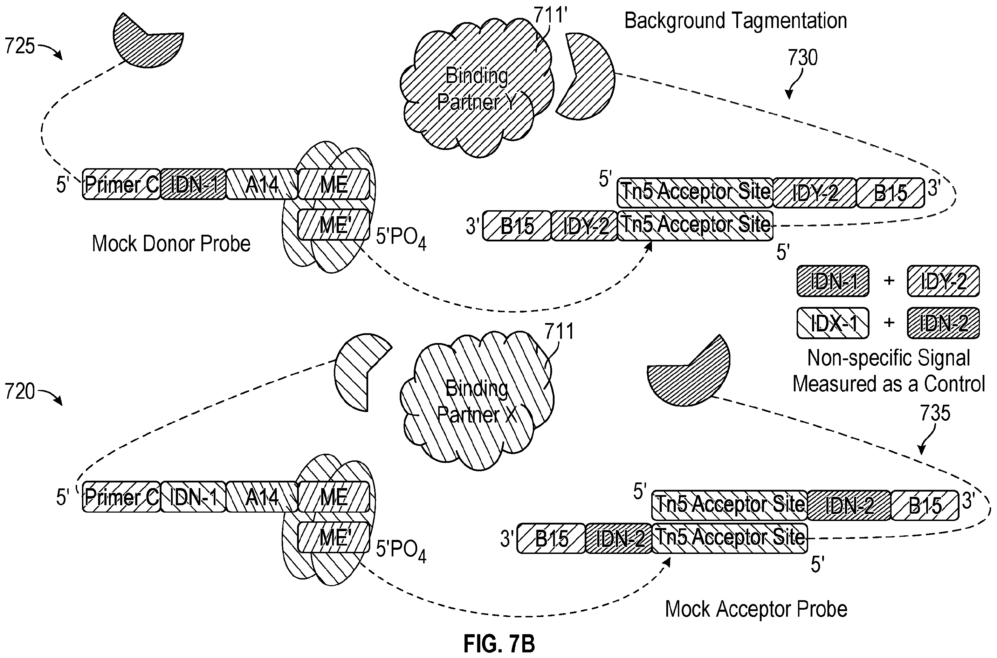

The sample may be incubated with a mixture of mock donor recognition probes 725 which do not specifically bind to molecules 711 or 711' with a distinguishable barcode "IDN-1" and acceptor recognition probes 730, and as may be seen in FIG. 7B, acceptor recognition probe 730 may specifically bind to molecule 711'. As a result of non-specific binding, mock donor recognition probe 725 may come into sufficient proximity to acceptor recognition probe 730 to perform proximity-induced tagmentation, generating a reporter polynucleotide that includes the barcodes IDN-1 and IDY-2. The sample (or another sample) may also be incubated with a mixture of donor recognition probes 720 and mock acceptor recognition probes 735 which do not specifically bind to molecules 711 or 711' and which include a distinguishable barcode "IDN-2". As may be seen in FIG. 7B, donor recognition probe 720 may specifically bind to molecule 711. As a result of non-specific binding, mock acceptor recognition probe 735 may come into sufficient proximity to donor recognition probe 720 to perform proximity-induced tagmentation, generating a reporter polynucleotide that includes the barcodes IDX-1 and IDN-2. The two reporter polynucleotides may be sequenced, from which the amount of background tagmentation in the proximity of molecules 711' and 711 respectively may be obtained. Such amounts may be compared to the amount specific tagmentation detected from the pairing of the pairing 711-711' obtained as described with reference to FIG. 7A as a control, e.g., so as to quantitate the amount of biomolecular interactions between molecules 711 and 711' within the sample. For example, the IDN signal will be indicative of background tagmentation occurring. More specifically, if the amount of IDN1-IDY2 and/or IDX1-IDN2 is high relative to the "real" signal of IDX1-IDY2, that would suggest the interaction between X and Y is not real. This may be measured as the fold difference of (IDX1-IDY2 signal)/(IDN signal) where the higher this value, the more confidence there is in there being an interaction.

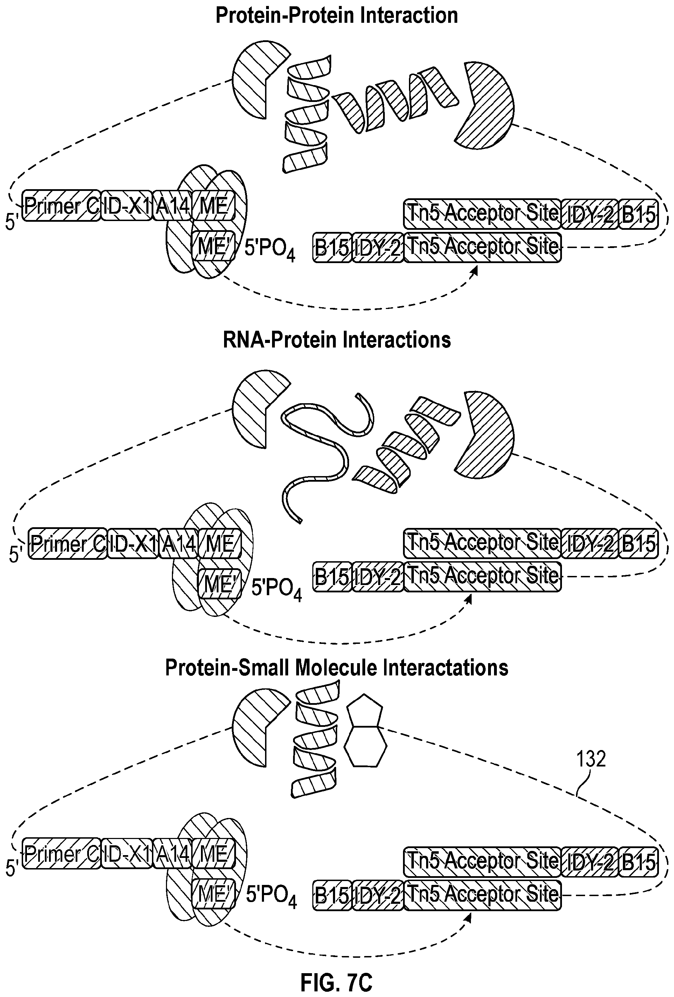

Using assays such as described with reference to FIGS. 7A-7B, a variety of molecular (e.g., biomolecular) interactions may be detected and quantified. For example, if the recognition elements of both the donor recognition probe and the acceptor recognition probe target proteins, protein-protein interactions will be detected, whereas if one of the recognition elements targets RNA and the other targets protein, then RNA-protein interactions will be detected. FIG. 7C schematically illustrates the use of proximity-induced tagmentation to detect protein-protein interactions, RNA-protein interactions, and protein-small molecule interactions. In the case of the protein-small molecule interaction illustrated in the lower panel of FIG. 7C, note that the small molecule is coupled to oligonucleotide 132 and is acted upon by the protein, accordingly the recognition element of the acceptor recognition probe may be omitted. Nonlimiting examples of biomolecules and corresponding recognition elements that may be used in the present assays are listed in Table 1:

| **Target Biomolecule** | **Example recognition elements** |
|---|---|
| Protein | Antibodies, aptamers, RNA, modified bases |
| RNA | rCas9, dCas13, RNA, ssDNA |
| ssDNA | dCas9, dCas13, RNA |
| Carbohydrate | Lectins |
| Peptide | Antibodies, aptamers |
| Small molecules | Proteins, aptamers |
| Lipids | Proteins, aptamers |
| Biotinylated biomolecule | Streptavidin |

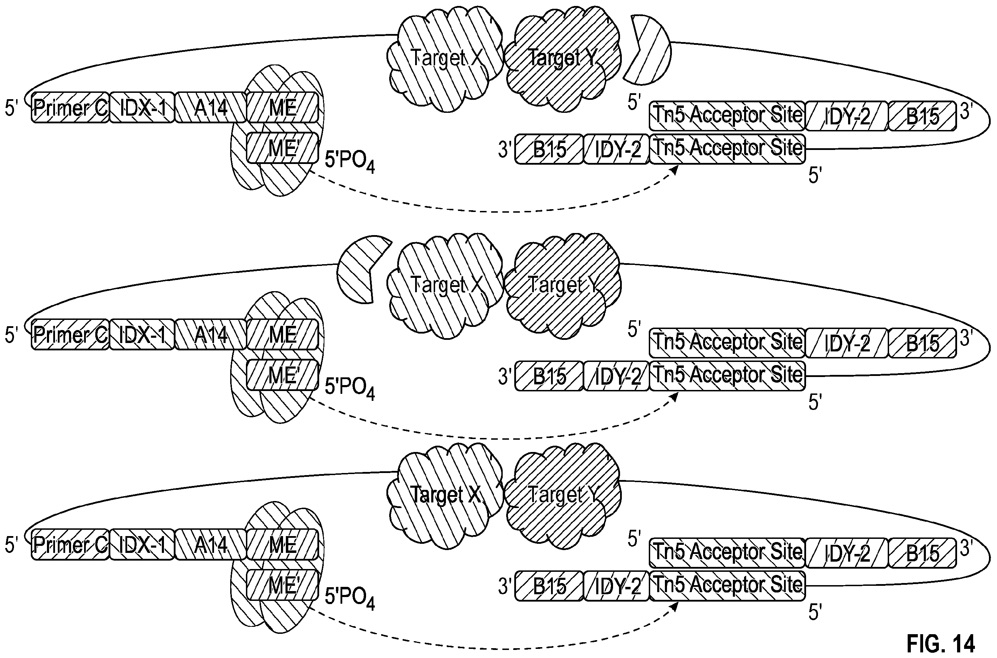

FIG. 14 illustrates additional examples in which proximity-induced tagmentation is used to detect molecular interactions in the absence of one or both recognition elements. Here, one or both of the recognition elements may be omitted because the transposome may be directly tethered to the target. The donor recognition probe may be attached to a first molecule (Target X), as illustrated in the top panel. The acceptor recognition probe may be attached directly to a second molecule (Target Y), as illustrated in the middle panel. Both the donor and the acceptor recognition probes may be attached directly to the molecules, as illustrated in the bottom panel.

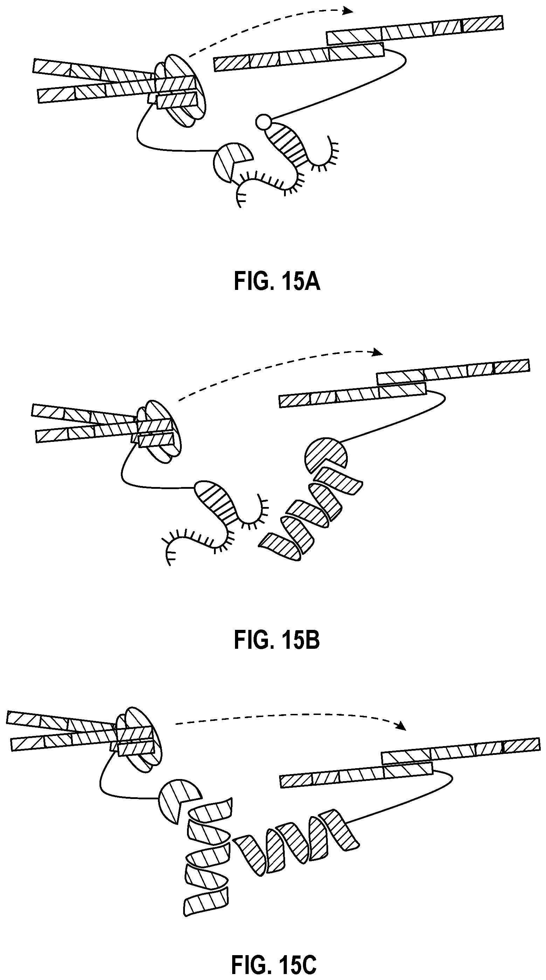

Other examples of biomolecules and interactions that may be evaluated when recognition probes are attached directly to a molecule of interest are illustrated in FIGS. 15A-15C. FIGS. 15A-15C schematically illustrate example operations and compositions in a process flow. FIG. 15A illustrates the detection of RNA modifications on a particular RNA target. FIGS. 15B and 15C illustrate the detection of molecular interactions using proximity-induced tagmentation. More specifically, FIG. 15A illustrates an acceptor recognition probe attached directly to an RNA modification for evaluating the presence of RNA molecules having said modification. FIG. 15B illustrates a donor recognition molecule attached directly to an RNA molecule of interest for evaluating interactions of said RNA molecule with a protein of interest. FIG. 15C illustrates an acceptor recognition molecule directly attached to a protein of interest for evaluating interactions of said protein with another protein of interest. In these examples, the RNA modification, RNA of interest, and protein of interest serve as recognition elements in the proximity-induced tagmentation assay. More examples of biomolecules that may be used as a recognition element, and the corresponding interactions that may be evaluated, are provided in Table 2:

| **Biomolecule used as Recognition Element** | **Example interactions** |
|---|---|
| Protein | Protein-protein (Figure 15C), Protein-RNA |
| RNA | Protein-RNA (Figure 15B), RNA-RNA |
| ssDNA | Protein-ssDNA |
| Modified nucleotide | Protein-nucleotide, presence of modification in RNA (Figure 15A), presence of modification in ssDNA |
| Carbohydrate | Protein-carbohydrate, RNA-carbohydrate |
| Small molecules | Protein-small molecule, RNA-small molecule |

Any mechanism for attaching a molecule of interest to a recognition probe may be used. For example, a protein of interest can be directly attached to a recognition probe by using a covalent attachment method (e.g. SNAP TAG). Additional attachment mechanisms can couple the donor or acceptor probe to nucleic acids via certain nucleotides (as described in Klocker et al. "Covalent labeling of nucleic acids," Chem Soc Rev. 49(23):8749-8773 (2020)), or certain nucleotide modifications (as described in Wang et al. "Antibody-free enzyme-assisted chemical approach for detection of N6-methyladenosine," Nat Chem Biol. 16(8):896-903 (2020) and Zhang et al. "Tet-mediated covalent labelling of 5-methylcytosine for its genome-wide detection and sequencing," Nat Commun. 4:1517 (2013)).

It will be appreciated that any suitable combination of recognition elements may be used to detect any suitable number of analytes, which optionally may be interacting with one another. Illustratively, a first molecule may include a first protein or first peptide; and a first recognition element may include a first antibody or a first aptamer that is specific to the first protein or first peptide. Or, for example, a first molecule may include a first target polynucleotide; and a first recognition element may include a first CRISPR-associated (Cas) protein that is specific to the first target polynucleotide. Or, for example, a first molecule may include a first carbohydrate; and a first recognition element may include a first lectin that is specific to the first carbohydrate. Or, for example, a first molecule may include a first biomolecule that is specific for the first recognition element. In examples in which an interaction between first and second molecules is being detected, the second molecule may include a second protein or second peptide; and the second recognition element may include a second antibody or a second aptamer that is specific to the second protein or second peptide. Or, for example, the second molecule may include a second target polynucleotide; and the second recognition element may include a second Cas protein that is specific to the second target polynucleotide. Or, for example, the second molecule may include a second carbohydrate; and the second recognition element may include a second lectin that is specific to the second carbohydrate. Or, for example, the second molecule may include a second biomolecule that is specific for the second recognition element.

As described elsewhere herein, the present donor recognition probes may include a recognition element coupled to a transposase and a first oligonucleotide (which may be referred to as a barcoded transposome), and indeed may include active transposome dimers although sometimes illustrated in simpler form. For example, an active transposome may carry two ME duplexes (which duplexes may be referred to elsewhere herein as annealed mosaic end transposon end sequences (ME, ME')), one ME duplex for each monomer of the transposase (e.g., Tn5). Any suitable method may be used to prepare the present donor recognition probes. FIGS. 8A-8C schematically illustrate example process flows for preparing donor recognition probes 120. In the example shown in FIG. 8A, each recognition element 121 carries one copy of oligonucleotide 122 to which a transposase 123 is loaded; the transposases of two such complexes then are dimerized to form the active transposome. Accordingly, donor recognition probe 120 illustrated in FIG. 8A may include two transposases, two first recognition elements, and two first oligonucleotides, wherein the two transposases form a dimer, each of the transposases being coupled to a corresponding one of the first recognition elements via a corresponding one of the first oligonucleotides.

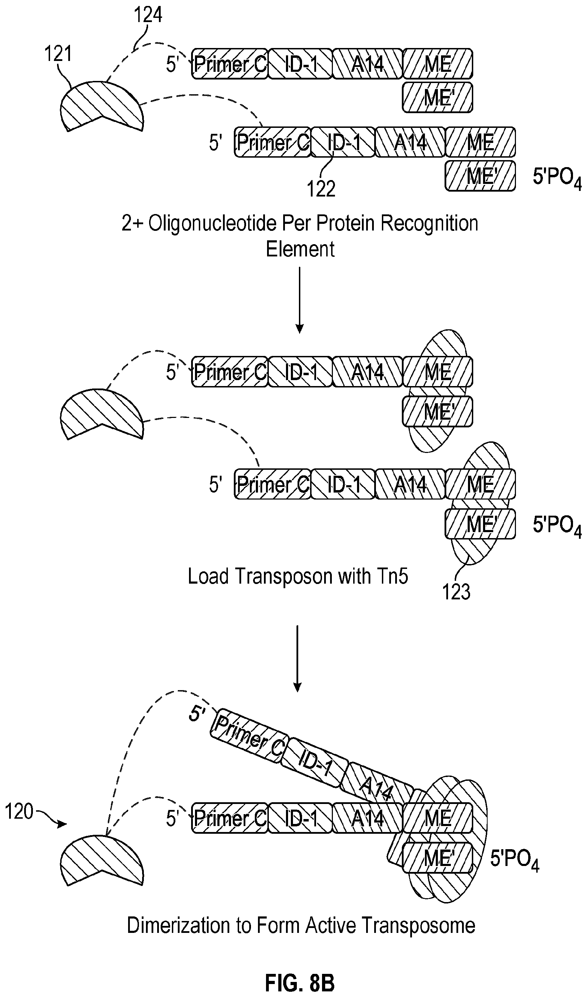

In another option, such as shown in FIG. 8B, two or more oligonucleotides 122 are coupled to a recognition element 121. Transposomes 123 are loaded to the respective oligonucleotides 122, and then are dimerized to form the active transposome. Accordingly, donor recognition probe 120 illustrated in FIG. 8B may include two transposases, one first recognition element, and two first oligonucleotides, wherein the two transposases form a dimer, each of the transposases being coupled to the one first recognition element via both of the first oligonucleotides.

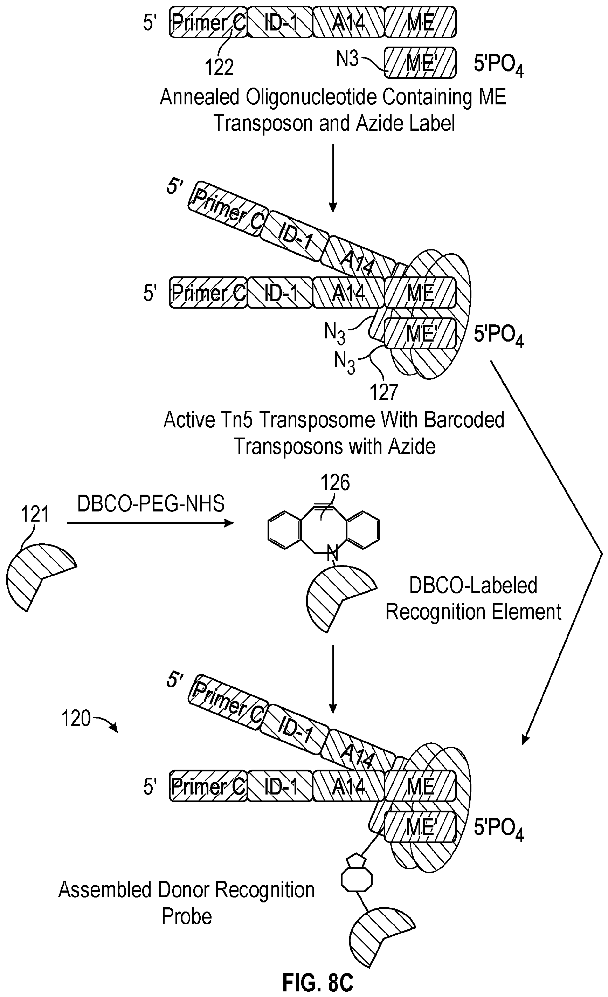

In another option, such as illustrated in FIG. 8C, an active transposome is formed before conjugation to the recognition element. For example, oligonucleotide 122 may be prepared, loaded into transposases 123, and the transposases dimerized to form the active transposome. Then, one or more recognition elements may be coupled to the active transposome. For example, recognition element 121 may be conjugated to a first moiety 126, and the oligonucleotides 122 or transposases 123 may be conjugated to a second moiety 127 that is reactive with the first moiety to form a bond. Illustratively, first moiety 126 may include a click chemistry moiety, such as dibenzocyclooctyne (DBCO), and second moiety 127 may include a complementary click chemistry moiety, such as an azide, that reacts with the first moiety to bond the recognition element 121 to oligonucleotide 122 or to transposase 123. In some examples, the recognition element 121 may be conjugated to DBCO or other suitable first moiety using NHS-PEG-DBCO in a manner such as described in Gong et al., "Simple method to prepare oligonucleotide-conjugated antibodies and its application in multiplex protein detection in single cells," Bioconjugate Chemistry 27(1): 217-225 (2016), the entire contents of which are incorporated by reference herein. In some examples, the oligonucleotide 122 may be conjugated to azide or other suitable second moiety using techniques known in the art. The active transposome may be assembled by incubating the synthetic oligonucleotide with the transposase (e.g., Tn5) enzyme. This transposase enzyme may be introduced as a monomer, or an obligate dimeric form of the enzyme with a peptide linker attaching two monomeric subunits may be used in a manner such as described in Blundell-Hunter, "Transposase subunit architecture and its relationship to genome size and the rate of transposition in prokaryotes and eukaryotes," Nucleic Acids Research 46(18): 9637-9646 (2018), the entire contents of which are incorporated by reference herein. The assembled transposome including the dimerized transposases and synthetic oligonucleotides then may be incubated with the recognition element(s), resulting in reaction of the first moiety 126 with the second moiety 127 and therefore covalent coupling of the transposome to the antibody, forming donor recognition probe 120 as illustrated in FIG. 8C. Accordingly, donor recognition probe 120 illustrated in FIG. 8C may include two transposases, one first recognition element, and two first oligonucleotides, wherein the two transposases form a dimer, at least one of the transposases being coupled to the one first recognition element via a covalent linkage.

Regardless of the particular manner in which the present donor recognition probes and acceptor recognition probes are prepared, and of the particular analytes which are to be detected, it may be useful to promote specificity of the recognition elements by reducing background interactions. For example, a long incubation time may be used to drive binding between the recognition elements and the analytes. During this incubation, there can be some non-specific binding and tagmentation of the donor recognition probe's transposome to the acceptor recognition probe's acceptor site 134 in the absence of target binding. These non-specific interactions may be expected to occur randomly rather than between pairs of acceptor and donor recognition probes that are specific for the same analyte. Accordingly, reporter polynucleotides with sequences including non-corresponding barcodes may be filtered out using bioinformatics in a manner such as described with reference to FIG. 1. The level of this type of background signal also may be monitored as a metric for assay performance.

However, having too many of these background products may interfere with sensitivity and/or may be addressed by increasing sequencing depth. So as to reduce background product formation further, any of several parameters of the assay may be adjusted. This may include concentrations of the donor recognition probes 120, concentrations of the acceptor recognition probes 130, incubation time, incubation temperature, and/or buffer conditions (e.g., addition or removal of Mg++). Additionally, or alternatively, the acceptor recognition probe's acceptor site 134 may be shortened or modified (e.g., by methylation) so as to reduce the non-specific affinity of the donor and acceptor probes 120, 130. Additionally, or alternatively, a non-hyperactive variant of the transposase (e.g., of Tn5) may be used to reduce the strength of DNA binding by the transposome in a manner such as described in Wiegand et al., "Characterization of two hypertransposing Tn5 mutants," J. Bacteriol. 174(4): 1229-1239 (1992), the entire contents of which are incorporated by reference herein.

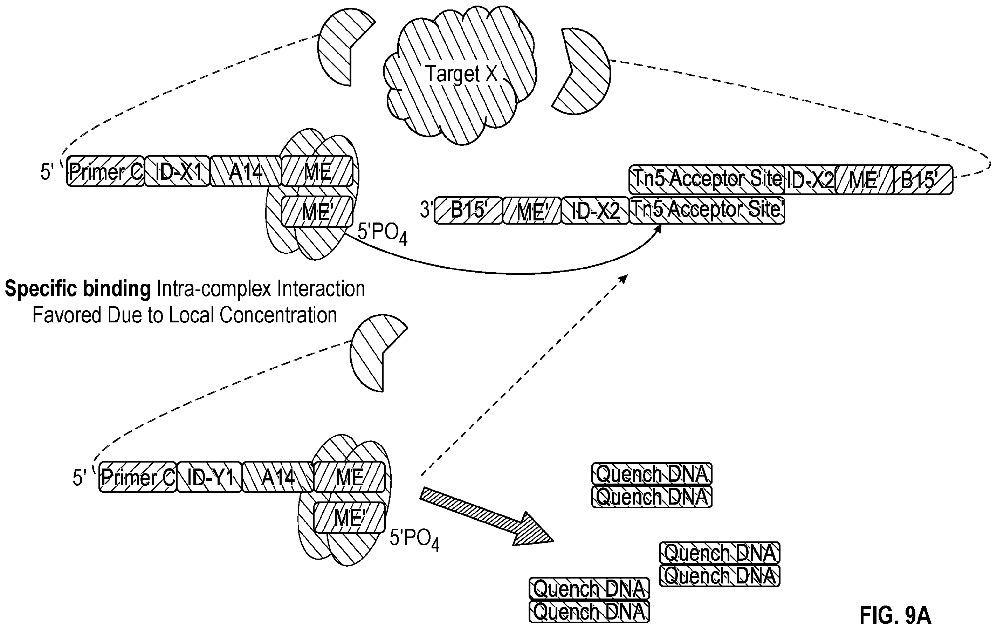

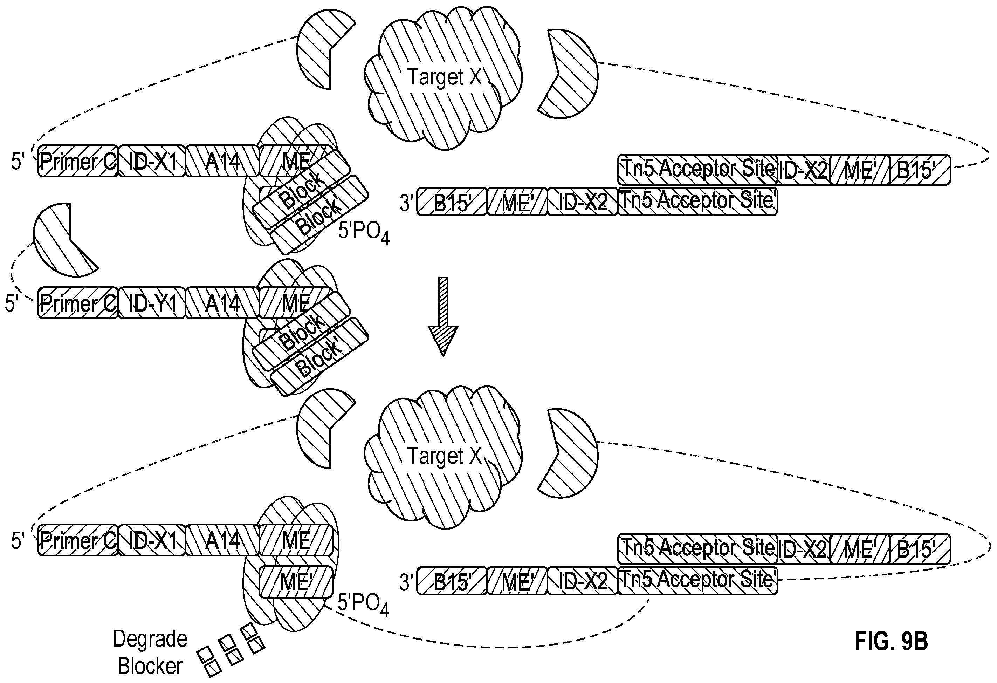

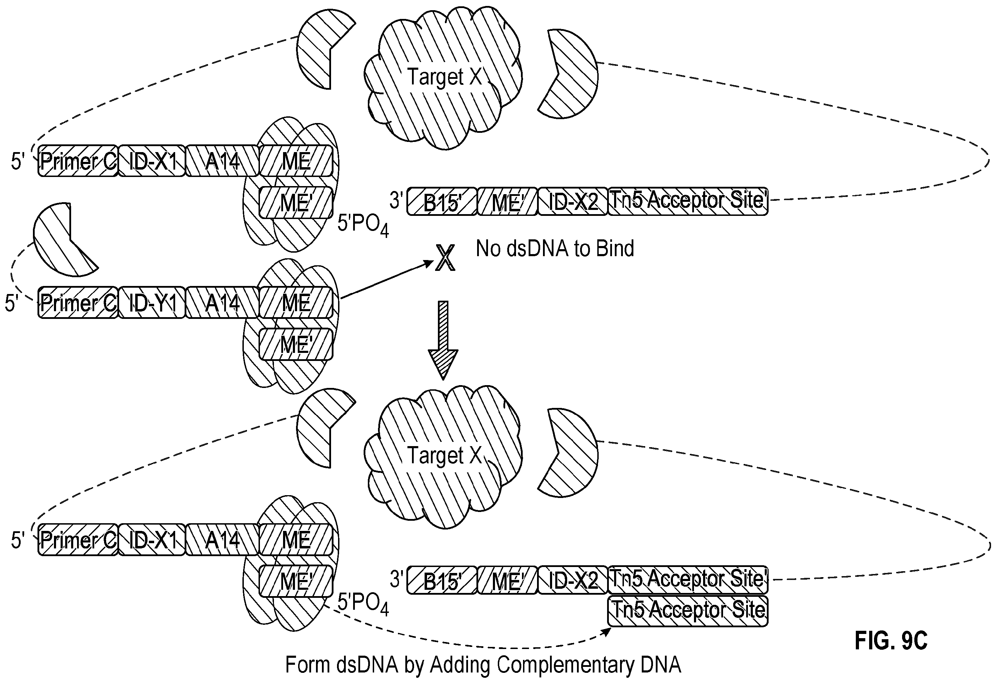

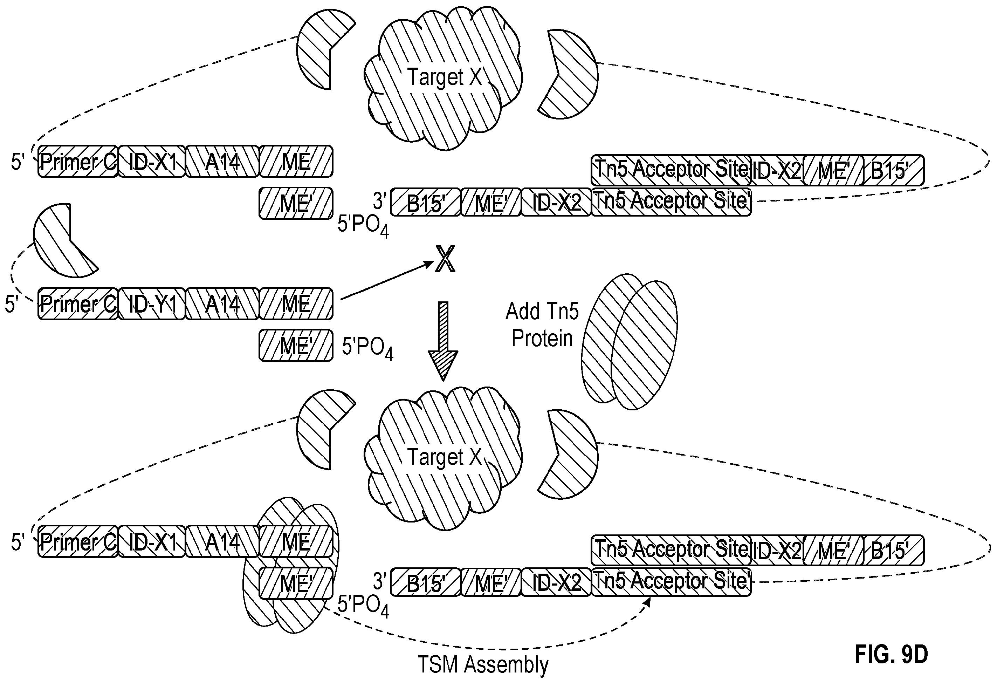

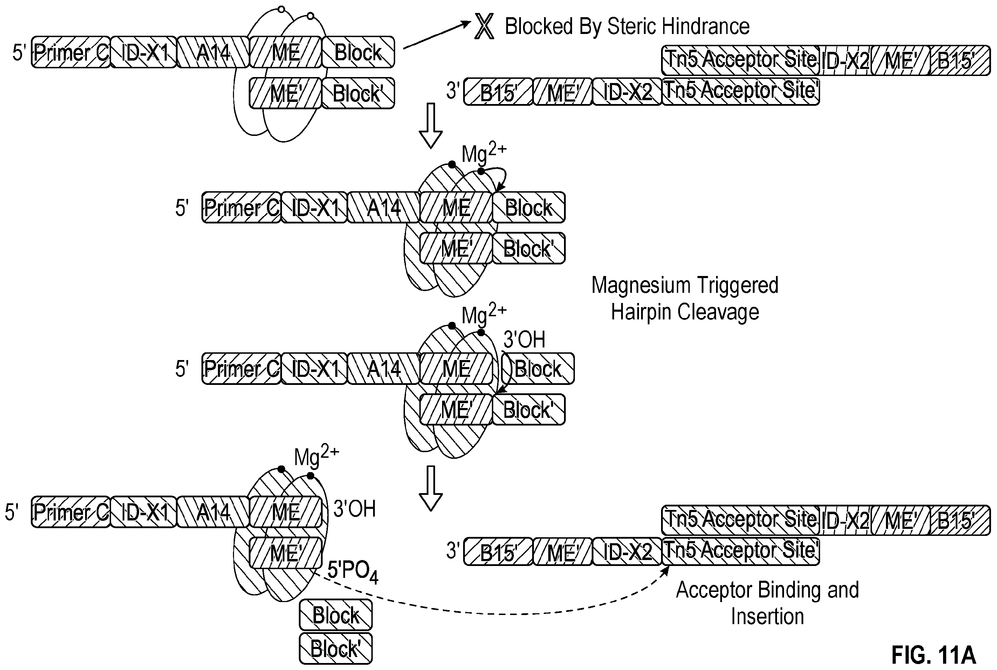

Such mitigations, such as removing magnesium, may reduce or inhibit premature enzymatic cleavage by the transposome, but may not fully prevent non-specific DNA binding; see, e.g., Amini et al., "Haplotype-resolved whole-genome sequencing by contiguity-preserving transposition and combinatorial indexing," Nat. Genet. 46: 1343-1349 (2014), the entire contents of which are incorporated by reference herein. So as to further reduce background product formation, additional components or changes to the workflow may be used. For example, FIGS. 9A-9E schematically illustrate example compositions and operations for reducing background tagmentation during proximity-induced tagmentation. FIG. 9A illustrates an option in which a dsDNA quencher molecule, which does not have priming sites for amplification, is used to compete against non-specific interactions. Specific interactions may be less affected because they are brought into proximity by the presence of an analyte, so the concentration of quencher may be set at a level that reduces background product formation while having no or little impact on specific interactions. FIG. 9B illustrates an option in which transposomes are pre-bound to a blocker that may be degraded after washing away any unbound donor recognition probes. Options for degradable transposome blocking include a DNA blocker including uracil (USER degradable), a blocker with RNA bases (RNAse degradable), or additional DNA at the 3' end of the ME sequence (cleaved away by the transposome in the presence of Mg++). Further details regarding the blocker are provided below with reference to FIGS. 11A-11C. FIG. 9C illustrates an option in which transposase acceptor site 134 is initially single-stranded and, shortly before tagmentation, a complementary oligonucleotide is introduced that generates the dsDNA target for transposase (e.g., Tn5) binding. FIG. 9D illustrates an option in which the transposome is assembled in situ. For example, the donor recognition probes may not include the transposases when binding to the analytes, and the transposases are added after the donor recognition probes bind to the analytes. The transposases assemble onto the blunt, annealed ME ends (with or without magnesium) and then bind to the oligonucleotide of the adjacent acceptor probe. Note that such option may be used with sufficient donor recognition probes (in excess of the acceptor recognition probes), because it may be useful to increase the number of acceptor-analyte complexes to be able to form a complex with the correct donor recognition probe.

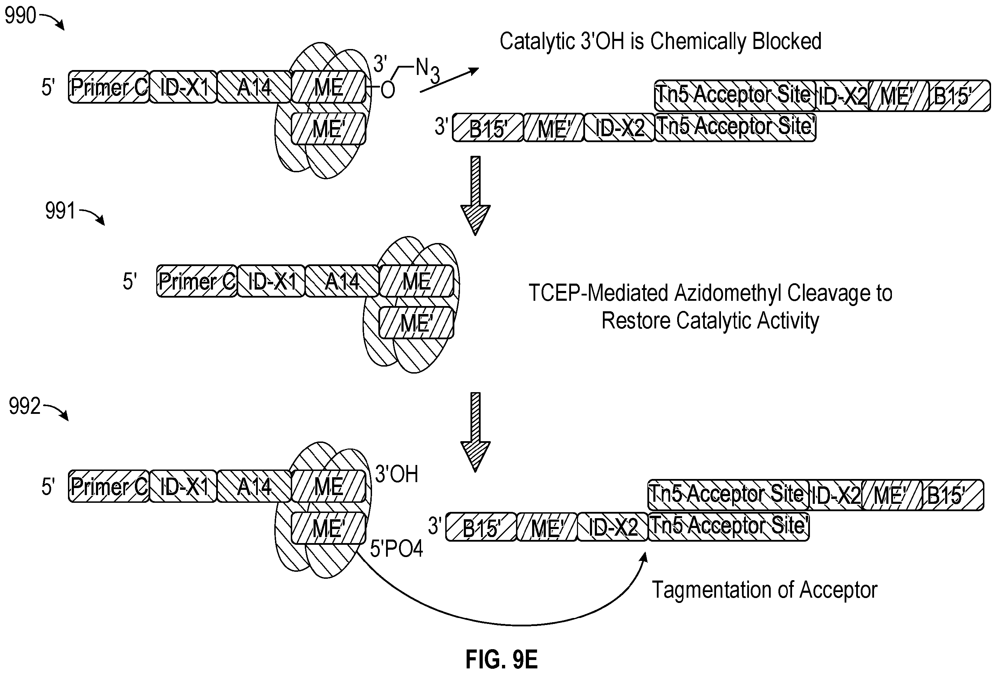

In the example illustrated in FIG. 9E, a chemical blocker may be incorporated into the transposon sequence of the donor recognition probe to reduce or inhibit background tagmentation. For example, Tn5 requires a 3' hydroxy group on the transposon to carry out tagmentation, and thus providing a blocking group at the 3' hydroxy group may reduce or inhibit tagmentation activity in a manner such as illustrated at operation 990 of FIG. 9E. A suitable reagent or reagents then may be used to remove the chemical blocker so that the transposase may tagment the oligonucleotide of the acceptor recognition probe in a manner such as illustrated at operation 991 of FIG. 9E. The transposase then may use the deblocked 3' hydroxyl group to tagment the acceptor probe in a manner such as illustrated at operation 992 of FIG. 9E. In the nonlimiting example illustrated in FIG. 9E, the 3' blocking group is azidomethyl group, which is cleaved using tris(2-carboxyethyl)phosphine) (TCEP) under mild conditions to generate a 3' hydroxyl group that permits Tn5 to tagment the acceptor probe in a manner such as described elsewhere herein. However, many different chemically cleavable blockers (and associated reagents) may be used, for example such as described in Chen et al., "The history and advances of reversible terminators used in new generations of sequencing technology," Genomics, Proteomics & Bioinformatics 11(1): 34-40 (2013), the entire contents of which are incorporated by reference herein.

In examples such as described with reference to FIGS. 9A-9E, the block (e.g., quencher, blocker, lack of double-stranded DNA to tagment, and/or the lack of transposase) may be used to provide sufficient time for the correct complexes to form with specificity between recognition elements and respective analytes prior to tagmentation. After sufficient time has passed, then the transposase may be activated, and the pre-formed complexes may be expected to react more quickly than non-specific interactions.

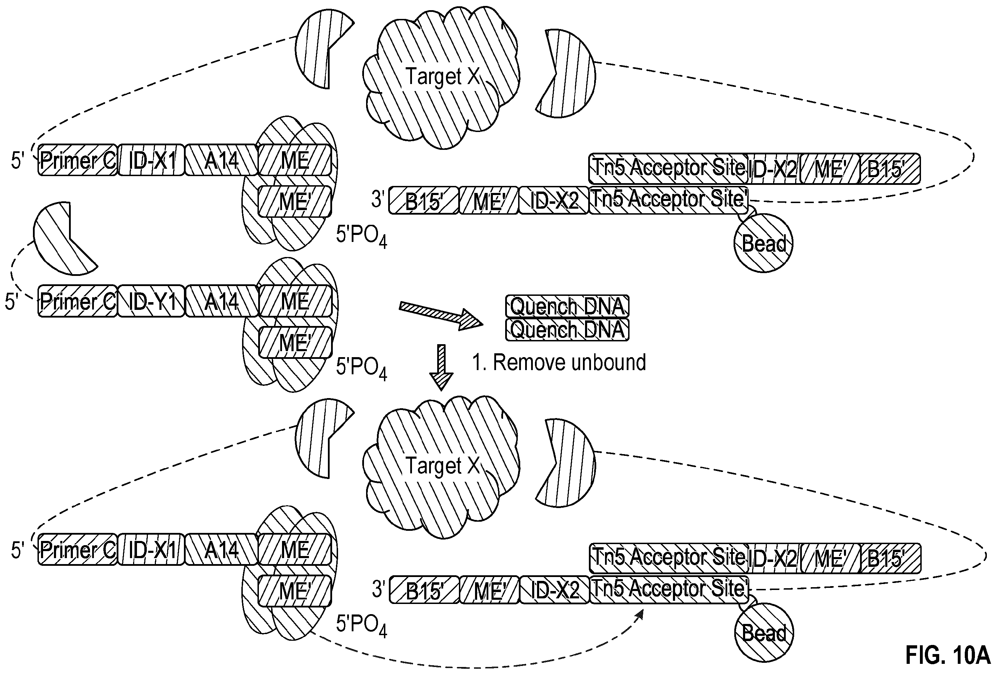

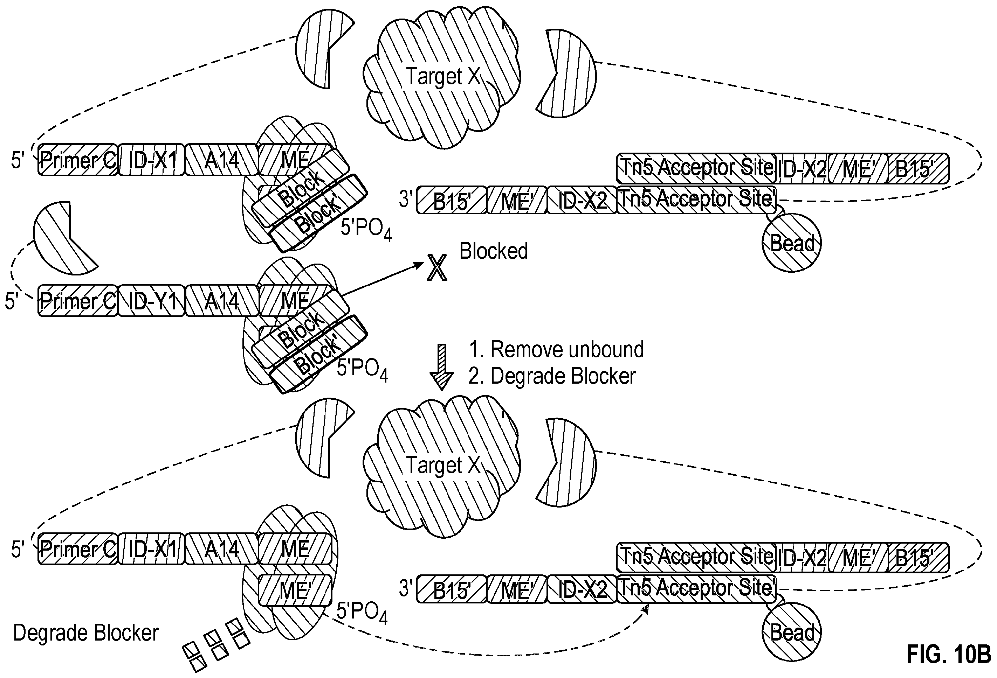

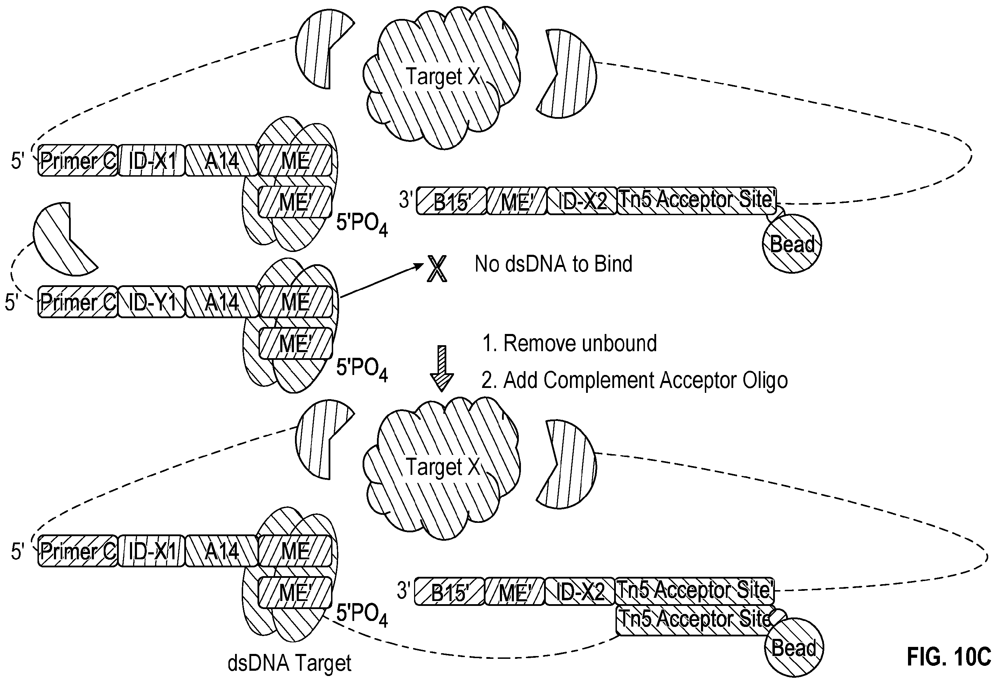

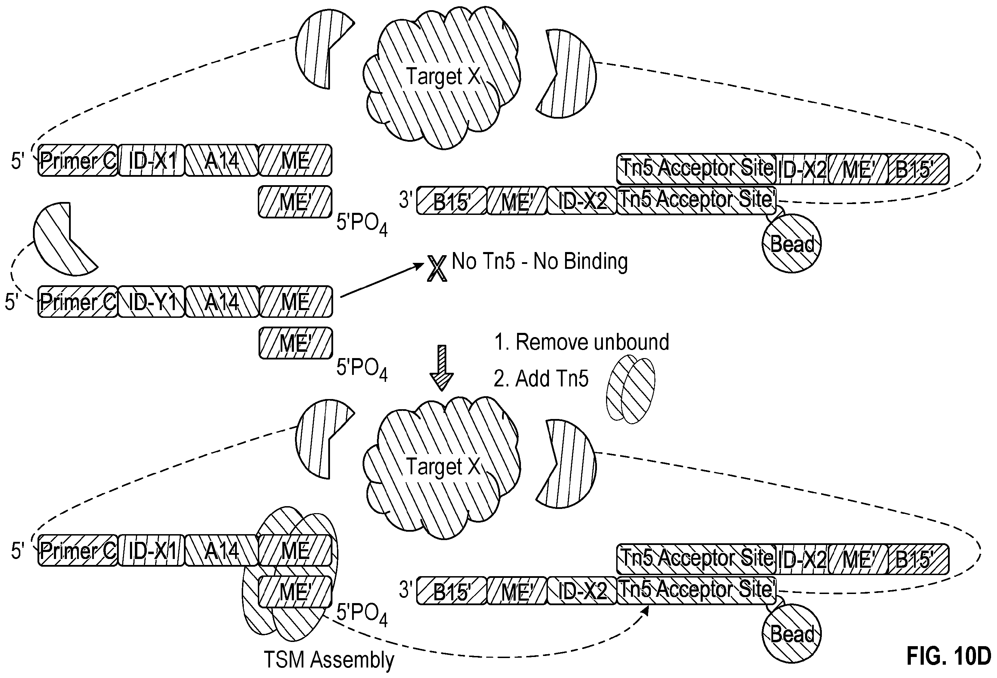

In some examples, substrates, such as beads, may be used to further reduce background product formation. For example, FIGS. 10A-10D schematically illustrate additional example compositions and operations for reducing background tagmentation during proximity-induced tagmentation. The examples illustrated in FIGS. 10A-10D are similar to those described with reference to FIGS. 9A-9D, but include an extra bead wash that removes unbound probes. More specifically, the acceptor recognition probe may be coupled to a substrate, which pulls down the analyte to which that probe and the corresponding donor recognition probe are coupled; any unbound donor recognition probes may be washed away before the block is removed or the transposase is otherwise activated. Alternatively, the donor recognition probe may be coupled to a substrate, which pulls down the analyte to which that probe and the corresponding acceptor recognition probe are coupled; any unbound acceptor recognition probes may be washed away before the block is removed or the transposase is otherwise activated. FIG. 10A illustrates an option in which a dsDNA quencher molecule, which does not have priming sites for amplification, is used to compete against non-specific interactions. FIG. 10B illustrates an option in which transposomes are pre-bound to a blocker that may be degraded after washing away any unbound donor recognition probes. FIG. 10C illustrates an option in which transposase acceptor site 134 is initially single-stranded and, shortly before tagmentation, a complementary oligonucleotide is introduced that generates the dsDNA target for transposase (e.g., Tn5) binding. FIG. 10D illustrates an option in which the transposome is assembled in situ. It will be appreciated that the operations described with reference to FIG. 9E similarly may be adapted for use with a bead. In the examples illustrated in FIG. 10A-10D, rather than relying on the pre-formed complex reacting more quickly than non-specific interactions, different buffers (e.g., with Tween or other mild detergents) may be used to remove non-specifically bound donor recognition probes prior to transposome unblocking or activation.

FIGS. 11A-11C schematically illustrate additional example compositions and operations for reducing background tagmentation during proximity-induced tagmentation, e.g., blocking related examples such as described with reference to FIGS. 9B and 10B. FIG. 11A illustrates an example using a magnesium-activated blocker. During assembly of an active transposome *in vivo*, the ME sequence is part of a longer piece of DNA, so there can be additional bases after the ME sequence. This also works for *in vitro* reactions; for example, the transposome may be assembled with additional DNA past the ME, e.g., in a manner such as described in Gradman et al., "A bifunctional DNA binding region in Tn5 transposase," Molecular Microbiology 67(3): 528-540 (2008), the entire contents of which are incorporated by reference herein. As provided herein, the additional bases may include purely DNA or may include a nick prior to the ME region that is expected to improve transposome formation. The additional bases may occupy the non-specific DNA binding pocket, and thus may need to be cleaved off before the transposome may bind target DNA (e.g., 134). Because this cleavage requires magnesium (Mg++), a "magnesium-activated" transposome is one assembled with this additional DNA at the end of oligonucleotide 122. Once magnesium is added, the transposase (e.g., Tn5) may cleave off the additional bases and be able to bind and tagment oligonucleotide 132.

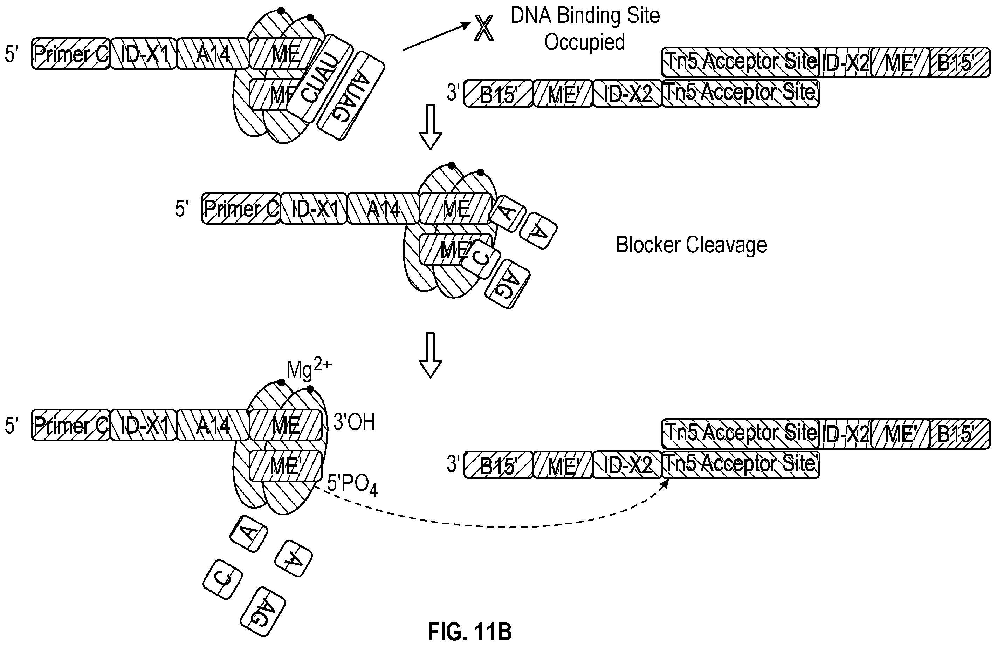

FIG. 11B illustrates a degradable blocker, e.g., a short blocker that may occupy the non-specific DNA binding pocket of the transposome and includes degradable residues (e.g., uracil or RNA). Before magnesium is introduced, the blocker is degraded (e.g., with USER or RNAse), allowing the transposome to bind target dsDNA. Then magnesium may be added to the reaction, to allow for proximity-induced tagmentation.

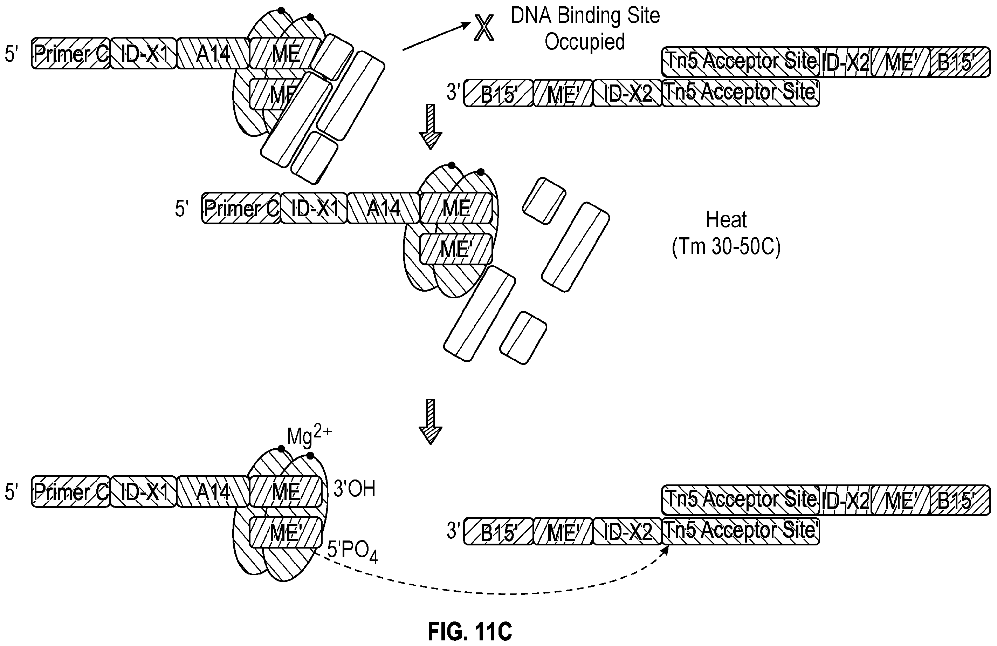

FIG. 11C illustrates a heat sensitive blocker, which may be used similarly as a degradable blocker but includes a short DNA fragment with several nicks. These nicks make the molecule more susceptible to melting and separating into single-stranded DNA at a relatively low temperature (e.g., 30-50°C). The melting temperature of the blocker may be lower than the melting temperature of the transposase acceptor site 134. After the analyte binding incubation (<30°C), the reaction may be warmed to approximately the melting temperature of the blocker and lower than the melting temperature of the transposase acceptor site 134. This allows the transposome to bind the transposase acceptor site 134. Then magnesium may be added to the reaction to allow for tagmentation.

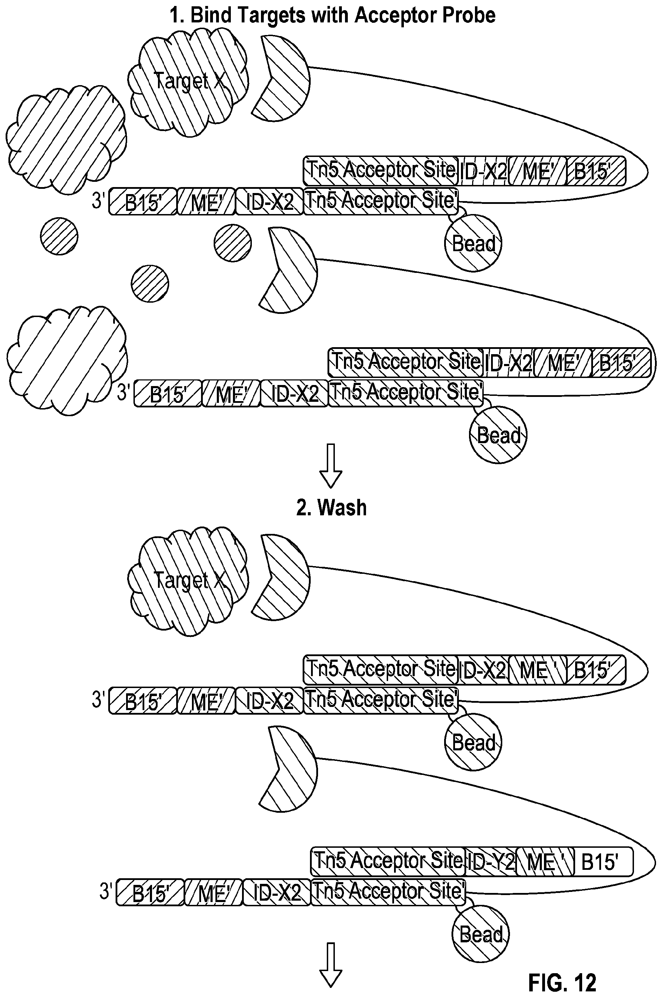

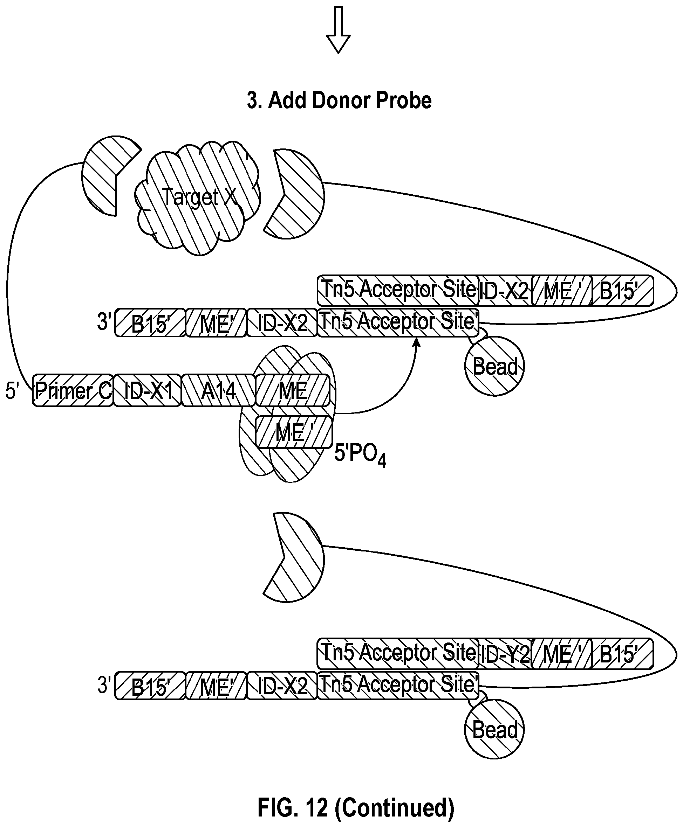

Other types of cleanup may be used after binding to provide for complex sample types. For example, some sample types may have a relatively high level of contaminants that would affect the assay. To assay those types of samples, a wash step may be used similar to that described with reference to FIGS. 10A-10D. More specifically, FIG. 12 schematically illustrates example compositions and operations for reducing contaminants during proximity-induced tagmentation. The acceptor recognition probe may be coupled to a substrate, which pulls down the analyte to which that probe; any unbound acceptor recognition probes - and any contaminants - may be washed away before the donor recognition probes are added. Alternatively, the donor recognition probe may be coupled to a substrate, which pulls down the analyte to which that probe; any unbound donor recognition probes - and any contaminants - may be washed away before the acceptor recognition probes are added. The proximity-induced tagmentation optionally may be further controlled in a manner such as described elsewhere herein, e.g., with reference to FIGS. 10A-10D. Additionally, in examples such as described with reference to FIG. 12, the volume of the reaction may be reduced. For example, after the first incubation and wash, the bound analytes may be resuspended in a smaller volume for the second incubation. Concentrating the reaction may speed up probe-analyte binding and improve sensitivity for low abundance analytes.

In examples such as described with reference to FIGS. 10A-10D and 12, the acceptor recognition probe or donor recognition probe may be coupled to the substrate in any suitable manner. Illustratively, the acceptor recognition probe or donor recognition probe may include a biotin handle, which binds to a streptavidin bead.

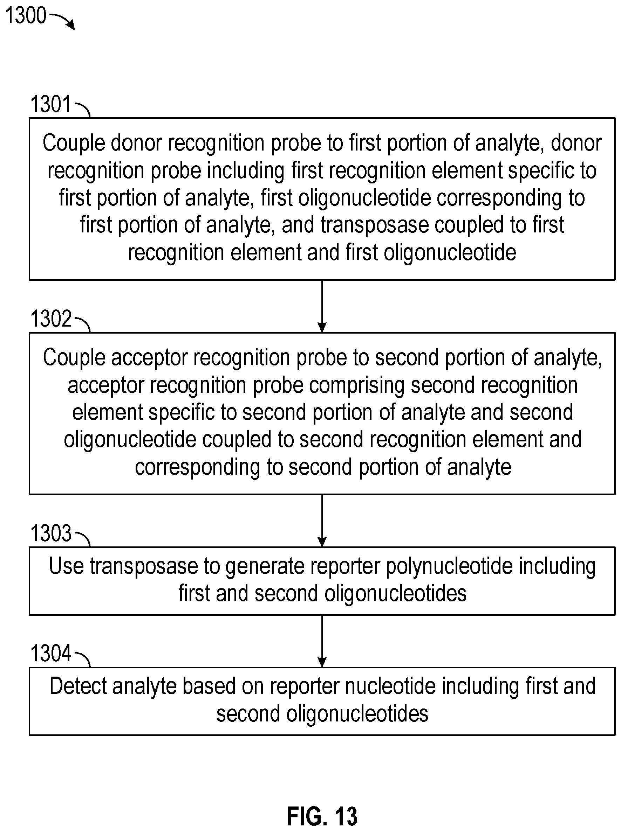

Accordingly, some examples herein provide for inhibiting activity of the transposase while specifically coupling the donor recognition probe to the first portion of the analyte and while specifically coupling the acceptor recognition probe to the second portion of the analyte, e.g., as described with reference to FIGS. 9A-9E and 10A-10D. The activity of the transposase is inhibited using a first condition of a fluid. For example, the first condition of the fluid may include at least one of (i) presence of a sufficient amount of EDTA to inhibit activity of the transposase and (ii) absence of a sufficient amount of magnesium ions for activity of the transposase. Additionally, or alternatively, the activity of the transposase may be inhibited using a dsDNA quencher, e.g., as described with reference to FIGS. 9A and 10A. Additionally, or alternatively, the activity of the transposase may be inhibited by associating a blocker with the transposase, e.g., as described with reference to FIGS. 9B, 10B, and 11A-11C. Additionally, or alternatively, the activity of the transposase may be inhibited by the second oligonucleotide being single stranded, e.g., as described with reference to FIGS. 9C and 10C. Additionally, or alternatively, the activity of the transposase may be promoted before using the transposase to generate the reporter polynucleotide, for example using a second condition of the fluid. Illustratively, the second condition of the fluid may include presence of a sufficient amount of magnesium ions for activity of the transposase. Additionally, or alternatively, the activity of the transposase may be promoted by degrading the blocker, e.g., as described with reference to FIGS. 9B, 10B, and 11A-11C. Additionally, or alternatively, the activity of the transposase may be promoted by annealing a third oligonucleotide to the second oligonucleotide to form a double-stranded polynucleotide, e.g., as described with reference to FIGS. 9C and 10C.FIG. 13 illustrates an example flow of operations in a method for detecting analytes using proximity-induced tagmentation. Method 1300 illustrated in FIG. 13 may include coupling a donor recognition probe to a first portion of the analyte (operation 1301). The donor recognition probe may include a first recognition element specific to the first portion of the analyte, a first oligonucleotide corresponding to the first portion of the analyte, and a transposase coupled to the first recognition element and the first oligonucleotide. For example, donor recognition probe 120 may be configured in a manner such as described with reference to FIGS. 1, 2, 8A, 8B, or 8C. Method 1300 also may include coupling an acceptor recognition probe to a second portion of the analyte (operation 1302). The acceptor recognition probe may include a second recognition element specific to the second portion of the analyte and a second oligonucleotide coupled to the second recognition element and corresponding to the second portion of the analyte. For example, acceptor recognition probe 130 may be configured in a manner such as described with reference to FIGS. 1 or 3. Method 1300 may include using the transposase to generate a reporter polynucleotide comprising the first and second oligonucleotides (operation 1303). For example, the transposase may perform proximity-induced tagmentation in a manner such as described with reference to FIGS. 1, 4A-4C, 5, 6, 7A, or 7C. The proximity-induced tagmentation optionally may be modulated in a manner such as described with reference to FIGS. 9A-9E, 10A-10D, 11, or 12. Method 1300 may include detecting the analyte based on the reporter polynucleotide comprising the first and second oligonucleotides (operation 1304). For example, the reporter polynucleotide may be sequenced, e.g., using sequencing-by-synthesis. Barcodes within the first and oligonucleotides may be used to detect the analyte to which the donor recognition probe and acceptor recognition probe had bound, e.g., to detect a molecule, a post-translational modification, or molecules interacting with one another.

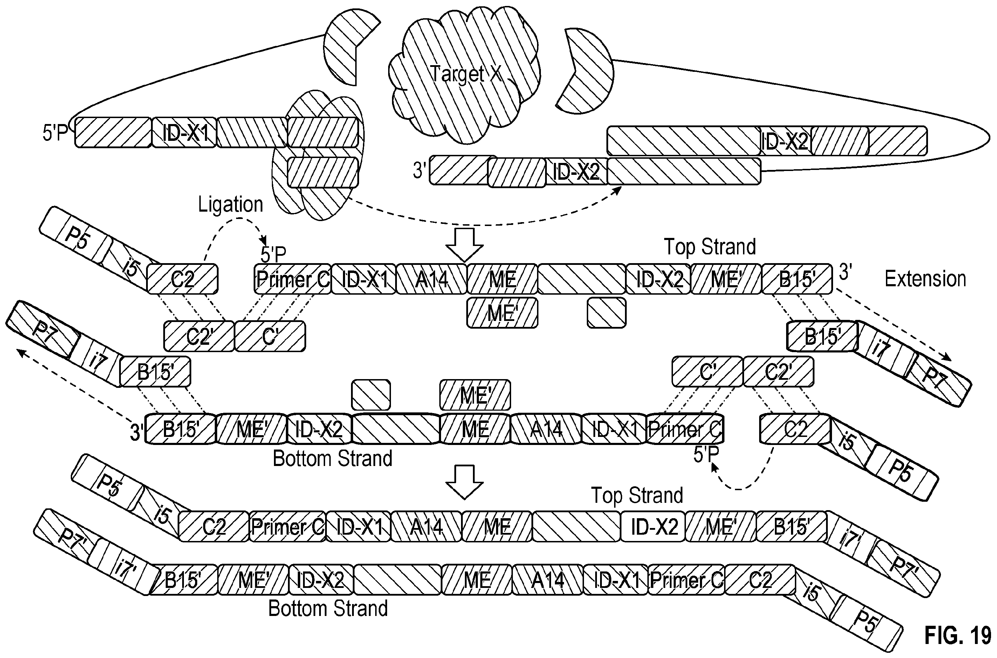

As an alternative to PCR-based amplification and sequencing techniques, other techniques may be used to detect the analyte. For example, as illustrated in FIG. 19, after proximity-induced tagmentation, sample indexing primers may be added through ligation and polymerase extension to yield elongated reporter polynucleotide that include both sample indexes and the barcodes corresponding to recognition elements, and which may be sequenced to identify the analyte.

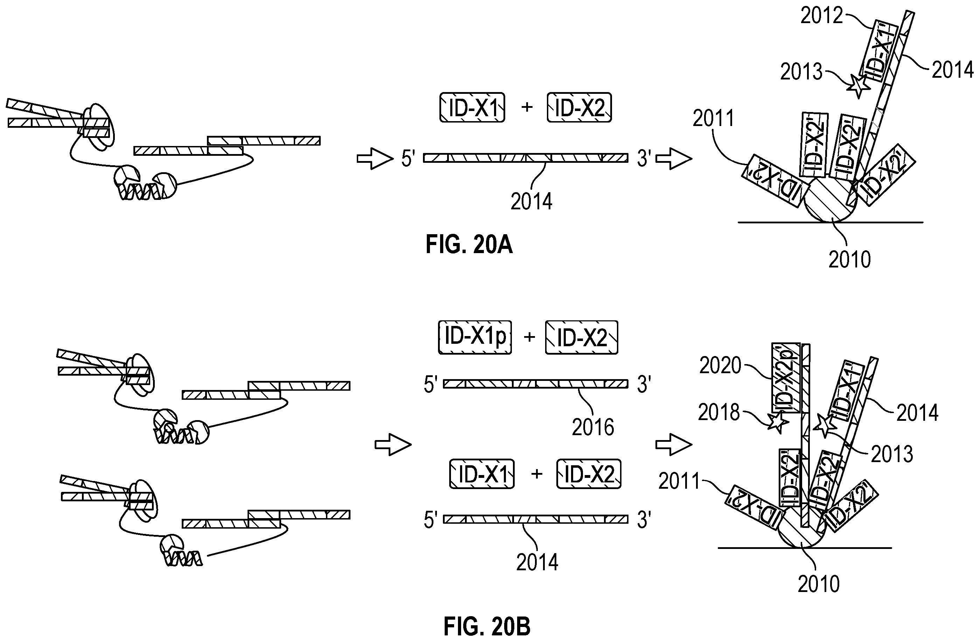

Another option for detecting the presence of an analyte is to use a bead array. as illustrated in FIGS. 20A-20B. FIG. 20A depicts proximity-induced tagmentation on a target protein, where the resulting reporter polynucleotide 2014 includes barcodes ID-X1 and ID-X2. Bead 2010 may include one or more capture probes 2011, designed to hybridize specifically to one of barcodes ID-X1 and ID-X2. The sample may include a detection probe 2012, labeled with a fluorophore 2013, and designed to hybridize specifically to the other of barcodes ID-X1 and ID-X2. After the sample is incubated to facilitate hybridization, the sample may be washed to remove reporter polynucleotides and detection probes that are not bound to the beads. Presence of the reporter polynucleotide may then be assessed by detecting and quantifying fluorescence from the fluorophores, e.g. using a suitable imaging camera and detection circuit in a manner similar to that described in International Publication No. WO 2021/074087, the entire contents of which are incorporated by reference herein.

As illustrated in FIG. 20B, more than one analyte may be assessed using this approach. For example, multiple species of the target analyte, e.g. analytes with post-translation modifications, nucleotide modifications, or the like may be assessed. Illustratively, a sample may include reporter polynucleotide 2014, produced from tagmentation on an analyte, as well as a second reporter polynucleotide 2016, produced from tagmentation on a modified form of the analyte. Bead 2010 may capture both reporter polynucleotides 2014, 2016 due to the presence of a common barcode, in this example ID-X2. However, a second detection probe 2020 is designed to hybridize specifically to the free barcode of the second reporter polynucleotide 2016. The second detection probe 2020 may be labeled with fluorophore 2018, which provides a different signal than fluorophore 2013. Therefore, when both analytes are present in the sample, they can be detected and quantified, relative to each other, by observing the total signal of fluorophores 2013 and 2018, as well as the ratio between the two signals, in a manner similar to that described in International Publication No. WO 2021/074087, the entire contents of which are incorporated by reference herein.

It will be appreciated that a sample may include any suitable number of different beads, each specific to a different reporter polynucleotide. Therefore, any number of analytes may be assessed in a sample, e.g. more than 100, more than 1,000, more than 10,000, more than 100,000, or more than 1,000,000.

The beads 2010 can be coupled to a surface, e.g., immobilized to a surface within a flow cell. In some examples, such coupling of beads 2010 to a surface may be performed before the reporter polynucleotides 2014 are coupled to the beads; for example, a solution including reporter polynucleotides 2014 may be flowed over the beads coupled to the surface, and the beads may capture from the solution the reporter polynucleotides to which those beads are specific. In other examples, such coupling of beads 2010 to a surface may be performed after the reporter polynucleotides 2014 are coupled to the beads; for example, a solution including reporter polynucleotides 2014 may be mixed with a solution including beads 2010 resulting in respective couplings between beads 2010 and the reporter polynucleotides 2014 to which those beads are specific, and the beads subsequently may be coupled to a surface, for example using bioorthogonal conjugation chemistries such as copper(I)-catalyzed click reaction (between azide and alkyne), strain-promoted azide-alkyne cycloaddition (between azide and DBCO (dibenzocyclooctyne), hybridization of an oligonucleotide to a complementary oligonucleotide, biotin-streptavidin, NTA-His-Tag, or Spytag-Spycatcher, charge-based immobilization such as amino-silane or poly-lysine, or non-specific such as with a polymer-coated surface.

Fluorophores may also be coupled to respective reporter polynucleotides at any suitable time during the assay. For example, fluorophore 2013 may be coupled to the reporter polynucleotide 2014 after the analyte is captured by the reporter polynucleotide 2014, before the reporter polynucleotide 2014 is coupled to the bead 2010, or after the reporter polynucleotide 2014 is coupled to the bead 2010.

In additional examples, the detection probes may be removed, e.g. by dehybridization, and further analyzed by sequencing by synthesis or other suitable method.

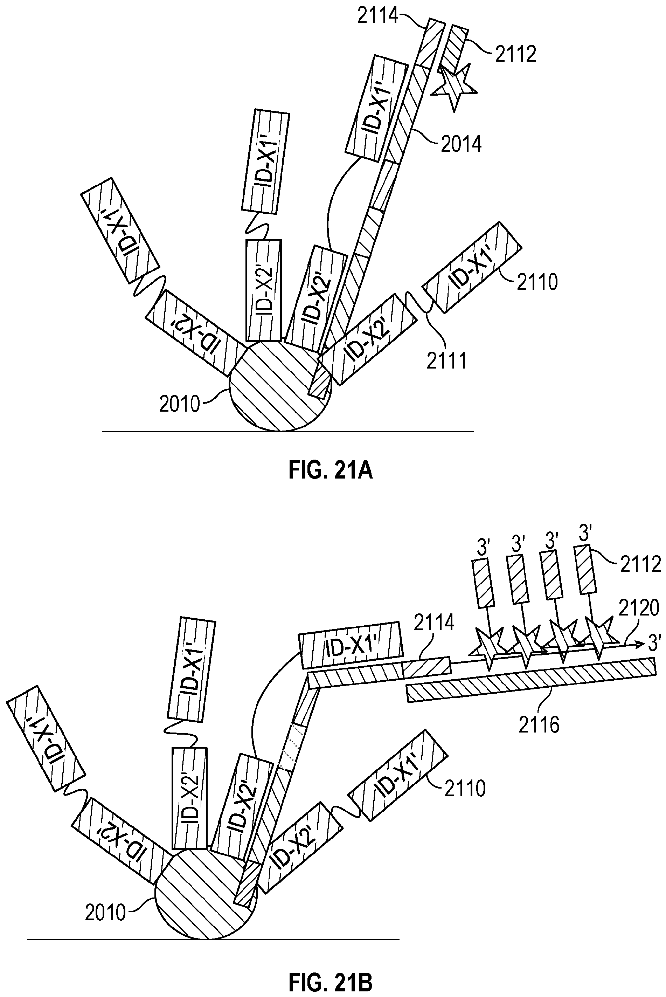

FIGS. 21A-21B illustrate an example in which both reporter polynucleotide barcodes are used for hybridization to the bead array. For example, bead 2010 includes capture probes 2110 which include two hybridization sites, which specifically bind to both ID-X1 and ID-X2 barcodes on reporter polynucleotide 2014. The two hybridization sites are separated by a spacer 2111 to reduce steric constraints. By providing two hybridization sites, capture probe 2110 has increased specificity, such that undesired reporter polynucleotides, e.g. having barcodes ID-X1 and ID-Y2 in this instance, are only partially complementary to the capture probes, and may be washed away with stringent washing, e.g. heat. As illustrated in FIG. 21A, for detection, a general primer binding site 2114 on the reporter polynucleotide 2014, e.g. Primer C, may be used for binding a fluorescent detection probe 2112. FIG. 21B illustrates a mechanism in which an amplification template 2116 is used to increase fluorescent signal. In this example, amplification template 2116 hybridizes to general primer binding site 2114, and the 3' end of the primer binding site 2114 is extended. The sample includes fluorescently labeled nucleotides 2118, which are incorporated into the growing strand to generate an increased detection signal. In some examples, each nucleotide may be labeled with a different fluorophore. For example, guanine nucleotides may be labeled with a first fluorophore, thymine nucleotides may be labeled with a second fluorophore, etc. The particular sequence of the elongated strand 2120, and thus the number, sequence, spacing, and types of fluorophores in elongated strand 2120 may be defined by the sequence of amplification template 2116. Different levels of and colors of fluorescence may be provided by tuning the length and sequence of amplification template 2116 so as to affect the number, density, and colors of fluorescently labeled nucleotides coupled thereto. Additionally, as illustrated in FIG. 21B, each incorporated nucleotide 2118 may be coupled to a secondary primer binding site 2122 which can each be extended by the incorporation of nucleotides, further enabling additional cycles of signal amplification in a manner similar to that described in International Publication No. WO 2021/074087, the entire contents of which are incorporated by reference herein.

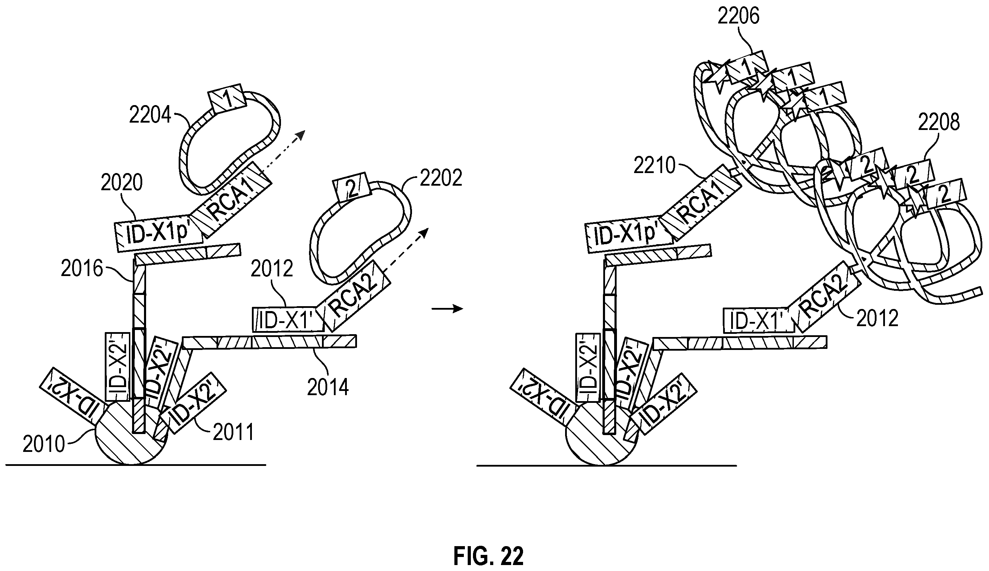

Another mechanism for increasing signal is rolling-circle amplification. As illustrated in FIG. 22, a capture probe 2011 on bead 2010 hybridizes a first barcode of reporter polynucleotide 2014, 2016, and a detection probe 2012, 2020 binds to the other barcode or reporter polynucleotide 2014, 2016. More than one detection probe 2012, 2020 may be used to bind to different barcodes corresponding to different analytes detected by proximity-induced tagmentation. In this example, each detection probe 2012, 2020 includes a 3' sequence 2210, 2212 (e.g. RCA1 and RCA2) that is complementary to a circular DNA template 2202, 2204. The circular DNA template includes a fluorophore binding sequence 2206, 2208. A processive polymerase (e.g. phi 29) may bind the 3' RCA sequence 2210,2212 and make copies of the circular DNA template 2202, 2204. As the circular DNA amplifies, fluorescently labeled nucleotides may be incorporated into the growing copies at the replicated fluorophore binding sequences 2206, 2208 in a manner similar to that described in International Publication No. WO 2021/074087, the entire contents of which are incorporated by reference herein. When assessing two forms of the analyte, the fluorophore binding sequences 2206 may recruit a different fluorophore than the fluorophore binding sequence 2208. When the amplification process is stopped, the signals for both the fluorophores specific to binding sequence 2206 and the fluorophores specific to binding sequence 2208 can be quantified and the ratio between the two signals can be compared.

The use of bead arrays to detect and quantify analytes is further described in WO2021/074087, the entire contents of each of which are incorporated by reference herein.

From the foregoing, it will be appreciated that proximity-induced tagmentation, using recognition elements that are coupled to active barcoded transposomes, may generate reporter polynucleotides in an irreversible (covalent) process, thus reducing the potential for non-specific background noise, and providing specific detection and quantitation of analytes of interest. Additionally, the proximity-induced tagmentation covalently links barcodes, from a pair of respective recognition elements, in the reporter polynucleotide. Linking barcodes from respective donor recognition probes and acceptor recognition probes allows for identification and filtering of any non-specific or off-target tagmentation from the data set, further improving specificity of the assay. Precise control of transposome activity is provided, e.g., via use of a double-stranded DNA handle to inhibit hybridization of common regions. This provides control of the start of tagmentation, and may improve specificity and signal to noise ratio of the assay. In some examples, covalent linkage of barcodes via tagmentation may provide for simultaneous measurements of PTMs and total protein amounts in a single assay, for example by introducing a third protein recognition element specific to PTMs, with an additional unique barcode. It will further be appreciated that the present approach may be used to measure interactions between molecules, including highly multiplexed protein-protein, protein-RNA, or protein-small molecule interactions, thus allowing additional information to be obtained about molecular interactions in a sample.

### Compositions and methods for detecting analytes using proximity-induced strand invasion, restriction, or ligation

Some examples herein provide for detecting analytes using proximity-induced strand invasion, restriction, or ligation.

As provided herein, proximity-induced strand invasion, restriction, or ligation is an alternative mechanism to address the problem of detecting analytes, such as proteins or other biomolecules. Described herein are high throughput methods to detect proteins, sugars, or biological species of interest in biological samples. A biomolecule or a synthetic molecule (*e.g.*, antibodies, toxins, ligands, lectins, and the like) that is connected to a nucleotide sequence can bind to targets or analytes of interest. The nucleotide sequence can be analyzed to determine the identity of the targets or analytes of interest. High-throughput sequencing methods can be used to analyze sequences allowing for detection and quantification of millions of targets or analytes of interest. For example, array technology can be used as part of a massive parallel detection scheme to identify and quantify the targets or analytes of interest.

Whole genome amplification (WGA) can be used to identify and quantify the targets or analytes of interest. There are different methods of WGA. These include WGA methods that require a polymerase chain reaction (PCR) step as well as WGA methods that rely on an isothermal reaction step, instead of PCR. In some examples, identifying and quantifying of the targets or analytes of interest are determined using WGA that includes an isothermal reaction. In some examples, the WGA comprises isothermal, multiple displacement amplification (MDA), a WGA method that relies on strand-displacement DNA polymerase to amplify genomic DNA.

An additional technique that can be used to identify and quantify the targets or analytes is targeted genome amplification (TGA). TGA focuses on targets or analytes that are or derive from a specific subset of genes within the genome. An alternative mechanism for identifying and quantifying the targets relies on capturing the nucleotide sequences that correspond to the targets on analytes on the surface of beads (bead capture), and amplifying the nucleotide sequences. Nonlimiting methods for amplifying nucleotide sequences coupled to a bead include bridge amplification, kinetic exclusion amplification (ExAmp), and the like.

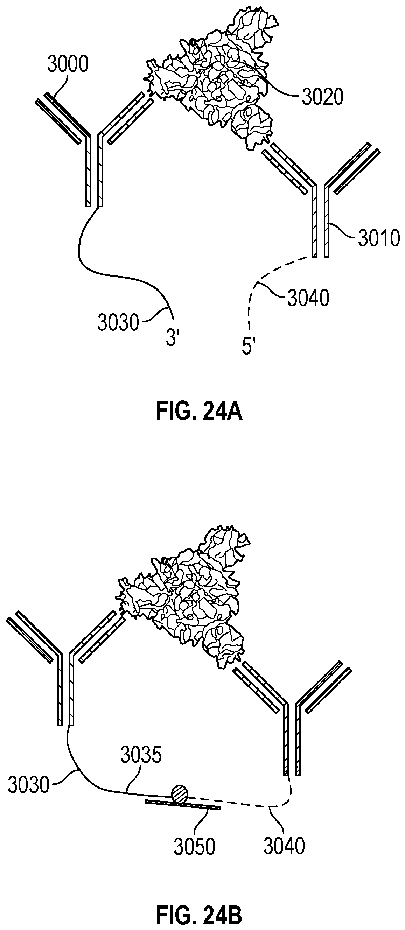

FIGS. 24A-24D schematically illustrate an example process of a proximity induced ligation assay, using a splint oligonucleotide. In the non-limiting example illustrated in FIG. 24A, a first antibody 3000 and a second antibody 3010 interact with an analyte 3020 in a manner such as described elsewhere herein. The first and second antibodies are non-limiting examples of recognition elements capable of interacting with the analyte, and any other recognition elements may be used such as described elsewhere herein. A first oligonucleotide 3030 is attached to the first antibody (or other recognition element) and a second oligonucleotide 3040 is attached to the second antibody (or other recognition element). As illustrated in FIG. 24B, a splint oligonucleotide 3050 binds to ends of both the first oligonucleotide 3030 and the second oligonucleotide 3040, resulting in ligation of the first oligonucleotide to the second oligonucleotide to form a reporter oligonucleotide 3035 (FIG. 24B). The ligation may be performed, for example, using any suitable ligase. The sequence of splint oligonucleotide 3050 may be selected such that the splint oligonucleotide may promote such ligation substantially only between first oligonucleotide 3030 and second oligonucleotide 3040, rather than between any other two pairs of oligonucleotides. For example, the splint oligonucleotide 3050 may include a first portion which is complementary to a sufficient number of bases at the 3' end of first oligonucleotide 3030 to hybridize thereto, and may include a second portion which is complementary to a sufficient number of bases at the 5' end of second oligonucleotide 3040 to hybridize thereto. Accordingly, splint oligonucleotide 3050 may be used to couple first oligonucleotide 3030 to second oligonucleotide 3040, thus generating reporter oligonucleotide 3050. Additionally, if any oligonucleotides other than first oligonucleotide 3030 and/or second oligonucleotide are brought into proximity with one another, e.g., due to non-specific binding to analyte 3020 or a random interaction in solution, splint oligonucleotide 3050 would not sufficiently hybridize to both of such oligonucleotides to promote ligation of the two oligonucleotides to one another.

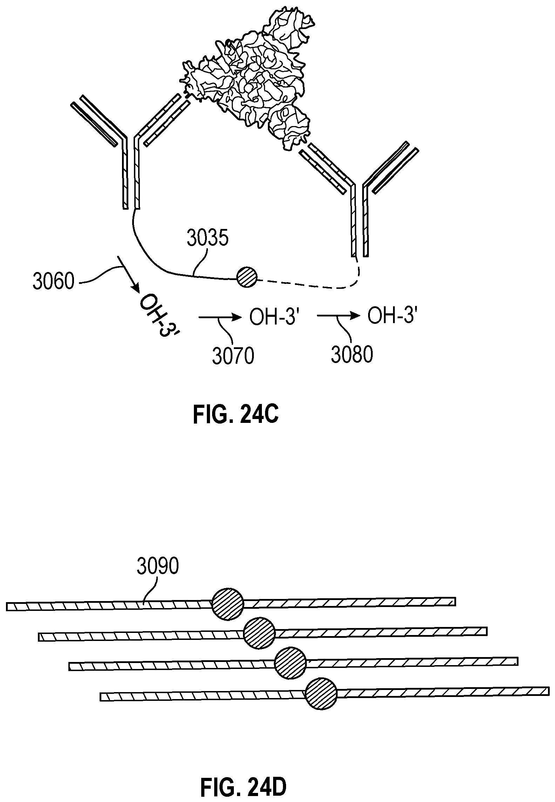

The ligated, reporter oligonucleotide 3035 can be amplified and/or sequenced in any suitable manner in a manner such as provided herein, or in a manner such as known in the art, and the analyte may be identified using the sequences of the ligated oligonucleotides. In some examples, the reporter oligonucleotide 3035 may be amplified using one or more primers 3060, 3070, and 3080 (FIG. 24C). For example, primers 3060, 3070, and 3080 may have sequences which are selected to bind to different portions of first oligonucleotide 3030 and/or second oligonucleotide 3040 within reporter oligonucleotide 3035. A suitable polymerase may be used to extend the primers using the sequences of the first and/or second oligonucleotides, forming a double-stranded oligonucleotide and the reporter oligonucleotide 3035. The amplicons may include sequences complementary to any suitable portion(s) of that of first oligonucleotide 3030 and/or second oligonucleotide 3040. WGA can then be used to analyze the amplified fragments (shown in FIG. 24D). For example, as illustrated in FIG. 24D, a plurality of the amplicons may be complementary both to a portion of the first oligonucleotide 3030 and to a portion of the second oligonucleotide 3040, such that sequencing the amplicons provides sequences of a portion of the first oligonucleotide and a portion of the second oligonucleotide. From the presence of both such sequences, the identity of the analyte may be determined. For example, in a manner similar to that described above with regards to proximity induced tagmentation, the first oligonucleotide 3030 may include a first sequence that corresponds to analyte 3020, and the second oligonucleotide 3040 may include a second sequence that corresponds to analyte 3020. From the presence of both the first and second sequences in the reporter oligonucleotide (or amplicon thereof), it may be determined that analyte 3020 was present in the sample. Additionally, in a manner similar to that described above with regards to proximity induced tagmentation, the amount of the reporter oligonucleotide (or amplicon thereof) may be used to determine the amount of the analyte 3020. In some examples, an oligonucleotide connected to a probe (e.g., oligonucleotide 3030, oligonucleotide 3040, and/or reporter oligonucleotide 3035) includes a barcode. In some examples, an oligonucleotide connected to a probe (e.g., oligonucleotide 3030, oligonucleotide 3040, and/or reporter oligonucleotide 3035) includes a partial barcode. In such examples, coupling oligonucleotide 3030 to oligonucleotide 3040 in a manner such as described with reference to FIG. 24B may generate a complete barcode composed of the partial barcodes.

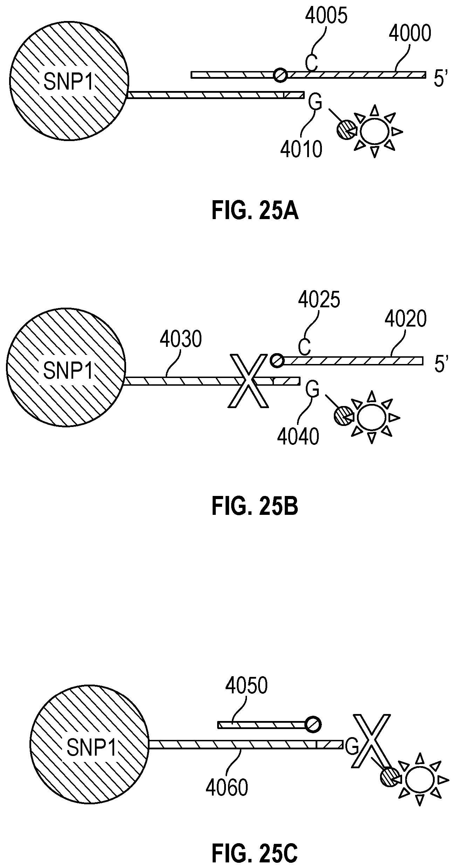

In other examples, TGA or bead capture (methods described herein) can be used to analyze the amplicons. Nonlimiting examples of use of bead capture to analyze amplicons are described further above, as well as further below with reference to FIGS. 25A-25C.

In examples such as illustrated in FIGS. 24A-24D, the first antibody 3000 and the second antibody 3010 form a probe that can be used to determine the identity of the analyte. In some examples, biomolecules or synthetic molecules, other than antibodies, can be used as probes to bind to and detect an analyte. In some examples, the probe is a biomolecule or synthetic molecule that includes an amino acid sequence. In some examples, the probe is a biomolecule or synthetic molecule that includes a nucleic acid sequence. In some examples, the probe is a biomolecule or synthetic molecule that includes a combination of amino acid and nucleic acid sequences. In some examples, the probe includes a lectin. In some examples, the probe includes an aptamer. In some examples, the probe includes a lectin and an aptamer. In some examples, the probe includes a lectin and an antibody. In some examples, the probe includes an aptamer and an antibody. Still other options may be envisioned based on the teachings herein.

In some examples, the probe incorporates a label capable of being detected. In some examples, the label comprises a fluorescent tag. In some examples, the label includes a fluorophore. In some examples, the label includes an enzyme. In some examples, the label includes biotin. In some examples, the label includes hapten.

FIGS. 25A-25C schematically illustrate examples of ways of differentiating between ligated and un-ligated oligonucleotides. Probes can be designed such that they are optimized to detect ligated products. For example, a ligated oligonucleotide 4000 including a single nucleotide polymorphism (SNP) 4005 can be used to form a stable duplex that incorporates a hapten-labeled modified base 4010 (FIG. 25A). The duplex is resistant to stringent wash steps. In contrast, an un-ligated oligonucleotide 4020 including a SNP 4025 may not be thermally stable with an oligonucleotide 4030 including the hapten-labeled modified base 4040, because the nucleotide overlap between oligonucleotides is minimal (FIG. 25B). Stringent wash steps result in removal of the un-ligated oligonucleotide. In some cases an un-ligated oligonucleotide may be capable of forming a stable duplex with an oligonucleotide that includes a hapten-labeled modified base (FIG. 25C). However, the un-ligated oligonucleotide does not include the SNP.

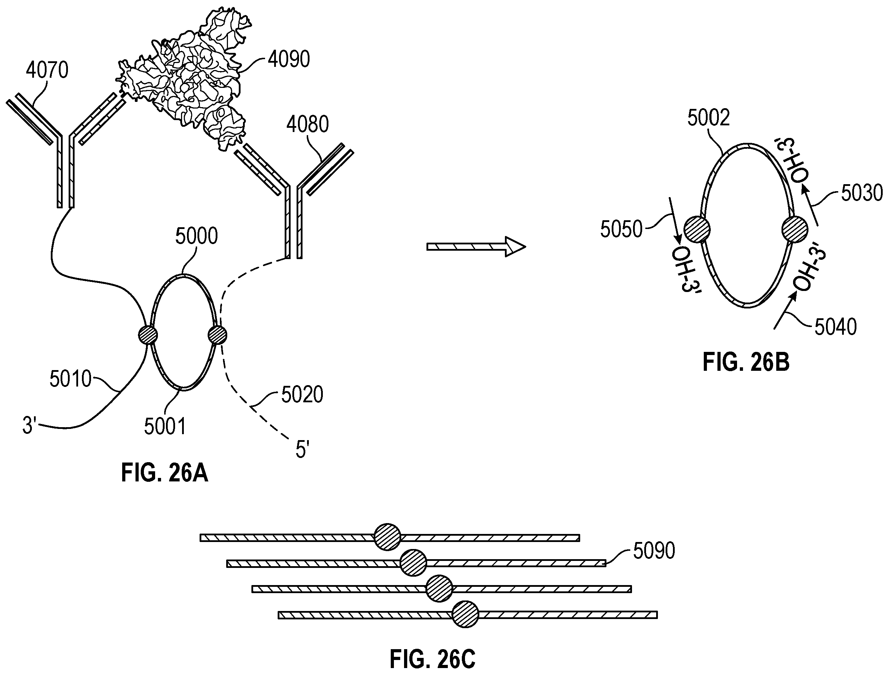

It will be appreciated that any suitable splint oligonucleotide may be used to generate a reporter polynucleotide using first oligonucleotide 3030 and second oligonucleotide 3040. For example, FIGS. 26A-26C schematically illustrate another example process of a proximity induced ligation assay, using a splint oligonucleotide. As illustrated in FIG. 26A, a first recognition element (e.g., antibody) 4070 and a second recognition element (e.g., antibody 4080) interact with an analyte 4090 in a manner similar to that described with reference to FIGS. 24A-24D. First splint oligonucleotide 5000 binds to both a first portion of first oligonucleotide 5010 that is connected to the first recognition element (e.g., antibody) and to a first portion of second oligonucleotide 5020 that is connected to the second recognition element (e.g., antibody). Additionally, second splint oligonucleotide 5001 binds to both a second portion of first oligonucleotide 5010 and a second portion of second oligonucleotide 5020. As illustrated in FIG. 26A, binding of first splint oligonucleotide 5000 to both the first oligonucleotide 5010 and the second oligonucleotide 5020, and binding of second splint oligonucleotide 5000 to both the first oligonucleotide 5010 and the second oligonucleotide 5020, results in ligation of the first splint oligonucleotide to the second splint oligonucleotide to form circular reporter oligonucleotide 5002 (FIG. 26A). The ligation may be performed, for example, using any suitable ligase.

The respective sequences of splint oligonucleotides 5000 and 5001 may be selected so as to promote such ligation substantially only between first splint oligonucleotide 5000 and second splint oligonucleotide 5001, rather than between any other two pairs of oligonucleotides. For example, the first splint oligonucleotide 5000 may include a first portion which is complementary to a sufficient number of bases along first oligonucleotide 5010 to hybridize thereto, and may include a second portion which is complementary to a sufficient number of bases along second oligonucleotide 5020 to hybridize thereto. Similarly, the second splint oligonucleotide 5001 may include a first portion which is complementary to a sufficient number of bases along first oligonucleotide 5010 to hybridize thereto, and may include a second portion which is complementary to a sufficient number of bases along second oligonucleotide 5020 to hybridize thereto. Accordingly, splint oligonucleotides 5000, 5001 may be used to couple first oligonucleotide 5010 to second oligonucleotide 5020, thus generating reporter oligonucleotide 5002. Additionally, if any oligonucleotides other than first oligonucleotide 5010 and/or second oligonucleotide 5020 are brought into proximity with one another, e.g., due to non-specific binding to analyte 4090 or a random interaction in solution, splint oligonucleotides 5000, 5001 would not sufficiently hybridize to both of such oligonucleotides to promote ligation of the two splint oligonucleotides to one another.

An exonuclease can be used to degrade the first oligonucleotide 5010 and the second oligonucleotide 5020, as well as any splint oligonucleotides which do not form circular reporter oligonucleotides, resulting in isolating the circular reporter oligonucleotide 5002 illustrated in FIG. 26B, which is resistant to DNA degradation. In a manner similar to that described with reference to FIG. 24C, multiple primers (illustratively, 5030, 5040, and 5050) can be used to amplify the circular splint oligonucleotide (FIG. 26B). Whole genome amplification (WGA) can then be used to amplify and analyze the fragments 5090 (shown in FIG. 26C), thereby determining the identity of the analyte in a manner similar to that described with reference to FIG. 24D. The isolated circular splint nucleotide can be analyzed using other techniques such as TGA and bead capture, as described herein.

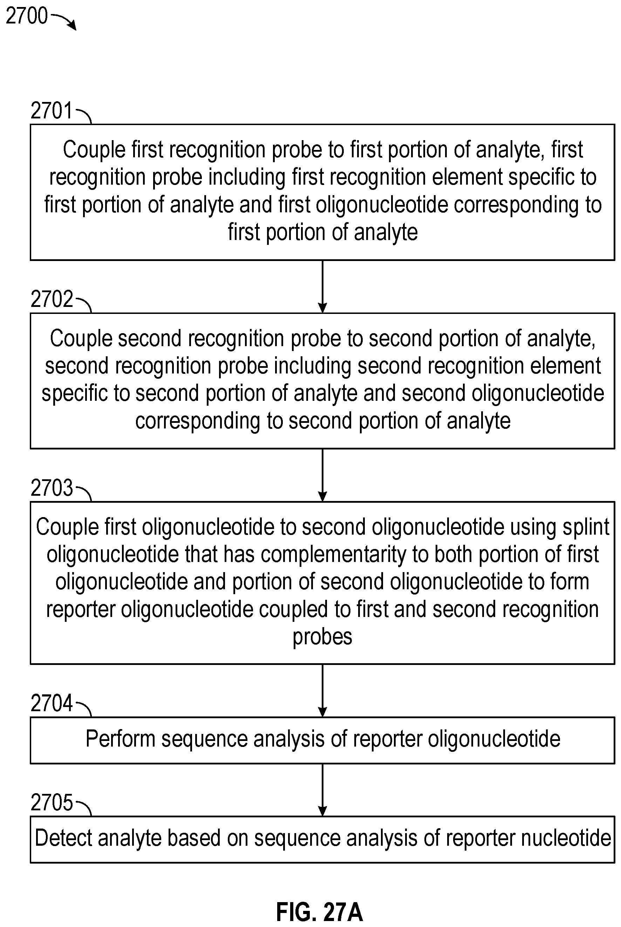

FIGS. 27A-27B illustrate flows of operations in example methods for detecting an analyte using a splint oligonucleotide, according to some examples herein. Referring first to FIG. 27A, method 2700 includes coupling a first recognition probe to a first portion of the analyte, the first recognition probe including a first recognition element specific to the first portion of the analyte and a first oligonucleotide corresponding to the first portion of the analyte (operation 2701). For example, in a manner such as described with reference to FIG. 24A, first recognition element 3000 (illustratively, a first antibody) is coupled to a first portion of analyte 3020. Or, for example, in a manner such as described with reference to FIG. 26A, first recognition element 4070 (illustratively, a first antibody) is coupled to a first portion of analyte 4090. Nonlimiting examples of recognition elements and analytes are described elsewhere herein. For example, the first recognition probe or the second recognition probe may include an antibody, a lectin, or an aptamer. Illustratively, the first recognition probe may include a first antibody, a first lectin, or a first aptamer, and the second recognition probe may include a second antibody, a second lectin, or a second aptamer. In one nonlimiting example, the analyte includes molecules interacting with one another in a manner such as described elsewhere herein.

Referring still to FIG. 27A, method 2700 may include coupling a second recognition probe to a second portion of the analyte, the second recognition probe including a second recognition element specific for the second portion of the analyte and a second oligonucleotide corresponding to the second portion of the analyte (operation 2702). For example, in a manner such as described with reference to FIG. 24A, second recognition element 3010 (illustratively, a second antibody) is coupled to a second portion of analyte 3020. Or, for example, in a manner such as described with reference to FIG. 26A, second recognition element 4080 (illustratively, a second antibody) is coupled to a second portion of analyte 4090. Nonlimiting examples of recognition elements and analytes are described elsewhere herein.

Method 2700 illustrated in FIG. 27A further may include coupling the first oligonucleotide to the second oligonucleotide using a splint oligonucleotide that has complementarity to both a portion of the first oligonucleotide and a portion of the second oligonucleotide to form a reporter oligonucleotide coupled to the first and second recognition probes (operation 2703). For example, in a manner such as described with reference to FIG. 24B, a linear splint oligonucleotide 3050 may include a first sequence that is complementary to a portion of first oligonucleotide 3030, and a second sequence that is complementary to a portion of second oligonucleotide 3040. In some examples, the linear splint oligonucleotide 3050 and a ligase may be used to ligate first oligonucleotide 3030 to second oligonucleotide 3040 to form reporter oligonucleotide 3035. In another example, in a manner such as described with reference to FIG. 26A, first and second splint oligonucleotides 5000, 5001 respectively may include first sequences that are complementary to respective portions of first oligonucleotide 5010, and second sequences that are complementary to respective portions of second oligonucleotide 5020. In some examples, a ligase is used to ligate the first and second splint oligonucleotides 5000, 5001 to one another, forming a reporter oligonucleotide 5002 that couples first oligonucleotide 5010 to second oligonucleotide 5020. In some examples, the first oligonucleotide includes a partial barcode, the second oligonucleotide includes a partial barcode, and coupling the first oligonucleotide to the second oligonucleotide results in a complete barcode that corresponds to the target analyte.

Method 2700 illustrated in FIG. 27A also may include performing a sequence analysis of the reporter oligonucleotide (operation 2704). In some examples, the sequence analysis includes amplifying the reporter oligonucleotide, e.g., using WGA, TGA, or bead-based amplification such as described elsewhere herein. Nonlimiting examples of performing WGA to amplify the reporter oligonucleotide are described with reference to FIGS. 24C-24D and 26B-26C. Optionally, a portion of the double-stranded oligonucleotide formed either before or during such amplification may be excised, and the sequence analysis may be performed on the excised portion of the double-stranded oligonucleotide. Such excision may be performed, for example, using a CRISPR-associated (Cas) protein, a restriction enzyme, or the like.

Method 2700 also may include detecting the analyte based on the sequence analysis of the reporter oligonucleotide (operation 2705). In some examples, performing the sequence analysis includes performing a polymerase chain reaction (PCR) on the reporter oligonucleotide. In some examples, the reporter oligonucleotide includes a unique molecular identifier (UMI) that is amplified during the PCR.

Although FIGS. 24A-24D, 25A-25C, 26A-26C, and 27A may focus on interactions between first and second recognition probes and an analyte for which those recognition probes are selective, it should be appreciated that such interactions may be multiplexed. For example, a sample may include a plurality of different analytes that may be detected in a multiplexed manner, for example, by contacting the analytes with a plurality of different recognition probes respectively corresponding to analytes that may be in the sample, and with a plurality of different splint oligonucleotides corresponding to the recognition probes.

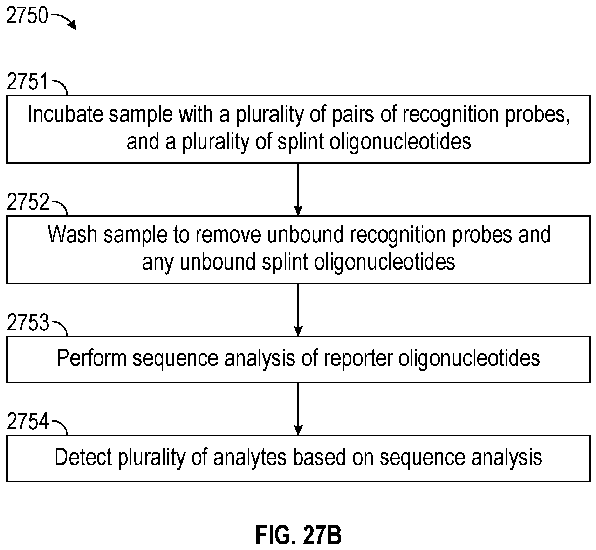

For example, FIG. 27B illustrates an example flow of operations in a method 2750 for detecting a plurality of analytes in a sample. Method 2750 may include incubating the sample with a plurality of pairs of recognition probes, and a plurality of splint oligonucleotides (operation 2751). Each pair of recognition probes includes a first recognition probe and a second recognition probe, and each pair of recognition probes is specific for a respective one of the analytes. Additionally, each first recognition probe and each second recognition probe are coupled to a respective oligonucleotide. Example configurations for recognition probes and example oligonucleotides are described elsewhere herein, e.g., with reference to FIGS. 24A-24D and 26A-26C. Each splint oligonucleotide may be complementary to portions of oligonucleotides that respectively are coupled to a first recognition probe and a second recognition probe of a pair of recognition probes which is specific to a respective one of the analytes, and complementary binding of each splint oligonucleotide to oligonucleotides that are coupled to first recognition probes and second recognition probes results in formation of reporter oligonucleotides. Example configurations for splint oligonucleotides and their use to form reporter oligonucleotides are described with reference to FIGS. 24A-24D and 26A-26C. Illustratively, incubating the sample in operation 2751 further may include incubation with a ligase.

Method 2750 further may include washing the sample to remove any unbound recognition probes and any unbound splint oligonucleotides (operation 2752). Method 2750 also may include performing a sequence analysis of the reporter oligonucleotides, for example after washing operation 2752 (operation 2753). Nonlimiting examples of sequence analyses are provided elsewhere herein. For example, performing the sequence analysis may include using any one or more of a microarray, a bead array, library preparation, or PCR. Method 2750 also may include detecting the plurality of analytes based on the sequence analysis. Example methods for detecting analytes based on sequence analysis are described elsewhere herein. It will be appreciated that although a plurality of analytes, recognition probes, and splint oligonucleotides may be incubated with one another for a given sample during operation 2751, pairs of recognition probes are specific for given analytes and splint oligonucleotides are specific for pairs of recognition probes, thus providing a relatively high degree of specificity in detection of the analytes. Additionally, the sequence analyses of the various reporter oligonucleotides may be conducted in a multiplexed manner, providing rapid analysis of different analytes in the sample without the need for separately performing different assays for the different analytes.

Some examples herein provide a kit that includes a plurality of pairs of recognition probes, and a plurality of splint oligonucleotides. In a manner similar to that discussed with reference to operation 2751 of FIG. 27B and elsewhere herein, each pair of recognition probes includes a first recognition probe and second recognition probe, each pair of recognition probes is specific for a respective one of the analytes, and each first recognition probe and each second recognition probe are coupled to a respective oligonucleotide. Additionally, in a manner similar to that discussed with reference to operation 2751 of FIG. 27B and elsewhere herein, each splint oligonucleotide is complementary to portions of oligonucleotides that respectively are coupled to a first recognition probe and a second recognition probe of a pair of recognition probes which is specific to a respective one of the analytes. Illustratively, the kit may be used in a manner such as described with reference to FIG. 27B. During use of such kit and/or during implementation of method 2750, operations such as described with reference to FIG. 27A may be performed.

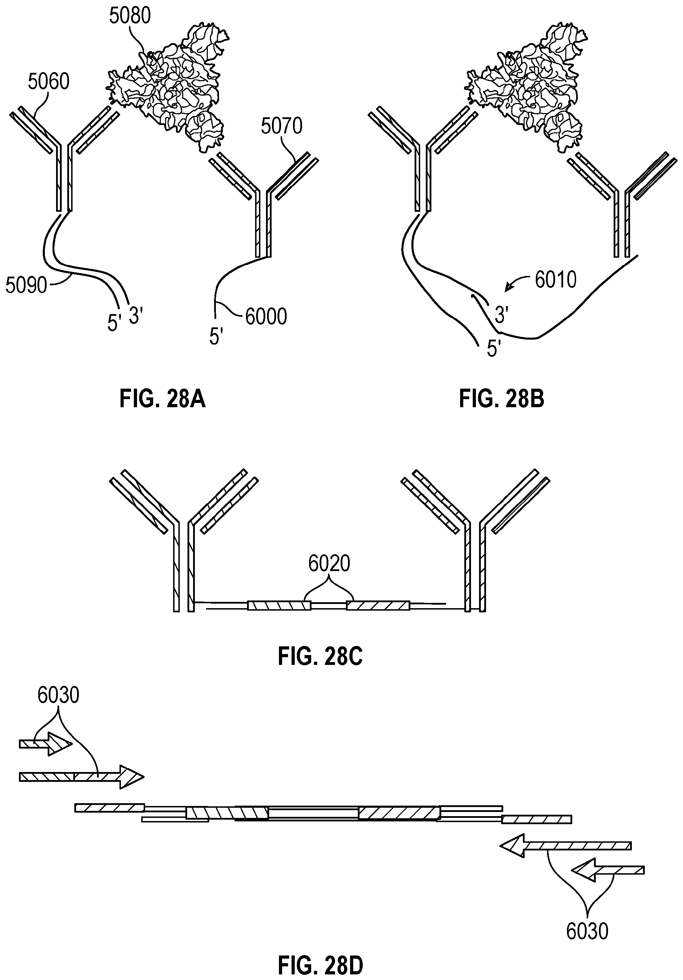

Still other operations and compositions may be used to generate reporter oligonucleotides for which sequence analysis may be performed, and for which analytes may be identified using the sequence analysis. For example, FIGS. 28A-28D schematically illustrate an example process of a proximity induced strand invasion assay. As illustrated in FIG. 28A, first recognition element (e.g., antibody) 5060 and a second recognition element (e.g., antibody 5070) interact with an analyte 5080. The first recognition element (e.g., antibody) is connected to a double-stranded oligonucleotide strand 5090, whereas the second recognition element (e.g., antibody) is connected to a single-stranded oligonucleotide strand 6000. The 5' end of single-stranded oligonucleotide 6000 invades the double stranded oligonucleotide 6010 (FIG. 28B). For example, the strand of double-stranded oligonucleotide 6010 with the 3' termination in FIG. 28A may hybridize less strongly to the strand of double-stranded oligonucleotide 6010 with the 5' termination, than does the 5' end of single-stranded oligonucleotide 6000. Accordingly, single-stranded oligonucleotide 6000 may partially displace the strand of double-stranded oligonucleotide 6010 with the 3' termination in FIG. 28A, forming a double-stranded oligonucleotide as indicated at 6010 in FIG. 28B. Strand invasion brings barcodes 6020 on each strand in proximity to each other (FIG. 28C). Primers 6030 can be used to amplify barcodes (FIG. 28D). Quantitative detection such as an array or sequencing technology can be used to analyze the amplified barcodes, thereby determining the identity of the analyte in a manner such as described elsewhere herein.

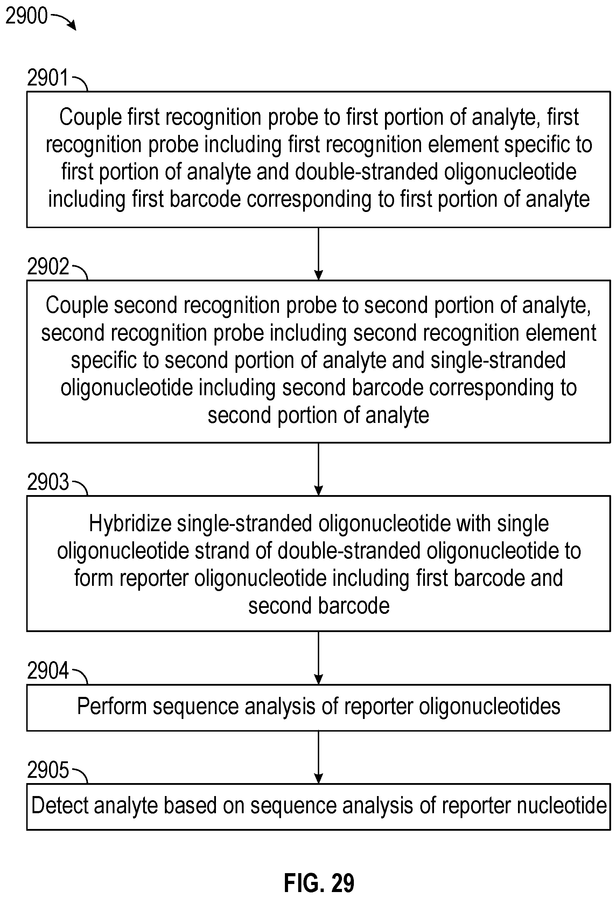

FIG. 29 illustrates a flow of operations in an example method 2900 for detecting an analyte using proximity induced strand invasion, according to some examples herein. Method 2900 illustrated in FIG. 29 may include coupling a first recognition probe to a first portion of the analyte (operation 2901). The first recognition probe may include a first recognition element specific to the first portion of the analyte and a double-stranded oligonucleotide including a first barcode corresponding to the first portion of the analyte, e.g., in a manner such as described with reference to FIGS. 28A-28D. Method 2900 also may include coupling a second recognition probe to a second portion of the analyte (operation 2902). The second recognition probe may include a second recognition element specific for the second portion of the analyte and a single-stranded oligonucleotide including a second barcode corresponding to the second portion of the analyte, e.g., in a manner such as described with reference to FIGS. 28A-28D. Nonlimiting examples of recognition elements and analytes are provided elsewhere herein. Method 2900 also may include hybridizing the single-stranded oligonucleotide with a single oligonucleotide strand of the double-stranded oligonucleotide to form a reporter oligonucleotide including the first barcode and the second barcode (operation 2903). In some examples, the hybridizing operation includes strand invasion of the double-stranded oligonucleotide by the single-stranded oligonucleotide. Such strand invasion may be performed in a manner such as described with reference to FIG. 28B. Method 2900 also may include performing a sequence analysis of the reporter oligonucleotide (operation 2904). Nonlimiting examples of sequence analysis are provided elsewhere herein. Illustratively, the sequence analysis that is performed may include any one or more of isothermal bead-based amplification, targeted genome amplification, and whole genome amplification. FIG. 28D illustrating one potential manner for performing sequence analysis. Method 2900 also may include detecting the analyte based on the sequence analysis of the reporter oligonucleotide (operation 2905). Example operations for detecting an analyte based on a sequence analysis of a reporter oligonucleotide are provided elsewhere herein. Optionally, detecting the analyte includes performing quantitative detection of the reporter oligonucleotide.

In still other examples, proximity induced restriction is used to detect analytes. For example, FIGS. 29A-29D schematically illustrate an example process of a proximity induced restriction assay. As illustrated in FIG. 29, first recognition element (e.g., antibody) 6040 and a second recognition element (e.g., antibody) 6050 interact with an analyte 6060 in a manner such as described elsewhere herein. The first recognition element (e.g., antibody) is connected to a first single-stranded oligonucleotide 6070, and the second recognition element (e.g., antibody) is connected to a second single-stranded oligonucleotide 6080. Each of the first and second single-stranded oligonucleotides include restriction endonuclease sites 6090. Complementary stands of each of the first and second single-stranded oligonucleotides hybridize to each other, e.g., at the location denoted 7000 in FIG. 28B. For example, a portion of first oligonucleotide 6070 may be complementary to a portion of second oligonucleotide 6080 such that the oligonucleotides hybridize to one another. The hybridized oligonucleotides can be cut at the restriction sites 6090 (FIG. 28C), for example using a restriction endonuclease such as EcoR1. In some examples, the cut DNA can be amplified with primers 7010 (FIG. 28D). Quantitative detection such as array or sequencing technology can be used to analyze the cut DNA. In some examples, the single-stranded oligonucleotides include any restriction endonuclease site known in the art.

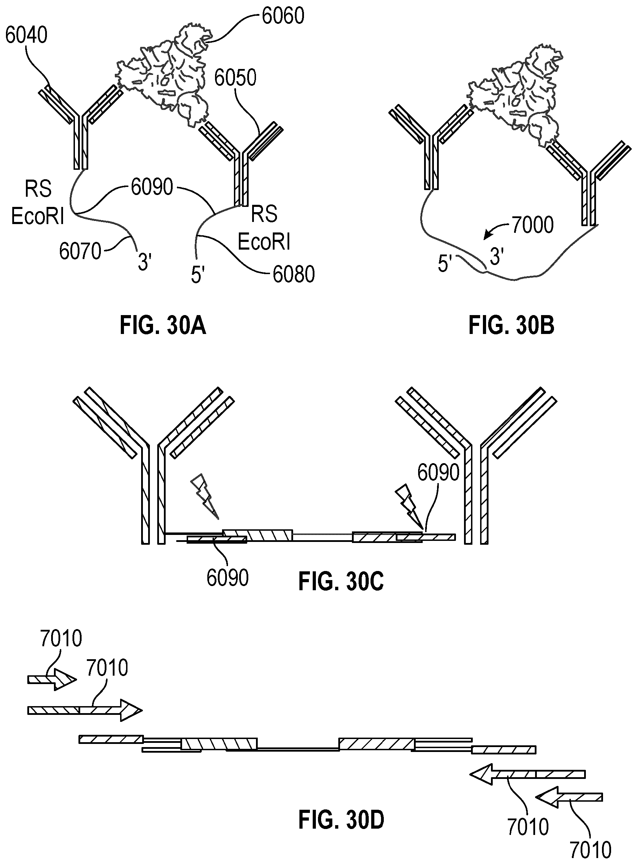

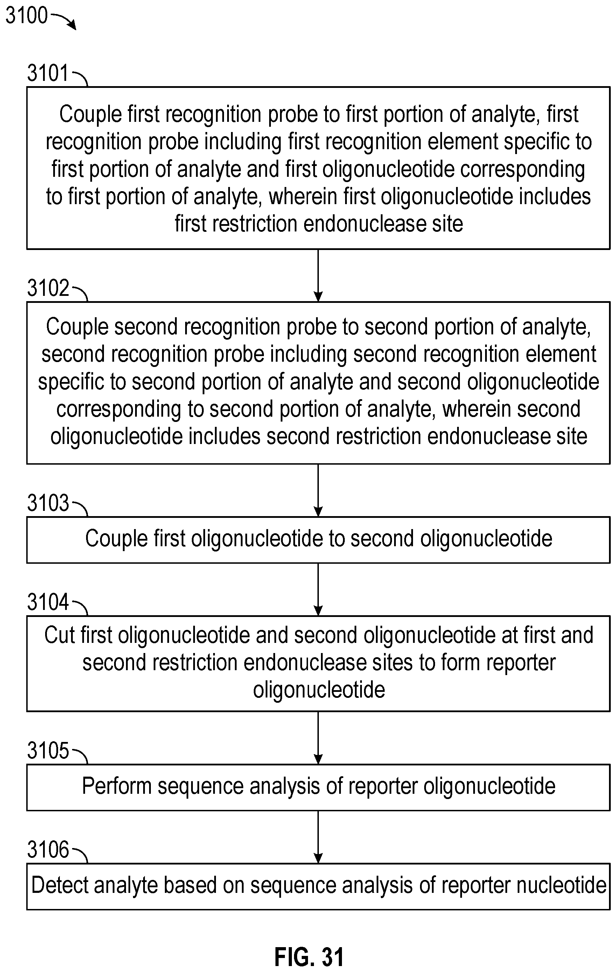

FIG. 31 illustrates a flow of operations in an example method for detecting an analyte using proximity induced restriction, according to some examples herein. Method 3100 illustrated in FIG. 31 includes coupling a first recognition probe to a first portion of the analyte (operation 3101). The first recognition probe may include a first recognition element specific to the first portion of the analyte and a first oligonucleotide corresponding to the first portion of the analyte, wherein the first oligonucleotide includes a first restriction endonuclease site. Method 3100 also may include coupling a second recognition probe to a second portion of the analyte (operation 3102). The second recognition probe may include a second recognition element specific for the second portion of the analyte and a second oligonucleotide corresponding to the second portion of the analyte, wherein the second oligonucleotide includes a second restriction endonuclease site. Operations 3101 and 3102 may be performed in a manner such as described with reference to FIG. 30A. Nonlimiting examples of recognition elements and analytes are provided elsewhere herein. Method 3100 also may include coupling the first oligonucleotide to the second oligonucleotide (operation 3103). For example, in a manner such as described with reference to FIGS. 30A-30B, a portion of first oligonucleotide 6070 may hybridize to second oligonucleotide 6080. Method 3100 also may include cutting the first oligonucleotide and the second oligonucleotide at the first and second restriction endonuclease sites to form a reporter oligonucleotide (operation 3104). The cutting optionally may include using one or more restriction endonucleases. Alternatively, instead of including restriction endonuclease sites in the first and second oligonucleotides, sequences may be included that can be targeted by a CRISPR-Cas ribonucleoprotein and the cutting performed by such a ribonucleoprotein. Method 3100 may include performing a sequence analysis of the reporter oligonucleotide (operation 3105), e.g., in a manner such as described elsewhere herein. For example, the sequence analysis that is performed may include any one or more of isothermal bead-based amplification, targeted genome amplification, and whole genome amplification. Method 3100 also may include detecting the analyte based on the sequence analysis of the reporter oligonucleotide (operation 3106), e.g., in a manner such as described elsewhere herein. Optionally, detecting the analyte includes performing quantitative detection of the reporter oligonucleotide.

### Compositions and methods for targeted epigenetic assays

Some examples herein provide for the enrichment of polynucleotides (such as DNA) to generate fragments of epigenetic interest, and assaying proteins at loci along those fragments. Several nonlimiting examples of assays are given with specific workflow operations and orderings, but other examples may readily be envisioned. In the present examples, the loci may be labeled using oligonucleotides which subsequently are sequenced, and the sequences of the oligonucleotides may be used to characterize the proteins that were respectively coupled to such loci. For example, the sequence of the oligonucleotides may provide information about the presence of the proteins at loci of a given fragment, may provide information about the location of the proteins at loci of a given fragment, may provide information about the quantity of the proteins at loci of a given fragment, or any suitable combination of such information. The fragments may be enriched, e.g., fragments to which proteins are bound may be specifically selected from a given polynucleotide, amplified, sequenced to obtain information therefrom, while other portions of that polynucleotide, and portions of other polynucleotides, may not be amplified or sequenced and thus may be discarded. Such locus-associated proteome analysis may be used, illustratively, to provide a genome-wide proteomic atlas that complements whole-genome sequencing to provide an enhanced characterization of the relationship between genotype phenotype, or to better characterize epigenetic features associated with specific loci and understand epigenetic mechanisms important for research or for clinical applications and therapies. For example, whereas previously known technology may allow detection of where a single protein binds at a time, the present epigenetic assays provide for targeted, multiplexed detection of multiple proteins across an entire chromosome, or even across a whole genome.

As provided herein, complexes that include transposomes coupled to antibodies may be used to generate fragments of a polynucleotide, and optionally of polynucleotides within a whole genome sample. The transposomes of the complexes may label each of the fragments with oligonucleotides that correspond to the particular proteins coupled to those fragments. For example, as now will be described, the loci of a polynucleotide may be labeled using a mixture of complexes respectively including antibodies that are specific to different proteins coupled to those loci. Each of the complexes also may include one or more transposomes, each of which optionally may include a dimer of transposases, and each of which transposases may be coupled to an oligonucleotide for labeling that locus in such a manner as to characterize the protein coupled to that locus. For example, the transposomes to which the antibodies are coupled may cut the polynucleotide and add the oligonucleotide to the cut ends in a process which may be referred to as "tagmentation." The respective sequences of the resulting fragments and oligonucleotides added by the transposomes may be used to identify the proteins which had been coupled to those fragments in a multiplexed manner, e.g., for an entire polynucleotide or even for a WG sample.

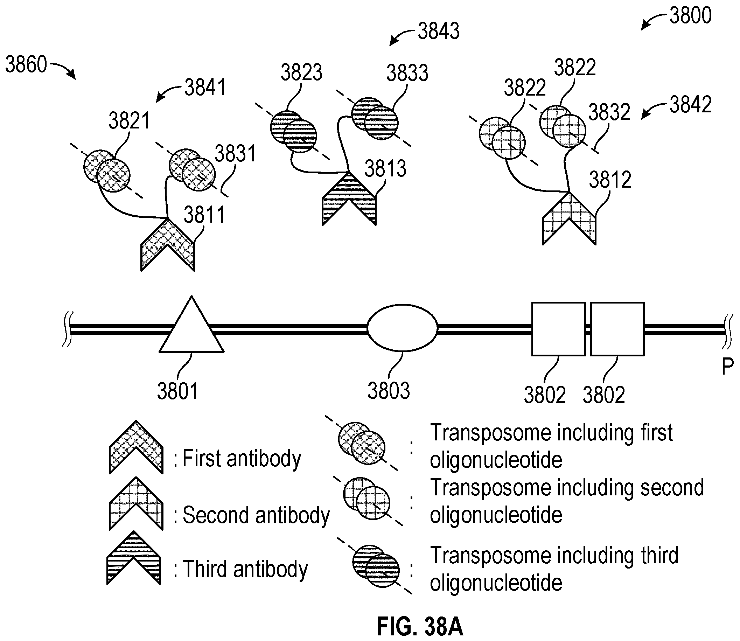

For example, composition 3800 illustrated in FIG. 38A includes polynucleotide P in contact with a mixture of complexes that are specific to different types of proteins, e.g., first, second, and third complexes 3841, 3842, 3843. Illustratively, polynucleotide P may be brought into contact with first, second, and third complexes 3841, 3842, 3843 using fluid 3860 in which such complexes are provided. Polynucleotide P may include different types of proteins coupled to respectively loci thereof, e.g., may include proteins 3801 and 3802 at respective loci, as well as chromatin 3803 (e.g., a nucleosome including DNA wrapped around histone proteins). Polynucleotide P may correspond to a representative polynucleotide within a purified, isolated whole genome sample from a cell or tissue. Alternatively, polynucleotide P may be enriched, for example using Cas9 based methods such as described in International Patent Application No. PCT/US2022/019252, filed March 8, 2022 and entitled "Genomic Library Preparation and Targeted Epigenetic Assays Using Cas-gRNA Ribonucleoproteins," the entire contents of which are incorporated by reference herein. As provided herein, proteins 3801 and 3802 may be assayed substantially without disrupting interactions between polynucleotide P and the proteins.

Each of the complexes 3841, 3842, 3843 may include an antibody corresponding to (selective for) a type of protein, an oligonucleotide corresponding to that type of protein, with a transposome that may be activated under certain conditions. The transposome may include an oligonucleotide which includes an ME sequence as well as a sequence that identifies a protein to which the antibody corresponds. For example, first complex 3841 includes first antibody 3811 coupled to first transposome 3821 including first oligonucleotide 3831. Second complex 3842 includes second antibody 3812 coupled to second transposome 3822 including second oligonucleotide 3832. Third complex 3843 includes third antibody 3813 coupled to third transposome 3823 including third oligonucleotide 3833. In nonlimiting examples such as illustrated in FIG. 38A, each antibody may be coupled to more than one transposome. For example, first complex 3841 may include first antibody 3811 coupled to two transposomes 3821, second complex 3842 may include second antibody 3812 coupled to two transposomes 3822, and third complex 3843 may include third antibody 3813 coupled to two transposomes 3823. However, each complex may include a single transposome coupled to each antibody, or more than two transposomes coupled to each antibody, or two antibodies coupled to each transposome, or more than two antibodies coupled to each transposome.

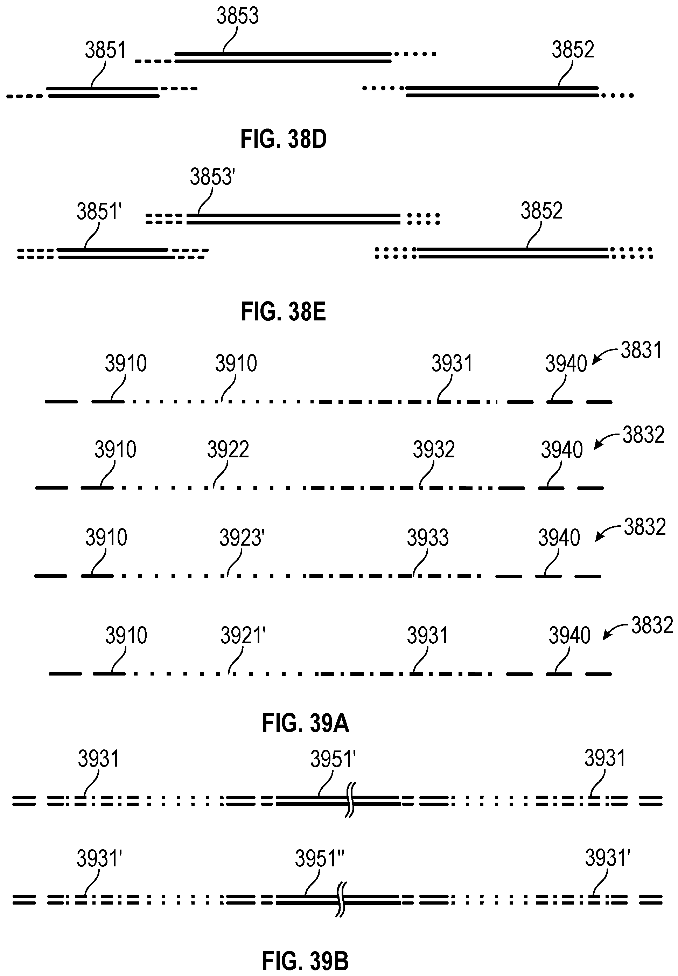

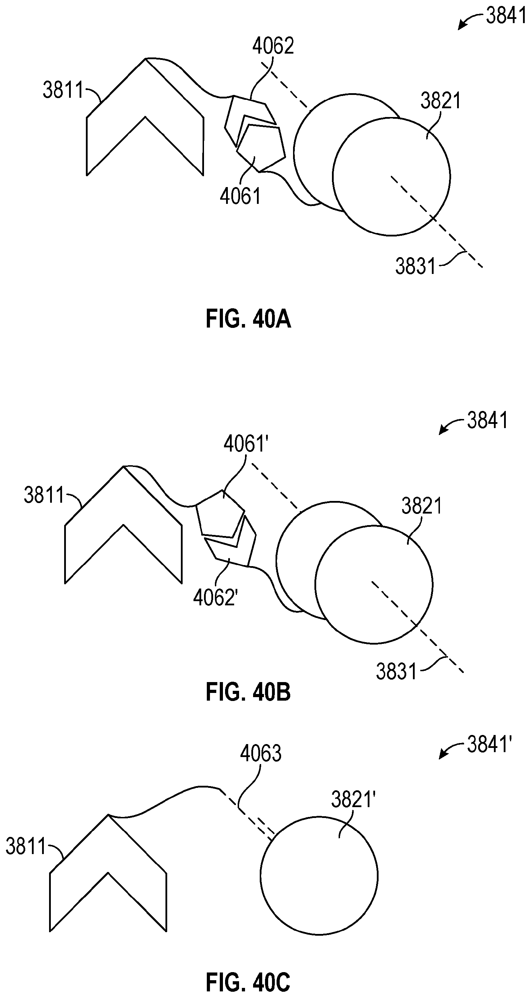

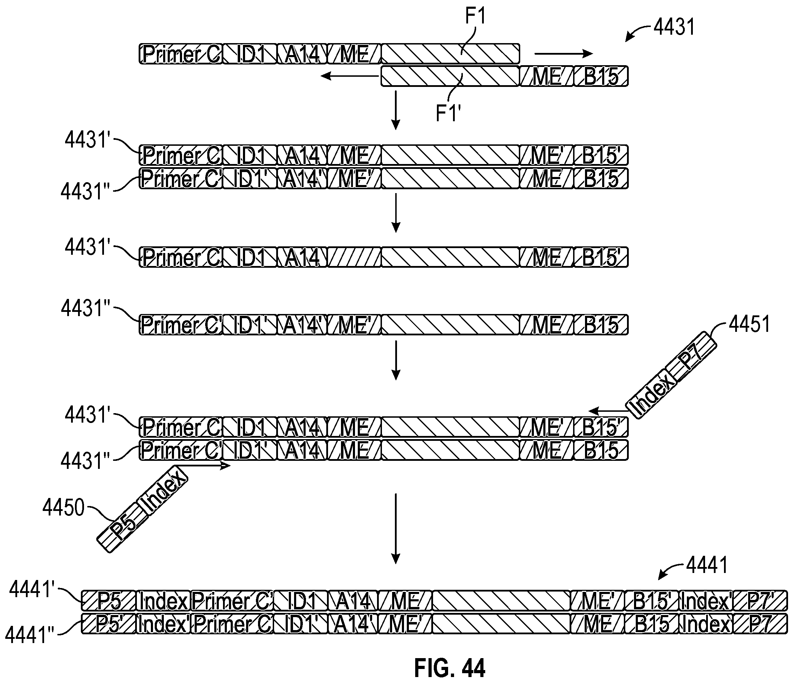

Each of the transposomes may include to any suitable number of oligonucleotides, e.g., one or more oligonucleotides. For example, each of transposomes 3821 may include two first oligonucleotides 3831 (one coupled to each transposase), each of transposomes 3822 may include two second oligonucleotides 3832 (one coupled to each transposase), and each of transposomes 3823 may include two third oligonucleotides 3833 (one coupled to each transposase). Transposomes 3821, 3822, 3823 otherwise may be substantially the same as one another, although they are shaded differently than one another in FIG. 38A, and shaded similarly as the antibodies to which they are respectively coupled, for ease of visual distinction. The oligonucleotides 3831, 3832, 3833 may have one or more subsequences in common with one another, and one or more subsequences that are different. Further details regarding first, second, and third oligonucleotides 3831, 3832, 3833 are provided below with reference to FIGS. 39A-39B and FIG. 44. Further details regarding preparation of complexes 3841, 3842, 3843 are provided below with reference to FIGS. 40A-40C, FIG. 41, FIG. 42, FIG. 45, and FIGS. 46A-46B.

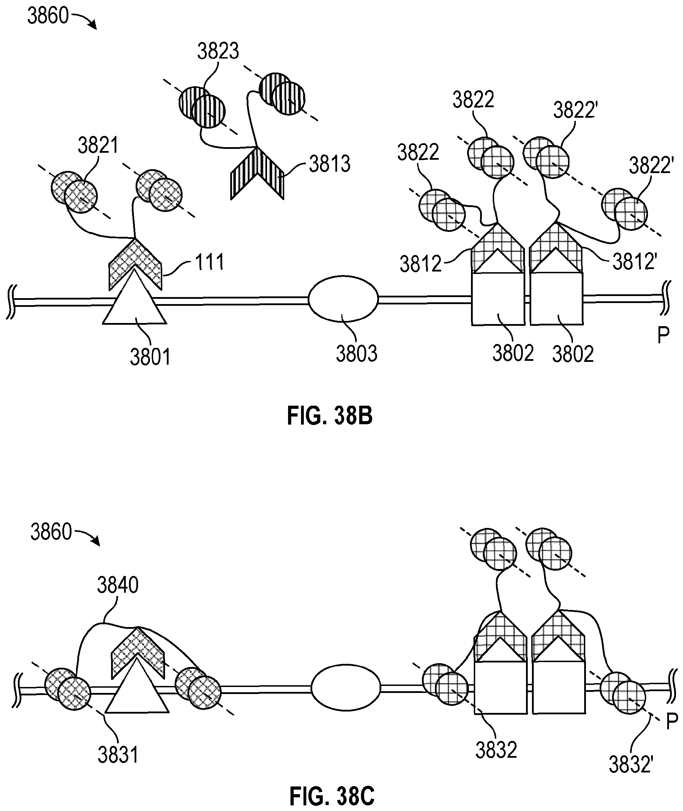

Each of antibodies 3811, 3812, 3813 is specific to a different protein, which protein may or may not necessarily be coupled to a locus of polynucleotide P. It will be appreciated that polynucleotide P may be contacted with any suitable number and type of different complexes respectively including antibodies that are specific to different proteins that potentially may be coupled to loci along polynucleotide P (and indeed the polynucleotides of a WG sample). Additionally, it will be appreciated that polynucleotide P (and indeed each of the polynucleotides of a WG sample) may include any suitable number and type of different proteins at loci along that polynucleotide. For any antibodies in the mixture that are specific to the proteins coupled to the respective loci of polynucleotide P, those antibodies, as well as the corresponding transposomes and oligonucleotides, may become coupled to those proteins. In the nonlimiting example illustrated in FIG. 38B, first antibody 3811 is specific to, and is coupled to, first protein 3801, while second antibody 3812 is specific to, and is coupled to, second protein 3802. Note that in this example, a plurality of second proteins 3802 are coupled to a respective one of the loci, and a plurality of second antibodies 3812 in the mixture are coupled to the proteins at that locus (the second one of such antibodies being labeled 3812' and its transposomes being labeled 3822' for ease of distinction). In this example, the portion of polynucleotide P illustrated in FIGS. 38A-38B does not include the protein for which third antibody 3813 is specific, and so that antibody (and its corresponding transposome(s) and oligonucleotide) do not become coupled to that portion of the polynucleotide. Proteins 3801 and 3802 may be transcriptionally active and thus of interest to assay, e.g., to determine which specific proteins (such as transcription factors, repressors, or the like) are bound to which specific loci of polynucleotide P.

At the particular times illustrated in FIGS. 38A and 38B, a condition of fluid 3860 optionally may be used that allows activity of antibodies 3811, 3812, 3813 and inhibits activity of transposomes 3821, 3822, 3823. For example, it is well known that different enzymes may use certain ions to function. Illustratively, transposomes 3821, 3822, 3823 may use magnesium ions (Mg2+) to function, e.g., to couple respective oligonucleotides to target polynucleotide P, while the presence or absence of magnesium ions may not affect the activity of antibodies 3811, 3812, 3813. Additionally, or alternatively, the presence of ethylenediaminetetraacetic acid (EDTA) in fluid 3860 may inhibit the activity of transposomes 3821, 3822, 3823, while the presence or absence of EDTA may not affect activity of antibodies 3811, 3812, 3813. Accordingly, by contacting polynucleotide P with fluid 3860 having a condition including presence of a sufficient amount of EDTA to inhibit activity of transposomes 3821, 3822, 3823, absence of a sufficient amount of magnesium ions for activity of transposomes 3821, 3822, 3823, or a combination of a sufficient amount of EDTA and absence of a sufficient amount of magnesium ions that activity of transposomes 3821, 3822, 3823 is inhibited, while antibodies 3811, 3812, 3813 may function properly. Additionally, or alternatively, the binding of the transposome may be inhibited in any suitable manner, e.g., reversibly blocking the binding site on the transposome, using a different temperature to bind the antibodies than is used for the transposome, and/or delaying binding of the transposase adaptors to the transposase until after the antibodies have bound so as to delay the transposome's ability to bind, and the like. Additionally, or alternatively, a sufficiently low concentration of complexes may be used that any off target tagmentation results in a product that may not be amplifiable and thus may not be detected using sequencing.

After any antibodies in fluid 3860 become coupled to respective proteins in polynucleotide P, the transposomes to which those antibodies are coupled may be activated in such a manner as to add the corresponding oligonucleotides to the polynucleotide in a manner such as illustrated in FIG. 38C. For example, a condition of fluid 3860 may be changed in such a manner as to promote activity of the transposomes. Illustratively, a sufficient amount of magnesium ions may be added to fluid 3860 for activity of transposomes 3821, 3822, 3822'. Responsive to such a change in condition of the fluid, first transposome 3821 may add first oligonucleotides 3831 to respective locations in polynucleotide P, and second transposomes 3822, 3822' may add second oligonucleotides 3832, 3832' to respective locations in polynucleotide P, while dividing the polynucleotide into a plurality of fragments. The fragments then may be released from first and second complexes 3841, 3842 and from proteins 3801 and 3802 and other chromatin 3803 to provide composition 3800' illustrated in FIG. 38D. Such releasing may be performed using proteinase K, sodium dodecyl sulfate (SDS), or both proteinase K and SDS. In addition to, or as an alternative to, the use of fluid conditions, transposomes 3821, 3822, 3823 may be selected so as to have relatively low activity, e.g., so as substantially only to tagment polynucleotide P when maintained in sufficient proximity to the polynucleotide by the corresponding antibodies. For example, transposases may be mutated to modulate their activity and/or the ME sequence may be changed to modulate the transposome's activity in a manner such as described in Reznikoff, "Tn5 as a model for understanding DNA transposition," Mol. Microbiol. 47(5): 1199-1206 (2003), the entire contents of which are incorporated by reference herein.

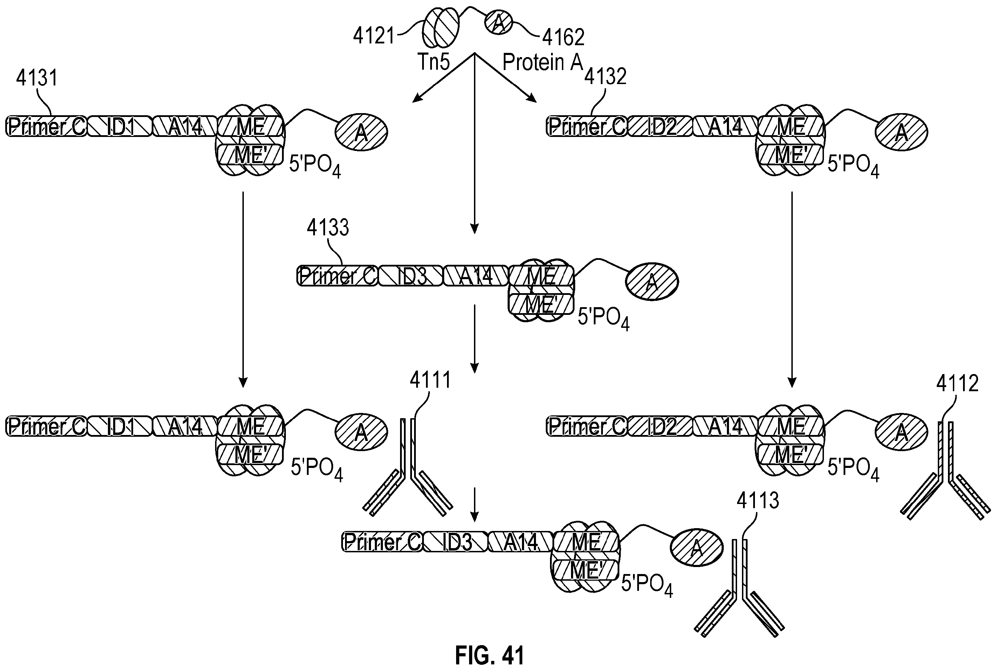

Ends of fragments 3851, 3852, which had been coupled to a protein for which an antibody had been selective, includes an oligonucleotide corresponding to that protein. One end of fragment 3853, which had not been coupled to a protein for which an antibody had been selective, includes an oligonucleotide corresponding to the protein which had been coupled to the adjacent fragment on that side, and the other end of fragment includes an oligonucleotide corresponding to the protein which had been coupled to the adjacent fragment on that side. Further details and examples of tagmentation, and example fragments generated thereby, are provided with reference to FIGS. 41, 42, 43, 44, 45, 46A-46B, and 47A-47C.

Note that a fragment's length may be related to the size and/or quantity of protein at the locus of that fragment. For example, as illustrated in FIG. 38C, the transposomes may be able to extend from the respective antibodies by a distance that is defined by the nature of the coupling between the transposome and the antibody. As such, when antibody 3811 is coupled to respective protein 3801 in polynucleotide P and transposomes 3821 is activated (e.g., using a condition of the fluid), the transposomes respectively may become coupled to regions of the polynucleotide that are relatively close to the antibody and thus relatively close to the protein, in any location that may permitted by the coupling, illustratively between 1-20 bases, or between 2-15 bases, or between 5-10 bases. Additionally, binding of transposomes may be inhibited by any proteins (e.g., chromatin 3803) occupying locations at which the transposome otherwise would become bound. Such inhibition may influence or affect the size of the fragments generated using the transposomes.

For antibodies 3812, 3812' coupled to proteins 3802, the situation is more complicated because more than one protein is coupled to that locus. As shown in FIG. 38C, one of the transposomes 3822 coupled to antibody 3812 may add the second oligonucleotide to polynucleotide P on one side of proteins 3802, and one of the transposomes 3822' coupled to antibody 3812' may add the second oligonucleotide to the polynucleotide on the other side of proteins 3802. The distance between transposomes and antibodies - and thus the distance between the proteins and the oligonucleotides which are added to polynucleotide P - may be controlled during preparation of the transposome-antibody complexes. Illustratively, transposome 3822 may add the second oligonucleotide to polynucleotide P within about 10 bases to one side of second proteins 3802, and transposome 3822 may add the second oligonucleotide to polynucleotide P within about 10 bases to the other side of second proteins 3802. Note that because second proteins include multiple proteins at that locus, the distance between the second oligonucleotide 3832 added by transposome 3822 and the second oligonucleotide 3832' added by transposome 3822' may be substantially different than the distance between the first oligonucleotides 3831 added by transposomes 3821. For example, the distance between the first oligonucleotides 3831 added by transposomes 3821 may correspond approximately to a lateral distance by which those transposomes extend on either side from antibody 3811. In comparison, the distance between the second oligonucleotides 3832 added by transposomes 3822, 3822' may correspond approximately to a lateral distance by which transposome 3822 may extend from the antibody 3812, plus a distance occupied by proteins 3802, plus a lateral distance by which transposome 3822' may extend from antibody 3812'. The number of proteins at the various loci may be determined based on the respective lengths of the subfragments. Accordingly, it may be understood that fragment 3851 has a length corresponding to the presence of one copy of protein 3801, while fragment 3852 has a length corresponding to the presence of two copies of protein 3802.

Fragments 3851, 3852, 3853 may be amplified and sequenced. As illustrated in FIG. 38E, amplification may generate extended fragments 3851', 3852', 3853' including full pairs of oligonucleotides at the ends of the fragments. The fragments generated by the corresponding transposomes, including the corresponding oligonucleotides (or lack thereof), may be sequenced in parallel with one another using any suitable method, such as by performing SBS on the fragment(s) to which the corresponding oligonucleotides are added. As such, the sequence of the fragments may be determined, in combination with the sequence of the oligonucleotides corresponding the protein(s) that had been coupled to that fragment may be determined. Because the fragments may be generated concurrently with one another, and such fragments may be individually labeled with oligonucleotides identifying the proteins that were present, the epigenetic proteins along an entire polynucleotide - or even along the polynucleotides of an entire WG sample - may be assayed in multiplex fashion to identify the particular proteins at particular loci of that polynucleotide(s). For example, a second amount of the same polynucleotide may be sequenced, e.g., using SBS, but without the use of the present epigenetic assays. The sequences of the different fragments resulting from the present epigenetic assays may be compared to the sequence of the polynucleotide, and based on such comparison the respective locations of each of the fragments within the overall polynucleotide may be determined. Based on the oligonucleotides which are at the ends of the fragments (which oligonucleotides are not present in the polynucleotide without use of the present epigenetic assays), the proteins that were coupled to those fragments may be identified.

It will be appreciated that suitable sequence oligonucleotide sequences may be used. FIG. 39A schematically illustrates example oligonucleotides that may be used in the process flow of FIGS. 38A-38E. In the nonlimiting example illustrated in FIG. 39A, oligonucleotides 3831, 3832, 3833 each include primer 3910 for use in amplifying the corresponding fragment (e.g., an A14 forward primer); a respective barcode 3921, 3922, 3923 that corresponds to the protein for which the respective antibody is specific; a respective UMI 3931, 3932, 3933 that may be used to identify the particular fragment molecule to which the protein is coupled; and a mosaic end (ME) transposon end 3940 that couples to the corresponding transposase. The oligonucleotides may include primers 3910 and ME transposon ends 3940 in common with each other, while the barcodes and UMIs are different. While individual example oligonucleotides are illustrated in FIG. 39A, each corresponding to a different protein, it will be appreciated that fluid 3860 may include a plurality of complexes that correspond to the same protein as one another, e.g., a plurality of complexes 3841, a plurality of complexes 3842, and a plurality of complexes 3843, each coupled to corresponding oligonucleotides. The UMIs of the oligonucleotides may be used to distinguish fragment molecules from one another, even when such fragments are coupled to the same types of proteins as one another. For example, FIG. 39A illustrates oligonucleotide 3831' which corresponds to the same protein as does oligonucleotide 3831, and thus includes the same barcode 3921 as oligonucleotide 3831, as well as the same primer 3910 and ME transposon end 3940 as the other oligonucleotides. However, oligonucleotide 3831' includes UMI 3931' which is different than UMI 3931 of oligonucleotide 3831. Similarly, any other oligonucleotides corresponding to the same protein as oligonucleotides 3831, 3831' may have the same primer 3910, barcode 3921, and ME transposon end 3940 as each other, but may have still different UMIs than another other of such oligonucleotides. As such, each fragment generated using such oligonucleotides may become coupled to an oligonucleotide including a different UMI, and such UMI may be used to identify which protein had been coupled to that specific fragment molecule.

For example, FIG. 39B schematically illustrates fragments coupled to example oligonucleotides of FIG. 39A, more specifically fragment 3851' coupled to oligonucleotide 3831 at each of its ends, and fragment 3851" coupled to oligonucleotide 3831' at each of its ends. Fragments 3851', 3851" may be generated using operations such as described with reference to FIGS. 38A-38E, in which different molecules of complex 3841 selectively couple to different molecules of protein 3802 and thus generate different fragment molecules. From the barcodes 3921 within the sequences of oligonucleotides 3831, 3831' it may be understood that the fragments were coupled to the same type of protein as one another, and from the UMIs within the sequences of oligonucleotides 3831, 3831' it may be understood that the fragments were generated using different molecules of the complex 3841 than one another. Note that during amplification of the fragments such as described with reference to FIG. 38E, both the barcode and the UMI are amplified, and as such each resulting amplicon may be correlated to the correct protein molecule that was initially coupled to the respective molecule of complex 3841. It will be appreciated that other fragments, to which were coupled other proteins, may have other oligonucleotides at their ends. Additionally, the lengths of the fragments may be significantly longer than that of the oligonucleotides. Additional, nonlimiting examples of oligonucleotides and fragments are provided further below with reference to FIG. 44.

Prior to contact with polynucleotide P, the complexes may be prepared by coupling the transposomes to respective antibodies in any suitable manner. Illustratively, each the antibodies may be coupled to the corresponding transposome via a covalent linkage, or via a non-covalent linkage. Covalent linkages may be formed, illustratively, copper(I)-catalyzed click reaction, or strain-promoted azide-alkyne cycloaddition. Non-covalent linkages may be formed in any suitable manner. For example, FIGS. 40A-40C schematically illustrate further details of a complex such as may be used in the process flow of FIGS. 38A-38E. It will be appreciated that complexes 3841 illustrated in FIGS. 40A-40C may include any suitable number of transposomes 3821 coupled to antibody 3811, although only one such transposome is shown for simplicity of illustration. It will also be appreciated that the particular coupling between antibody 3811 and transposome 3821 may define the distance by which the transposome may extend from the antibody in a manner such as described with reference to FIGS. 38A-38E, e.g., about 1-20 bases, or about 2-15 bases, or about 5-10 bases.

In some examples, in a manner such as illustrated in FIG. 40A, complex 3841 may include transposome 3821 coupled to antibody 3811 via reaction between any suitable elements, such as Click chemistry reactants or an antigen-antibody coupling. For example, antibody 3811 may include or may be coupled (covalently or non-covalently) to element 4062, and transposome 3821 may be coupled (covalently or non-covalently) to element 4061 which may be suitably reacted with element 4062 is coupled to couple antibody 3811 to transposome 3821. In some examples, antibody 3811 may include multiple active sites. One or more of the active sites may be used to couple corresponding transposome(s) to the antibody 3811 in a manner such as illustrated in FIG. 40A, and another one or more of the active sites may be used to selectively couple the antibody to a protein on the polynucleotide. In one specific example, transposome 3821 is coupled to Protein A (optionally, transposome 3821 and Protein A form a fusion protein), and the protein A may be coupled to antibody 3811 in a manner such as described in greater detail with reference to FIG. 41. In some examples, the transposomes may be modified so as to be targeted to the desired antibodies, e.g., so as to be fused with the common regions of antibodies, but it will be appreciated that any suitable number of transposome(s) may be coupled to any suitable portion(s) of antibodies using any suitable technique. Alternatively, in a manner such as illustrated in FIG. 40B, complex 3841 may include transposome 3821 coupled to antibody 3811 via an alternative coupling between element 4061' and element 4062'. It will be appreciated that complex 3841 may include one or more additional transposomes 3821 coupled to antibody 3811, although only one such transposome is shown in FIG. 40B for simplicity of illustration. Elements 4061, 4061' and 4062, 4062' may for example, include reactants such as SNAP proteins with O-benzylguanine; CLIP proteins with O-benzylcytosine; SpyTag with SpyCatcher; biotin with streptavidin; NTA with His-Tag; anti-FLAG antibodies and FLAG tags; and the like.

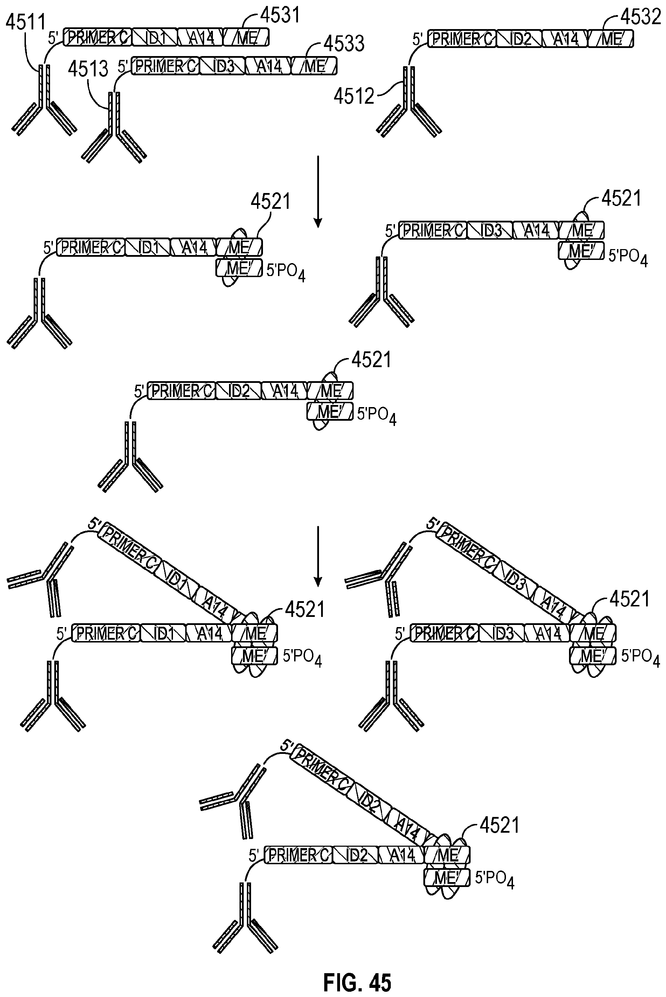

As yet another example, in a manner such as illustrated in FIG. 40C, partial complex 3841' may include antibody 3811 non-covalently coupled to a first subunit (transposase) 3821' of a transposome via an oligonucleotide 4063 which antibody 3811 has been modified to include. Oligonucleotide 4063 may include the sequence corresponding to the type of protein for which antibody 3811 is selective and the ME sequence. A complementary oligonucleotide may be annealed to that ME sequence only to make it double stranded. Antibody 3811 and the transposase (single subunit) may be incubated so as to bind that double stranded ME. A similar operation may be performed on the other subunit of the transposome, e.g., in a manner similar to that described with reference to FIG. 45. The two subunits then may be dimerized to form complex 3841. The resulting transposome 3821 may include two separate ME sequences, each of which couples an antibody to a respective subunit. Custom oligonucleotide-conjugated antibodies are commercially available, or may be prepared using known techniques, e.g., such as described in the following references, the entire contents of each of which are incorporated by reference herein: Gong et al., "Simple method to prepare oligonucleotide-conjugated antibodies and its application to multiplex protein detection in single cells," Bioconjugate Chem. 27: 217-225 (2016); and Stoeckius et al., "Simultaneous epitope and transcriptome measurement in single cells," Nature Methods 14: 865-868 (2017).

Additional nonlimiting examples of the present transposome-antibody complexes, methods of using such complexes for tagmentation, oligonucleotides that may be added during tagmentation, and amplification of such oligonucleotides, now will be described with reference to FIGS. 41, 42, 43, 44, 45, 46A-46B, and 47A-47C.

Referring now to FIG. 41, another example flow of operations for generating complexes respectively including a transposome coupled to an antibody is schematically illustrated. A plurality of fusion proteins, each including transposome 4121 coupled to protein A 4162, may be generated in a manner similar to that described in Kaya-Okur et al., "CUT&Tag for efficient epigenomic profiling of small samples and single cells," Nature Communications 10: article 1930 (2019), the entire contents of which are incorporated by reference herein. Different volumes of the fusion proteins may be contacted with different oligonucleotides that correspond to different proteins. For example, from the 5' end, first oligonucleotide 4131 may include a forward primer (e.g., Primer C), a first barcode sequence (unique sequence referred to as "ID1") which is designated as corresponding to a first protein, a sequencing primer (e.g., A14), and a duplex for insertion into the corresponding transposase that includes a forward ME sequence hybridized to a complementary ME' sequence. Similarly, from the 5' end, second oligonucleotide 4132 may include a forward primer (e.g., Primer C), a second barcode sequence (unique sequence referred to as "ID2") which is designated as corresponding to a second protein, a sequencing primer (e.g., A14), and a duplex for insertion into the transposase that includes a forward ME sequence hybridized to a complementary ME' sequence. Similarly, from the 5' end, third oligonucleotide 4133 may include a forward primer (e.g., Primer C), a third barcode sequence (unique sequence referred to as "ID3") which is designated as corresponding to a third protein, a sequencing primer (e.g., A14), and a duplex for insertion into the transposase that includes a forward ME sequence hybridized to a complementary ME' sequence.

The different volumes of the fusion proteins, with the oligonucleotides coupled thereto, may be kept separate from one another and coupled to respective antibodies that are selective for the proteins to which the barcode sequences respectively correspond. For example, protein A 4162 of the fusion protein coupled to first oligonucleotide 4131 may be coupled to first antibody 4111; protein A 4162 of the fusion protein coupled to second oligonucleotide 4132 may be coupled to second antibody 4112; and protein A 4162 of the fusion protein coupled to third oligonucleotide 4133 may be coupled to second antibody 4113, in a manner similar to that described in Kaya-Okur et al., "CUT&Tag for efficient epigenomic profiling of small samples and single cells," Nature Communications 10: article 1930 (2019), the entire contents of which are incorporated by reference herein. The resulting transposome-antibody complexes thus are coupled to oligonucleotides that correspond to the proteins for which the respective antibodies are selective.

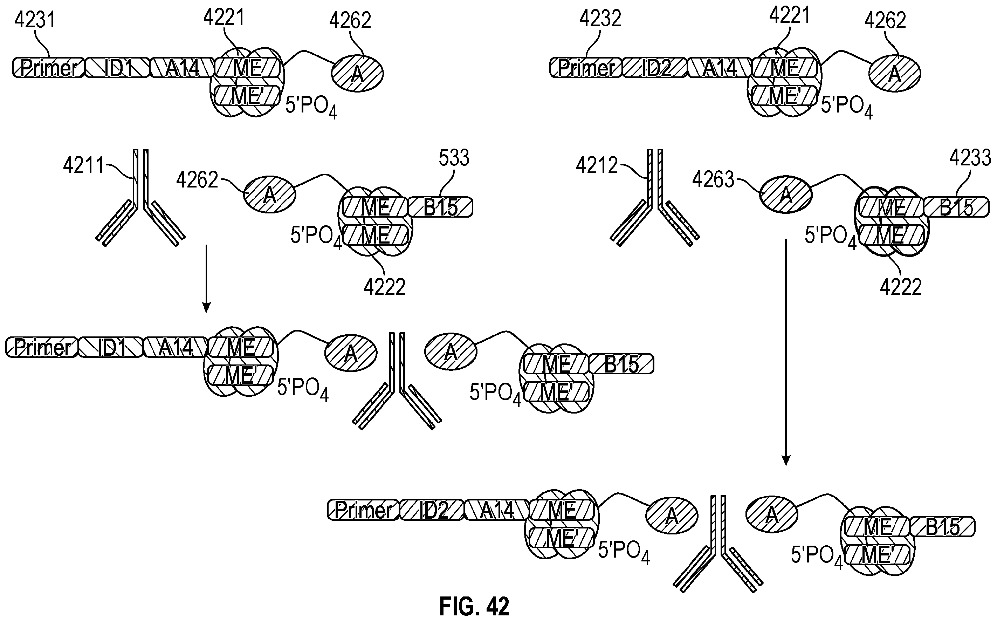

It will be appreciated that any suitable number of transposomes may be coupled to an antibody to provide the present complexes, and that such transposomes need not necessarily include the same oligonucleotides as one another. For example, FIG. 42 schematically illustrates an example flow of operations for generating complexes respectively including multiple transposomes coupled to an antibody. A plurality of fusion proteins, each including transposome 4221 coupled to protein A 4262, may be generated in a manner similar to that described with reference to FIG. 41. Different volumes of the fusion proteins may be contacted with different oligonucleotides that correspond to different proteins. For example, first oligonucleotide 4231 may include a forward primer, a first barcode sequence (unique sequence referred to as "ID1") which is designated as corresponding to a first protein, a sequencing primer (e.g., A14), and a duplex for insertion into the corresponding transposase that includes a forward ME sequence hybridized to a complementary ME' sequence. Similarly, second oligonucleotide 4232 may include a forward primer, a second barcode sequence (unique sequence referred to as "ID2") which is designated as corresponding to a second protein, a sequencing primer (e.g., A14), and a duplex for insertion into the transposase that includes a forward ME sequence hybridized to a complementary ME' sequence. Additionally, third oligonucleotide 4231 may include a reverse primer (e.g., B15), and a duplex for insertion into the corresponding transposase 4222 that includes a forward ME sequence hybridized to a complementary ME' sequence.

The different volumes of the fusion proteins, with the oligonucleotides coupled thereto, may be kept separate from one another and coupled to respective antibodies that are selective for the proteins to which the barcode sequences respectively correspond. For example, in a manner similar to that described with reference to FIG. 41, protein A 4262 of the fusion protein coupled to first oligonucleotide 4231, and protein A 4262 of the fusion protein coupled to third oligonucleotide 4233, may be coupled to first antibody 4211; and protein A 4262 of the fusion protein coupled to second oligonucleotide 4232, and protein A 4262 of the fusion protein coupled to third oligonucleotide 4233, may be coupled to second antibody 4212. The resulting transposome-antibody complexes thus are coupled to oligonucleotides that correspond to the proteins for which the respective antibodies are selective.

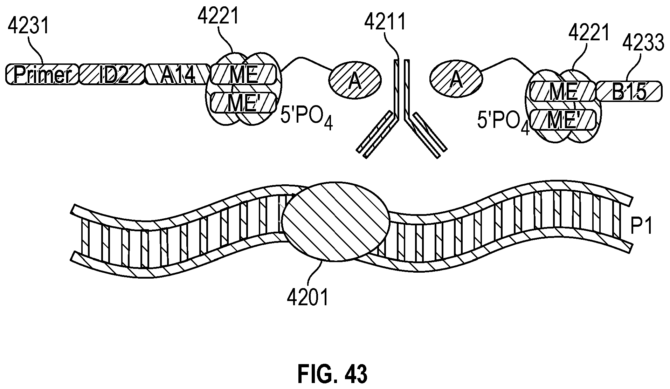

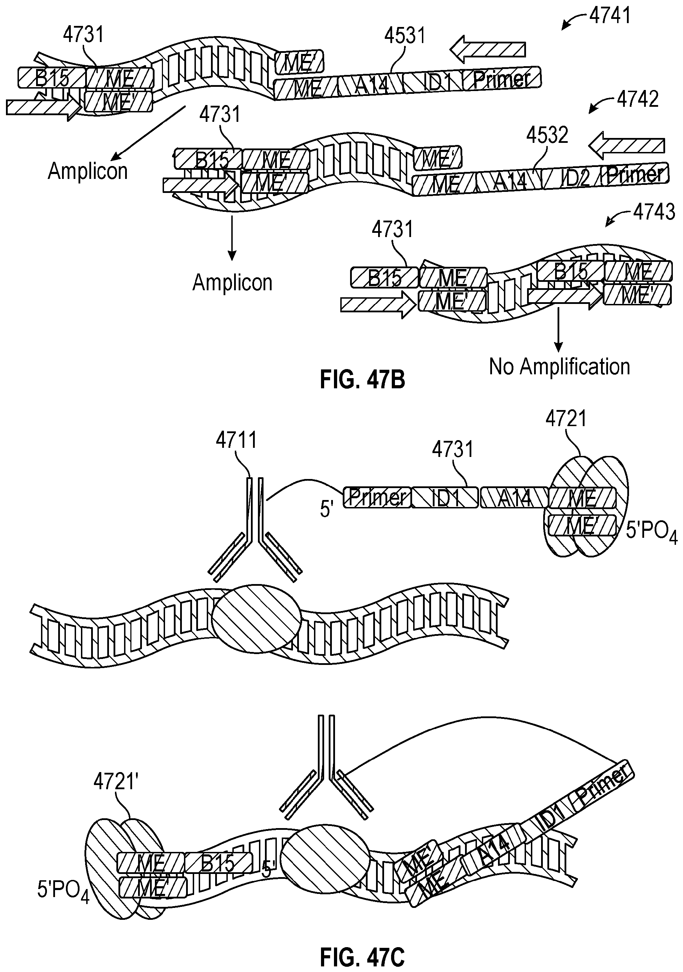

Complexes prepared in a manner such as described with reference to FIGS. 41 and 42 may be used in a manner similar to that described with reference to FIGS. 38A-38E. For example, FIG. 43 schematically illustrates an operation in which the antibody of one of the complexes of FIG. 42 selectively binds to protein 4201 at a locus of polynucleotide P1. As illustrated in FIG. 43, selective binding of antibody 4211 to protein 4201 while transposomes 4221, 4222 are inactive brings transposomes 4221, 4222 sufficiently close to polynucleotide P1 such that when the transposomes are activated they respectively may tagment the polynucleotide with oligonucleotide 4231 on one end and with oligonucleotide 4233 on the other end. It will be appreciated that polynucleotide P1 may be contacted with a pool of different complexes that are selective for different proteins that may, or may not be, at different loci of polynucleotide P1. Fragments generated in a manner such as illustrated in FIG. 43 may be amplified and sequenced so as to determine the identity of protein 4201 coupled to that fragment. Note that due to variations in their manufacture, some complexes that are used to generate fragments may not necessarily include both transposome 4221 and transposome 4222; instead, some fragments may include two transposomes 4221 and no transposomes 4222, or may include two transposomes 4222 and no transposomes 4221. In a manner similar to that described with reference to FIG. 47B, fragments generated by any such complexes may not include all amplification adapters that are needed to amplify such fragments, e.g., using operations such as will now be described with reference to FIG. 44.

FIG. 44 schematically illustrates an example flow of operations for amplifying a fragment of a polynucleotide following tagmentation by transposomes of a complex. Following tagmentation and purification to remove the protein and transposome-antibody complex, fragment 4431 may include two strands hybridized to one another. The first strand, from the 5' end to the 3' end, may include a primer (e.g., Primer C), a first barcode sequence (unique sequence referred to as "ID1") which is designated as corresponding to a first protein, a sequencing primer (e.g., A14), a forward ME sequence, and fragment region F1 which transposomes 4221 and 4222 cut from polynucleotide P1. The second strand 4431", from the 5' end to the 3' end, may include a reverse primer (e.g., B15), an ME sequence, and complementary fragment region F1' which transposomes 4221 and 4222 cut from polynucleotide P1. As illustrated in FIG. 44, the single-stranded portions of fragment 4431 may be extended so as to form a full duplex including strand 4431' and complementary strand 4431". Strand 4431', from the 5' end to the 3' end, may include a primer (e.g., Primer C), a first barcode sequence (unique sequence referred to as "ID1") which is designated as corresponding to a first protein, a sequencing primer (e.g., A14), a forward ME sequence, fragment region F1 which transposomes 4221 and 4222 cut from polynucleotide P1, complementary ME' sequence, and complementary reverse primer (e.g., B15'). Strand 4431", from the 5' end to the 3' end, may include a reverse primer (e.g., B15), an ME sequence, complementary fragment region F1' which transposomes 4221 and 4222 cut from polynucleotide P1, complementary forward primer (e.g., A14), complementary first barcode sequence (complement ID1' of ID1), and complementary primer (e.g., Primer C').

As illustrated in FIG. 44, primers and sample indices may be annealed to fragments 4431', 4431" for subsequent use in amplifying the fragments. For example, primer 4450 annealed to complementary strand 4431" may include (a) a primer (e.g., primer C) which may be annealed to the complementary forward primer (e.g., primer C') of strand 4431", (b) a sample index (unique identifier corresponding to the sample), and (c) an amplification primer (e.g., P5 primer). Primer 4451 may include (a) a primer (e.g., primer B15) which may be annealed to the complementary reverse primer (e.g., primer B15') of strand 4431", (b) a sample index (unique identifier corresponding to the sample), and (c) an amplification primer (e.g., P7 primer). As illustrated in FIG. 44, primers 4451, 4450 may be extended so as to form a full duplex 4441 between primer-extended strand 4441' and complementary primer-extended strand 4441". Strand 4441' may be similar to strand 4431' but include at its 3' end the sample index and amplification primer (e.g., P7), and may include at its 5' end the sample index and amplification primer (e.g., P5). Strand 4441" may be the complement of strand 4441'.

While FIGS. 41 and 42 illustrate one example preparation of the present complexes, it will be appreciated that other preparations suitably may be used. For example, FIG. 45 schematically illustrates another example flow of operations for generating complexes respectively including a transposome coupled to multiple antibodies. A plurality of antibodies, each coupled to different oligonucleotides, may be prepared in a manner such as described in Weiner et al., "Preparation of single- and double-oligonucleotide antibody conjugates and their application for protein analytics," Scientific Reports 10: 1457 (2020). In the example illustrated in FIG. 45, first antibody 4511 may be selective for a first protein and may be coupled to the 5' end of first oligonucleotide 4531; second antibody 4512 may be selective for a second protein and may be coupled to the 5' end of second oligonucleotide 4532; and third antibody 4513 may be selective for a third protein and may be coupled to the 5' end of third oligonucleotide 4533. From the 5' end, first oligonucleotide 4531 may include a forward primer (e.g., Primer C), a first barcode sequence (unique sequence referred to as "ID1") which is designated as corresponding to the first protein, a sequencing primer (e.g., A14), and a duplex for insertion into the corresponding transposase that includes a forward ME sequence. Similarly, from the 5' end, second oligonucleotide 4532 may include a forward primer (e.g., Primer C), a second barcode sequence (unique sequence referred to as "ID2") which is designated as corresponding to the second protein, a sequencing primer (e.g., A14), and a duplex for insertion into the transposase that includes a forward ME sequence. Similarly, from the 5' end, third oligonucleotide 4533 may include a forward primer (e.g., Primer C), a third barcode sequence (unique sequence referred to as "ID3") which is designated as corresponding to the third protein, a sequencing primer (e.g., A14), and a duplex for insertion into the transposase that includes a forward ME sequence. In the nonlimiting example illustrated in FIG. 45, the different antibodies coupled to respective oligonucleotides contacted with transposases 4521 which become coupled to the respective oligonucleotides. The transposases then optionally may be dimerized as illustrated in FIG. 45 to form transposomes, each of which is coupled to two antibodies. The resulting transposome-antibody complexes thus are coupled to oligonucleotides that correspond to the proteins for which the respective antibodies are selective. The complexes then may be pooled. It will be appreciated that transposomes such as described herein may include any suitable number of transposases, e.g., may include a transposase monomer, dimer, or tetramer.

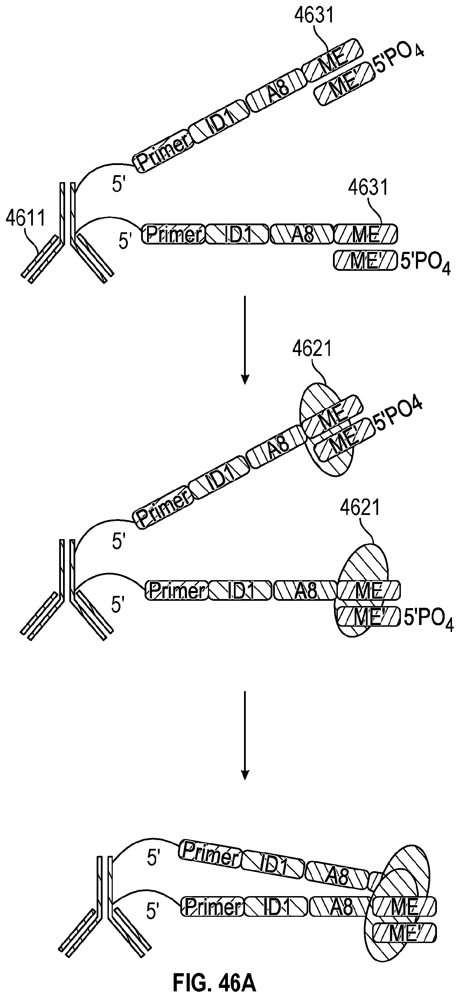

It will further be appreciated that any suitable number of transposomes may be coupled to an antibody to provide the present complexes, and that such transposomes need not necessarily be coupled to the same oligonucleotides as one another. For example, FIGS. 46A-46B schematically illustrate example flows of operations for generating complexes respectively including transposomes coupled to an antibody. For example, as illustrated in FIG. 46A, antibody 4611 may be selective for a protein and may be coupled to the 5' end of each of two oligonucleotides 4631 including ME duplexes. Oligonucleotide 4631 may have a similar sequence as first oligonucleotide 4531. In the nonlimiting example illustrated in FIG. 46A, antibody 4611 coupled to oligonucleotides 4631 may be contacted with transposases 4621 which become coupled to the ME duplexes of the oligonucleotides 4631. The transposases 4621 then may dimerize as shown in FIG. 46A to form a transposome coupled to antibody 4611. The resulting transposome-antibody complexes thus are coupled to oligonucleotides that correspond to the proteins for which the respective antibodies are selective.

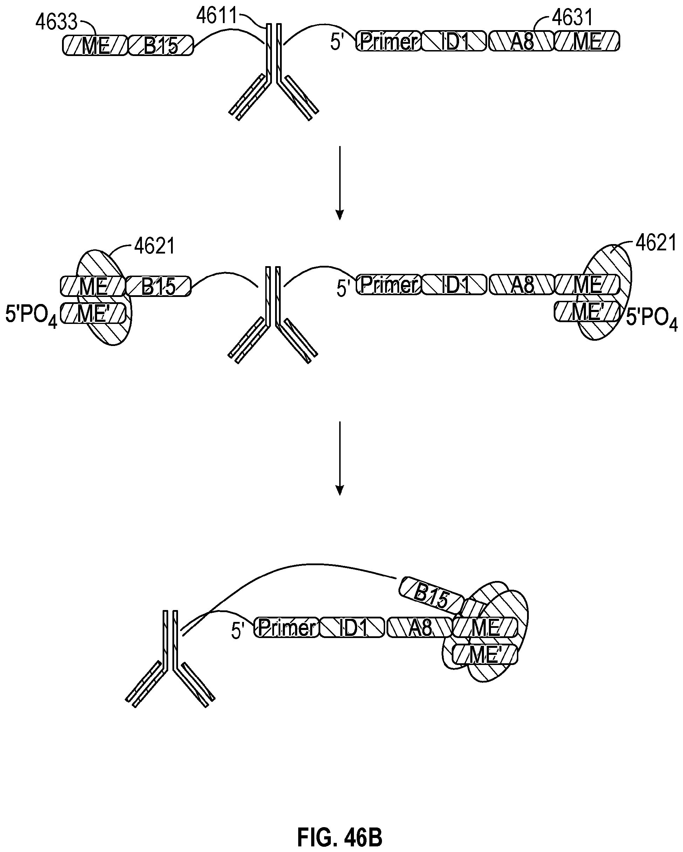

In the example illustrated in FIG. 46B, antibody 4611 may be selective for a protein and may be coupled to the 5' end of first oligonucleotide 4631 and to the 5' end of third oligonucleotide 4633. Oligonucleotide 4631 may have a similar sequence as oligonucleotide 4531 and an ME duplex. Oligonucleotide 4633 may include a reverse primer (e.g., B15), and a duplex for insertion into the corresponding transposase that includes a forward ME sequence. In the nonlimiting example illustrated in FIG. 46B, antibody 4611 coupled to oligonucleotides 4631, 4633 may be contacted with transposases 4621 which become coupled to the ME duplexes of the oligonucleotides 4631. The transposases 4621 then may dimerize as shown in FIG. 46B to form a transposome coupled to antibody 4611. The resulting transposome-antibody complexes thus are coupled to oligonucleotides that correspond to the proteins for which the respective antibodies are selective. Note that dimerization of transposases 4621, such as described with reference to FIGS. 46A-46B, may be performed at sufficiently low concentrations that the transposases 4621 coupled to the same antibody as one another are far more likely to dimerize with each other than with transposases coupled to other antibodies.

Complexes prepared in a manner such as described with reference to FIGS. 45 and 46A-46B may be used in a manner similar to that described with reference to FIGS. 38A-38E or FIG. 43, and may be used to generate fragments that then may be amplified in a manner such as described with reference to FIG. 44.

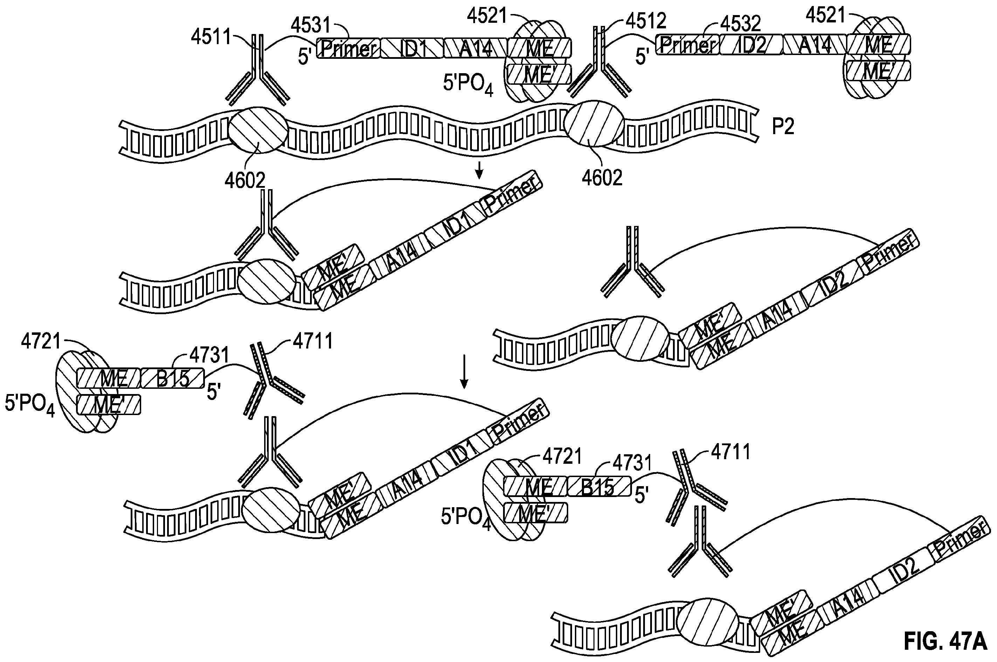

Still other complexes and methods may be used to tagment a polynucleotide. For example, FIG. 47A schematically illustrates an example flow of operations in which proteins at respective loci of a polynucleotide are sequentially bound by antibodies of primary and secondary complexes. For example, as illustrated in FIG. 47A, polynucleotide P2, including proteins 4701 and 4702 at respective loci, is contacted with complexes such as described with reference to FIG. 45 or FIG. 46A, e.g., a complex including first oligonucleotide 4511 coupled to first oligonucleotide 4531, and a transposome including transposase 4521 coupled to first oligonucleotide 4531; and a complex including second oligonucleotide 4512 coupled to second oligonucleotide 4532, and a transposome including transposase 4521 coupled to second oligonucleotide 4532. As illustrated in FIG. 47A, selective binding of antibody 4511 to protein 4701 brings transposase 4521 sufficiently close to polynucleotide P2 so as to tagment the polynucleotide with oligonucleotide 4531 on one end. Similarly, selective binding of antibody 4512 to protein 4702 brings transposase 4521 sufficiently close to polynucleotide P2 so as to tagment the polynucleotide with oligonucleotide 4532 on one end. It will be appreciated that polynucleotide P1 may be contacted with a pool of different complexes that are selective for different proteins that may, or may not be, at different loci of polynucleotide P1.

As also illustrated in FIG. 47A, polynucleotide P2 having the complexes selectively coupled thereto then may be contacted with a mixture of second complexes that are specific to the first complexes. For example, in a manner similar to that described with reference to FIG. 45, each of the second complexes may include an antibody 4711, an oligonucleotide 4731 coupled to the antibody, and a transposome including transposase 4721 coupled to the oligonucleotide. Antibody 4711 may recognize the antibody common region, and thus may be compatible with all of antibodies 4711 and 4712, as well as other antibodies with which polynucleotide P2 may be contacted. As illustrated in FIG. 47A, binding of antibody 4711 to antibody 4711 brings transposase 4721 sufficiently close to polynucleotide P2 so as to tagment the polynucleotide with oligonucleotide 4731 on the opposite end from oligonucleotide 4731. Similarly, binding of antibody 4711 to antibody 4712 brings transposase 4721 sufficiently close to polynucleotide P2 so as to tagment the polynucleotide with oligonucleotide 4731 on the opposite end from oligonucleotide 4732.

FIG. 47B schematically illustrates example fragments of the polynucleotide of FIG. 47A following tagmentation. One 5' end of fragment 4741 includes oligonucleotide 4731 to which a forward primer may be annealed in a manner similar to that described with reference to FIG. 44, and the other 5' end of fragment 4741 includes oligonucleotide 4731 to which a reverse primer may be annealed, and the fragment then amplified, in a manner similar to that described with reference to FIG. 44. One 5' end of fragment 4742 includes oligonucleotide 4732 to which a forward primer may be annealed in a manner similar to that described with reference to FIG. 44, and the other 5' end of fragment 4742 includes oligonucleotide 4732 to which a reverse primer may be annealed, and the fragment then amplified, in a manner similar to that described with reference to FIG. 44. In some circumstances, although antibodies 4711 are specific to antibodies 4711, 4712, they may bind elsewhere as well and as such the transposomes coupled thereto may generate fragments 4743 including oligonucleotides 4731 on both ends. Because only reverse primers (e.g., B15) may anneal to such fragments, the fragments may not be amplified.

Note that a secondary antibody need not necessarily be used to provide a reverse primer (e.g., B15) suitable for use in amplifying a fragment which has been tagmented to include oligonucleotide 4731 in a manner such as described with reference to FIG. 47A. For example, as illustrated in FIG. 47C, a complex including antibody 4711 and transposase 4721 may be used to tagment a polynucleotide in a manner similar to that described with reference to FIG. 47A. Standard transposition on the entire genome then may be performed using a transposome 4721' which is loaded with, e.g., a B15-ME sequence, and which is not coupled to any antibody. Transposome 4721' may tagment the entire genome, but only regions that have both the primer (via tagmentation using oligonucleotide 4731) and the reverse primer (e.g., B15) in a similar manner to fragment 4741 described with reference to FIG. 47B.

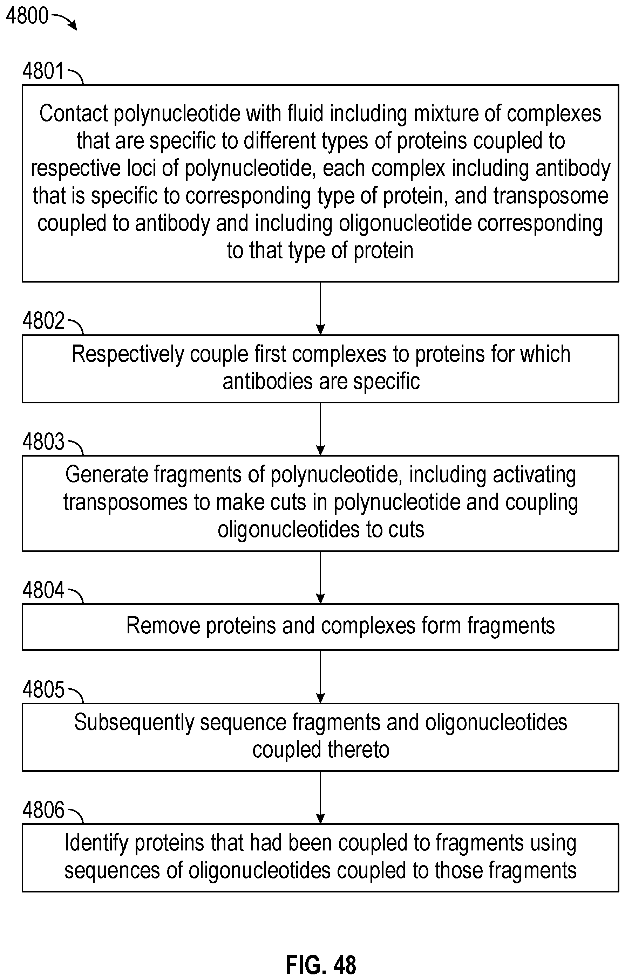

FIG. 48 illustrates an example flow of operations in a method for targeted epigenetic assays. Method 4800 illustrated in FIG. 48 may be used to characterize proteins coupled to respective loci of a polynucleotide, and may include contacting the polynucleotide with a mixture of complexes that are specific to different types of proteins that may or may not be coupled to respective loci of the polynucleotide (operation 4801). Each of the complexes may include an antibody that is specific to a corresponding type of protein, and a transposome coupled to the antibody and including an oligonucleotide corresponding to that type of protein. Example complexes are described with reference to FIGS. 40A-40C, 41, 42, 45, 46A-46B, and 47A-47C. Example oligonucleotides are described with reference to FIGS. 39A-39B and 44.

Method 4800 illustrated in FIG. 48 further may include, respectively coupling the complexes to proteins for which the antibodies are specific (operation 4802). Optionally, operation 4802 may include deactivating the transposomes. Example conditions for deactivating transposomes while coupling antibodies to proteins are described with reference to FIGS. 38A-38B. Additionally, or alternatively, a sufficiently low concentration of complexes may be used that any off target tagmentation results in a product that may not be amplifiable and thus may not be detected using sequencing.

Method 4800 illustrated in FIG. 48 further may include generating fragments of the polynucleotide, including activating the transposomes to make cuts in the polynucleotide and coupling the oligonucleotides to the fragments (operation 4803). Example conditions for activating transposomes are described with reference to FIG. 38C. Example fragments to which oligonucleotides may be coupled using transposomes are described with reference to FIGS. 38D-38E, 43, 44, and 47A-47C.

Method 4800 illustrated in FIG. 48 further may include removing the proteins and complexes from the fragments (operation 4804). Example fluid conditions to remove proteins and complexes are described with reference to FIG. 38D. Note that the proteins and complexes may be removed at any suitable step prior to sequencing.

Method 4800 illustrated in FIG. 48 further may include subsequently sequencing the fragments and the oligonucleotides coupled thereto (operation 4805). For example, SBS may be performed on the fragments and the oligonucleotides coupled thereto.

Method 4800 illustrated in FIG. 48 further may include identifying the proteins that had been coupled to respective fragments using the sequences of the oligonucleotides coupled to those fragments (operation 4806). For example, in a manner such as described with reference to FIG. 38E, a second amount of the same polynucleotide may be sequenced, e.g., using SBS, but without the use of the present epigenetic assays. The sequences of the different fragments resulting from the present epigenetic assays may be compared to the sequence of the polynucleotide, and based on such comparison the respective locations of each of the fragments within the overall polynucleotide may be determined. Based on the oligonucleotides which are at the ends of the fragments (which oligonucleotides are not present in the polynucleotide without use of the present epigenetic assays), the proteins that respectively were coupled to those fragments may be identified.

### WORKING EXAMPLES

The following examples are intended to be purely illustrative, and not limiting of the present invention.

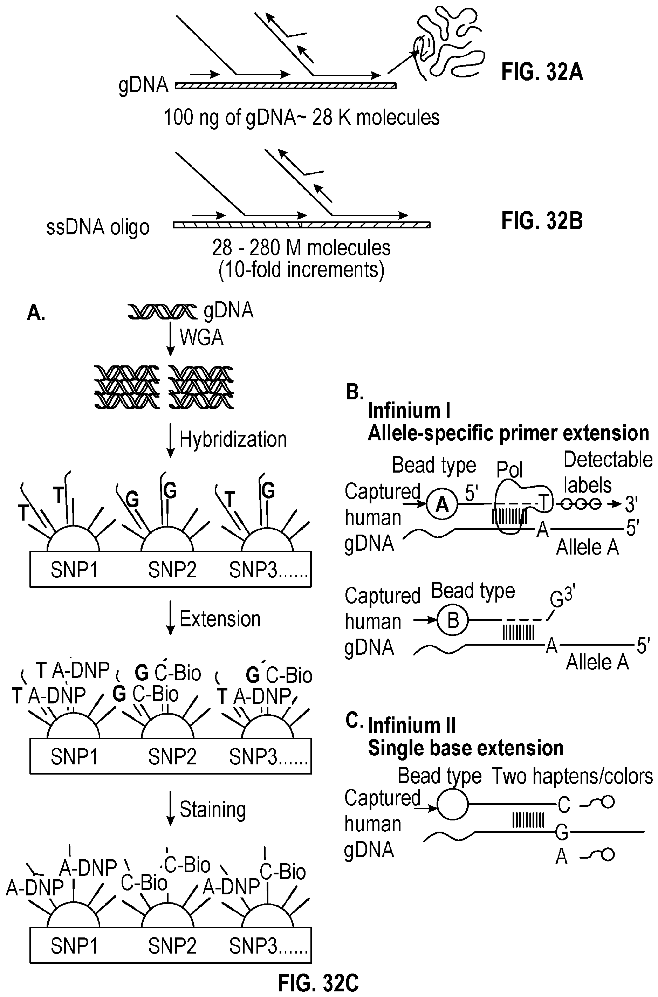

Illumina, Inc.'s Infinium platform was designed to detect millions of single nucleotide polymorphisms (SNPs) per genomic sample. For example, FIGS. 32A-32C schematically illustrate example operations and compositions for use in whole genome amplification (WGA) using random-primed, isothermal multiple displacement amplification (MDA), as implemented on the Infinium platform. More specifically, FIG. 32A illustrates WGA using random-primed, isothermal MDA. MDA uses random primers and strand-displacing DNA polymerase to exponentially amplify genomic DNA with minimal representation bias. For example, a 3-hour incubation leads to ~100 µg DNA yield, independent of DNA input (illustratively, > 10 ng, std. workflow uses 100 ng of input gDNA representing ~28K target molecules). This may mitigate PCR bottleneck due to the isothermal requirement. FIG. 32B illustrates use of synthetic oligonucleotides with about 101 nucleotides to model denatured DNA that can hybridize with random primers. The segment on the left represents the probe complement and the segment on the right represents the overhang. A series of input concentrations were tested that ranged from 20 to 280 M molecules. FIG. 32C illustrates the Infinium workflow capability of simultaneously detecting millions of SNPs. For further details regarding WGA and the Infinium platform and its use, see the following references, the entire contents of each of which are incorporated by reference herein: Gunderson et al., "Decoding randomly ordered DNA arrays," Genome Research 14(5): 870-877 (2004); Gunderson et al., "Whole genome genotypic technologies on the BeadArray™ platform," Biotechnol. 2: 41-49 (2007); and Peiffer et al., "High-resolution genomic profiling of chromosomal aberrations using Infinium whole-genome genotyping," Genome Research 16(9): 1136-1148 (2006).

In the present examples, beads were conjugated to a single 95 nucleotide (nt) long synthetic oligonucleotide (oligo). The oligo sequence included two domains: a 45-nt decode segment and a 50-nt probe. The beads were loaded onto a microfabricated beadchip. Sequencing by hybridization was used to generate a spatial decode map based on the decode sequence. Mapping the decode enabled the corresponding classification of the probe that was used to bind to a sample target strand. The beadchip construction was completed with a hybseal that partitioned regions into wells for individual sample loading.

Fragmented WGA materials were then loaded onto the beadchip wells and in the presence of a buffer incubated at temperatures suitable for hybridization of the SNP probes to the corresponding DNA targets. After a wash, the sample wells were subjected to a polymerase extension reaction to incorporate the next correct non-extendable dideoxynucleotide that was hapten labeled. Post extension, the sample wells were treated with a stringency wash to remove the hybridized target. The hapten labels were subsequently exposed to three rounds of immunostaining for robust target detection.

The DNA samples (targets) used in the foregoing analysis were prepared for genotyping by amplifying genomic DNA with a WGA method. WGA uses a proprietary multiple displacement amplification method that is isothermal, fast, efficient, and cost effective (FIG. 32A). Genomic DNA (gDNA) was chemically denatured, and random sequence primers were hybridized. The gDNA hybridized to random primers were then mixed with an isothermal extension formulation that contained a strand displacement polymerase, catalytic metal, and dNTPs. A fraction of the dTTP was substituted with dUTP, which allowed the products to be fragmented to shorter segments (less than about 500 base pairs on average) with uracil-DNA glycosylase (UDG) to excise the base followed by heat to break the remaining phosphate bond. The fragments were designed to sample the SNPs of interest independently.

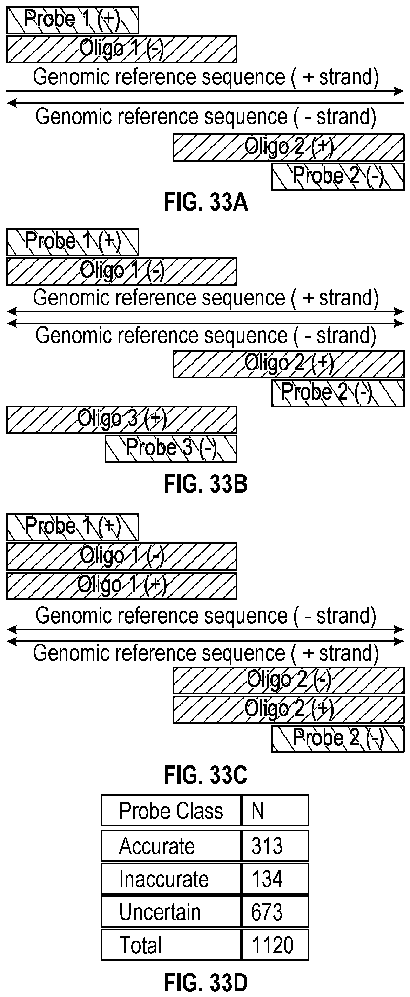

It was demonstrated that the Infinium workflow could be extended to detect synthetic oligos with similar sensitivity as WGA DNA (~1 M-10 M molecule range). Oligos with 101 nts were synthesized using established phosphoramidite oligosynthesis. FIGS. 33A-33C schematically illustrate example synthetic oligonucleotide sequences, which were used to demonstrate proof of concept. More specifically, FIG. 33A illustrates a synthetic oligo sequence representative of a human genome segment (101 nt) that was synthesized. The representative segment was selected from the - or + strand. The synthetic oligo segment included a 50 nt segment that complemented a probe sequence on the Global Screening Array (GSA) pharmacogenomic (PGx) beadchip (commercially available from Illumina, Inc., San Diego CA). FIG. 33B illustrates the full complements from the sequences in 33A, which were synthesized and utilized to model double stranded DNA (dsDNA). The dsDNA targets were used for potential enzyme detection schemes described herein. FIG. 33C illustrates synthetic oligo targets with overlapping regions on the human genome, which were tested to demonstrate robustness to target probe activity. FIG. 33D is a table with the corresponding number of targets synthesized for each probe class.

Additionally, two scenarios were modeled to demonstrate utility: i) the full complement of the 101 nt oligo was synthesized to represent a dsDNA (FIG. 33B) and ii) 101 nt segments overlapped when mapped on genome sections. dsDNA substrates were used for subsequent enzymatic activity with certain implementations. Robustness to overlapping genome segments demonstrated the robustness to cross-reactivity.

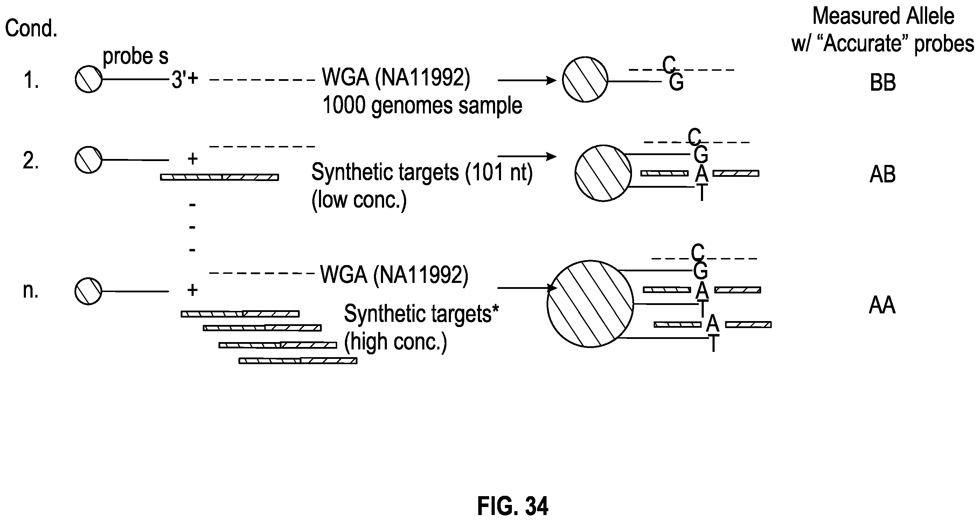

The synthetic model composed of 101 nt oligo sequence targets was selected to perform detection with an on-market GSA PGx beadchip. The synthetic targets were designed to report an alternate allele than what was expected using human genomic DNA input (NA11922). For example, if the WGA sample derived from NA11922 resulted in an AA allele, then successful binding of the synthetic target resulted in an AB allele when the synthetic target and the WGA target were stoichiometrically balanced. Increasing the synthetic target concentration shifted the allele detection to BB exclusively. For example, FIG. 34 schematically illustrates an example synthetic model system that was used to evaluate detection of synthetic oligonucleotides. In FIG. 34, condition 1 corresponds to the control condition with WGA NA11992 DNA tested with three probe classes (accurate, inaccurate, uncertain). The accurate class of probes leads to an AA allele result in the absence of synthetic targets. Condition 2 corresponds to probes tested with WGA and low input amounts of synthetic targets. Increasing the input synthetic target amount to about 3 pM led to a heterozygous AB allele signal with the accurate probe class. Condition n corresponds to probes tested with an increasing amount of synthetic target, leading to a dominant signal with the opposite allele (BB) with the accurate probe class. The allele readout was obtained from GenomeStudio software.

The GDA PGx beadchip contains a subset of probes for rare alleles that only detect either AA or BB alleles (conditions 1 and *n* in FIG. 34) with NA11922 DNA input. For example, if the AA allele is detected, then the AB and BB alleles are not measurable. Oligo sequences (FIGS. 33A-33C) were designed to hybridize to these probes and to enable the detection of the AB genotype. In addition, three classes of probes were selected to demonstrate how synthetic oligos performed: accurate, inaccurate, and uncertain. Accurate probes were those where concordance is consistent with the 1000 genome next generation sequencing (NGS) standards. Inaccurate probes were those with call rates that do not match the 1000 genome NGS standards. Uncertain probes were those for which there was not sufficient data with the standard WGA material to generate the signal needed to assign the probe into the accurate or inaccurate probe class. Synthetic oligos (FIGS. 33A-33C) were designed against these three classes of probes.

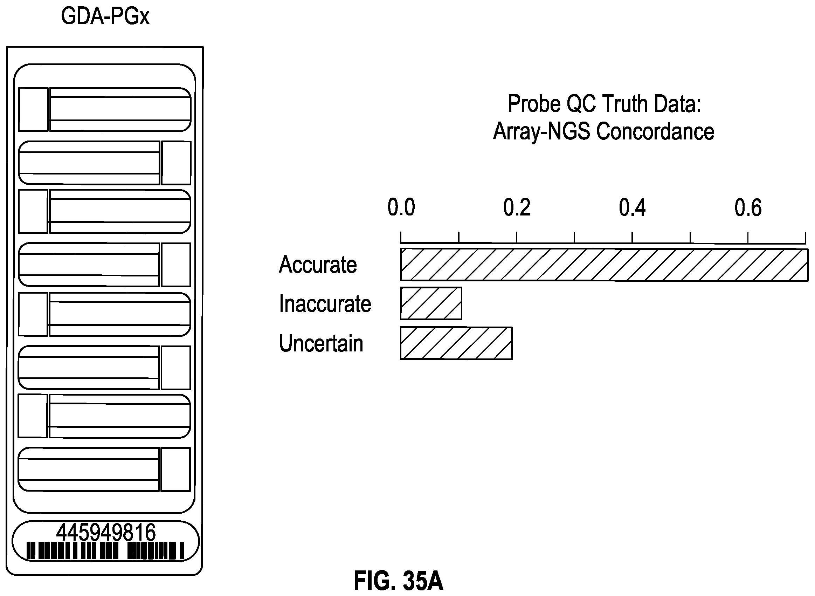

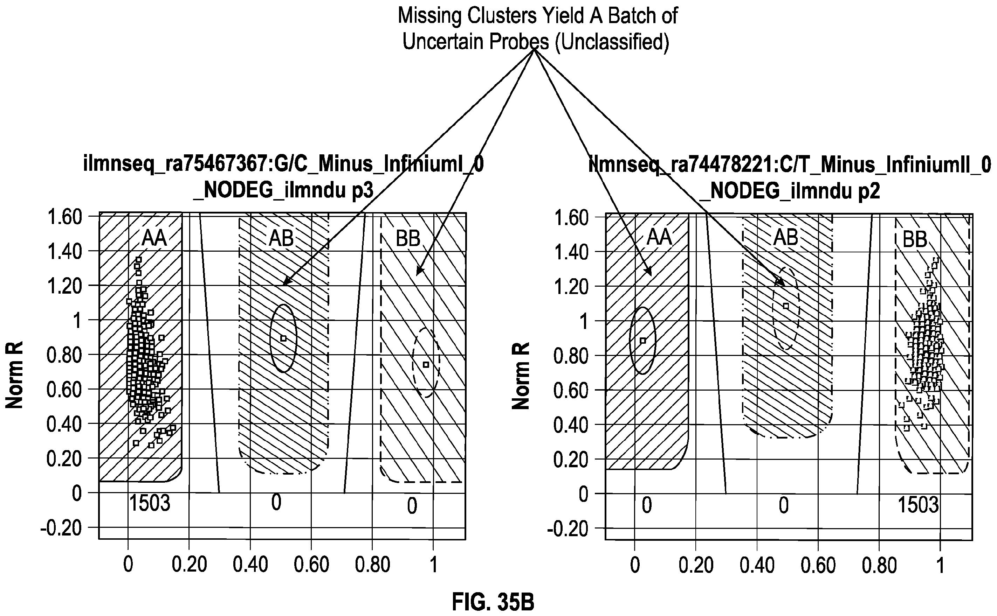

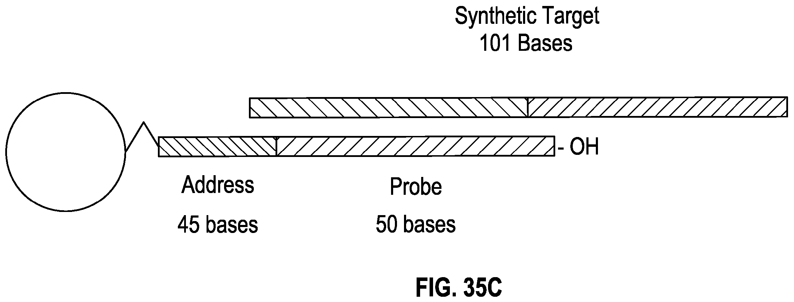

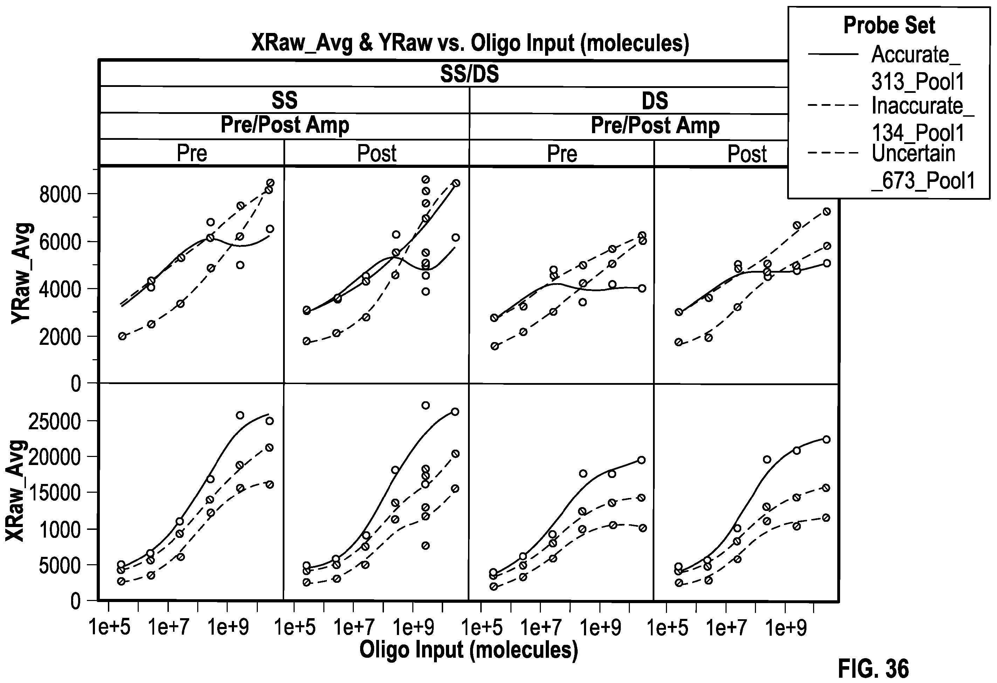

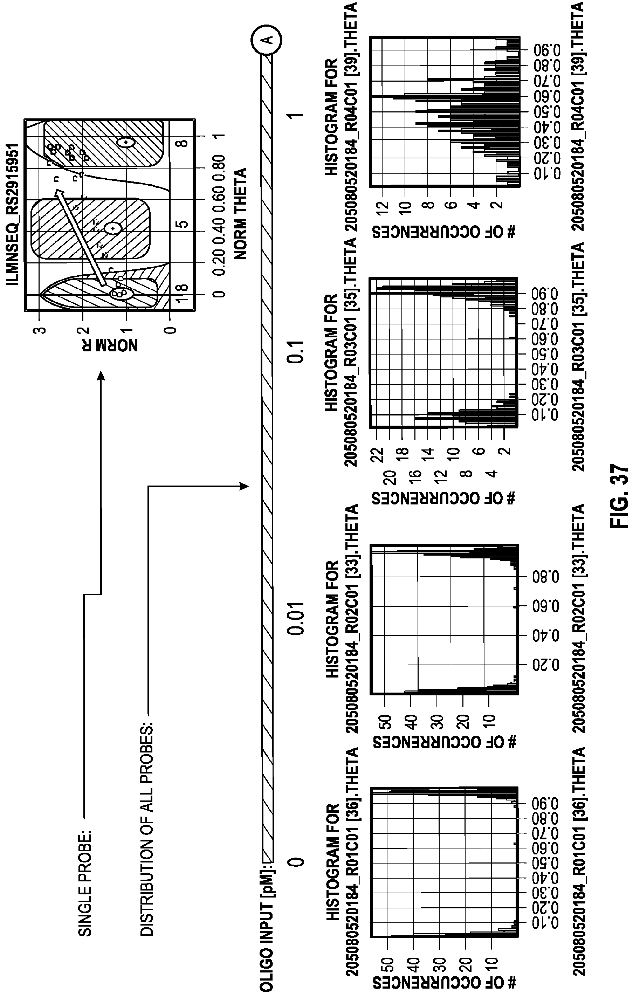

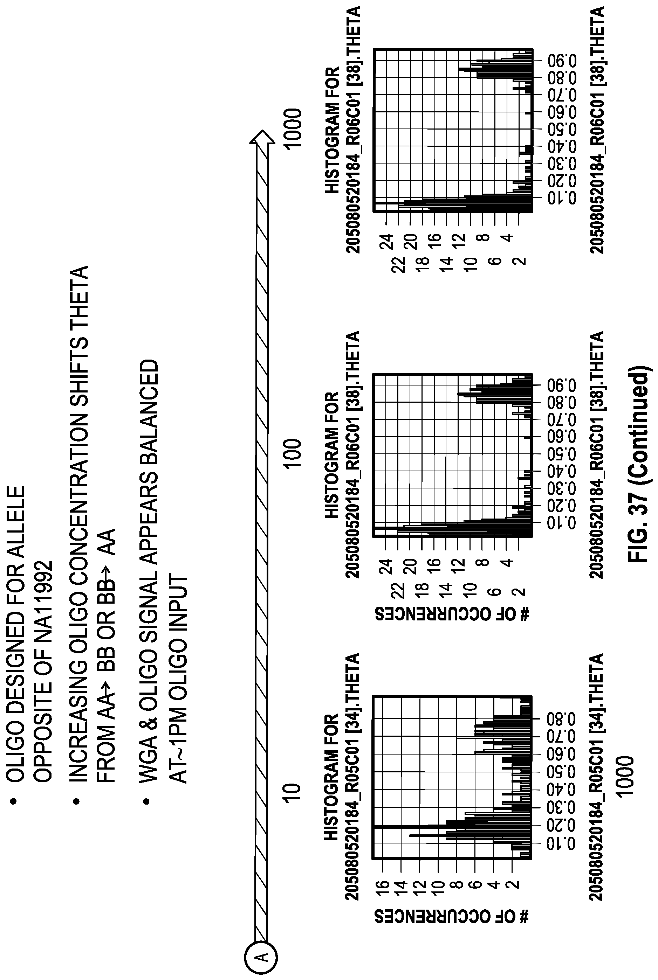

FIGS. 35A-35C schematically illustrate an example synthetic model system that was used to evaluate detection of synthetic oligonucleotides. More specifically, FIG. 35A illustrates the GDA-PGx beadchip and probe QC truth data for the three probe classes. The fluorescence intensity response curves for the three probe classes are shown in FIG. 35B. In FIG. 35B, probes classified as "accurate" have samples for all allele types (AA, AB, BB) enabled gentrain validation and have a concordant genotype NGS data. Inaccurate probe signals are not concordant with NGS. Uncertain probes are not categorized due to very low Minor Allele Frequency (MAF). These probes produce signals that are either AA or BB alleles. Uncertain class probe performance with synthetic targets was measured and compared against an artificial intelligence (AI) model prediction. Alleles with corresponding fluorescent signal: AA = red, BB = green, AB = red/green. FIG. 35C illustrates synthetic oligos (101 nt) designed to model synthetic targets binding to probes conjugated to bead immobilized on a beadchip. FIG. 36 illustrates fluorescence measured during use of the example synthetic model system of FIGS. 34 and 35A-35C. FIG. 37 illustrates the results of additional measurements made during use of the example synthetic model system of FIGS. 34 and 35A-35C.

The synthetic oligos were either spiked-in to the WGA reaction pre- or post-incubation. The pre-incubation steps provided the opportunity for the synthetic oligos to be amplified during the WGA step with randomers. Synthetic oligos added post WGA incubation did not undergo further amplification or fragmentation. A titration series was performed with both pre- and post-incubation formats. The final oligo concentrations were: 0 pM, 0.003 pM, 0.03 pM, 0.3 pM, 3 pM, 30 pM, and 300 pM. In FIG. 35B and FIG. 36, the Xraw and Yraw values correspond to the red and green signal from their respective channel. Across all conditions, the signal increased with increasing synthetic target concentration. The signal also increased with increasing probe concentration.

The probes were designed to demonstrate the homozygous allele signal (AA or BB) can be converted to a heterozygous allele (AB) signal with a balanced input amount of synthetic DNA (FIG. 34). WGA amplified material provided the background homozygous allele (AA or BB) and the synthetic probe introduced the opposite allele to generate a heterozygous AB allele. Heterozygous AB alleles were detected with approximately 0.3 pM of synthetic oligo input (FIG. 35B and FIG. 36). The 3 pM input corresponds to approximately 1-10 M molecules per sample well, which is consistent with the amount of input generated from genomic DNA after the WGA step per SNP. Furthermore, each sample well contained about 12 beads per probe type, and each contained approximately 60K oligos per bead. That suggested that the amount of SNP synthetic target needed for detection is approximately 10-fold excess of the amount of probe present. Increasing the amount of synthetic probe above about 3 pM led to a signal plateau and shifted the allele to homozygous (AA or BB) as the synthetic target outcompeted the WGA SNP input. The synthetic oligo concentration needed for detection (about 0.3 pM) was about 10,000,000-fold lower concentration than what was synthesized, and about 1,000,000-fold lower than what was required for standard PCR reaction (about 0.1-0.5 µM).

An application that extends beyond protein detection is to use microarrays to perform quality control (QC) on probe mixtures that are required for PCR or targeted enrichment application; high plexity PCR applications can extend up to >10K probes in a single formulation. Typical assay QC involves repeating the assay with multiple oligo pool lots to demonstrate failure modes are due to intrinsic target tissue and to rule out missing oligos. Using microarrays may mitigate the need to repeat PCR multiplex assays, which can be expensive and time-consuming.

### Additional comments

The practice of the present disclosure may employ, unless otherwise indicated, conventional techniques of molecular biology (including recombinant techniques), microbiology, cell biology, biochemistry, and immunology, which are within the skill of the art. Such techniques are explained fully in the literature, such as, Molecular Cloning: A Laboratory Manual, 2nd ed. (Sambrook et al., 1989); Oligonucleotide Synthesis (M. J. Gait, ed., 1984); Animal Cell Culture (R. I. Freshney, ed., 1987); Methods in Enzymology (Academic Press, Inc.); Current Protocols in Molecular Biology (F. M. Ausubel et al., eds., 1987, and periodic updates); PCR: The Polymerase Chain Reaction (Mullis et al., eds., 1994); Remington, The Science and Practice of Pharmacy, 20th ed., (Lippincott, Williams & Wilkins 2003), and Remington, The Science and Practice of Pharmacy, 22th ed., (Pharmaceutical Press and Philadelphia College of Pharmacy at University of the Sciences 2012).

All publications, patents, and patent applications mentioned in this specification are herein incorporated by reference to the same extent as if each individual publication, patent, or patent application was specifically and individually indicated to be incorporated by reference.

While various illustrative examples are described above, it will be apparent to one skilled in the art that various changes and modifications may be made therein without departing from the invention. The appended claims are intended to cover all such changes and modifications that fall within the true spirit and scope of the invention.

It is to be understood that any respective features/examples of each of the aspects of the disclosure as described herein may be implemented together in any appropriate combination, and that any features/examples from any one or more of these aspects may be implemented together with any of the features of the other aspect(s) as described herein in any appropriate combination to achieve the benefits as described herein.
The invention can additionally be described in the following clauses:
1. A method for detecting an analyte, the method comprising:
   coupling a donor recognition probe to a first portion of the analyte, the donor recognition probe comprising a first recognition element specific to the first portion of the analyte, a first oligonucleotide corresponding to the first portion of the analyte, and a transposase coupled to the first recognition element and the first oligonucleotide;
   coupling an acceptor recognition probe to a second portion of the analyte, the acceptor recognition probe comprising a second recognition element specific to the second portion of the analyte and a second oligonucleotide coupled to the second recognition element and corresponding to the second portion of the analyte;
   using the transposase to generate a reporter polynucleotide comprising the first and second oligonucleotides; and
   detecting the analyte based on the reporter polynucleotide comprising the first and second oligonucleotides.
2. The method of clause 1, wherein the analyte comprises a first molecule.
3. The method of clause 2, wherein the first portion of the analyte comprises a first portion of the first molecule, and wherein the second portion of the analyte comprises a second portion of the first molecule.
4. The method of clause 2, wherein:
   the first molecule comprises a protein or peptide;
   the first recognition element comprises a first antibody or a first aptamer that is specific to a first portion of the protein or peptide; and
   the second recognition element comprises a second antibody or a second aptamer that is specific to a second portion of the protein or peptide.
5. The method of clause 2, wherein:
   the first molecule comprises a target polynucleotide;
   the first recognition element comprises a first CRISPR-associated (Cas) protein that is specific to a first subsequence of the target polynucleotide; and
   the second recognition element comprises a second Cas protein that is specific to a second subsequence of the target polynucleotide.
6. The method of clause 5, wherein the target polynucleotide comprises RNA, and wherein the first and second Cas proteins independently are selected from the group consisting of rCas9 and dCas13.
7. The method of clause 2, wherein:
   the first molecule comprises a carbohydrate;
   the first recognition element comprises a first lectin that is specific to a first portion of the carbohydrate; and
   the second recognition element comprises a second lectin that is specific to a second portion of the carbohydrate.
8. The method of clause 2, wherein:
   the first molecule comprises a biomolecule;
   wherein the biomolecule is specific for the first and second recognition elements.
9. The method of clause 2, wherein the analyte further comprises a second molecule interacting with the first molecule.
10. The method of clause 9, wherein the first portion of the analyte comprises the first molecule, and wherein the second portion of the analyte comprises the second molecule.
11. The method of clause 10, wherein:
   the first molecule comprises a first protein or first peptide; and
   the first recognition element comprises a first antibody or a first aptamer that is specific to the first protein or first peptide.
12. The method of clause 10, wherein:
   the first molecule comprises a first target polynucleotide; and
   the first recognition element comprises a first CRISPR-associated (Cas) protein that is specific to the first target polynucleotide.
13. The method of clause 10, wherein:
   the first molecule comprises a first carbohydrate; and
   the first recognition element comprises a first lectin that is specific to the first carbohydrate.
14. The method of clause 10, wherein:
   the first molecule comprises a first biomolecule that is specific for the first recognition element.
15. The method of any one of clauses 11 to 14, wherein:
   the second molecule comprises a second protein or second peptide; and
   the second recognition element comprises a second antibody or a second aptamer that is specific to the second protein or second peptide.
16. The method of any one of clauses 11 to 14, wherein:
   the second molecule comprises a second target polynucleotide; and
   the second recognition element comprises a second Cas protein that is specific to the second target polynucleotide.
17. The method of any one of clauses 11 to 14, wherein:
   the second molecule comprises a second carbohydrate; and
   the second recognition element comprises a second lectin that is specific to the second carbohydrate.
18. The method of any one of clauses 9 to 14, wherein:
   the second molecule comprises a second biomolecule that is capable of interacting with the second recognition element.
19. The method of clause 18, wherein the second biomolecule is specific for the second recognition element.
20. The method of any one of clauses 1 to 19, wherein a portion of the second oligonucleotide comprises a double-stranded polynucleotide to which the transposase tagments the first oligonucleotide to generate the reporter polynucleotide.
21. The method of any one of clauses 1 to 20, wherein the first oligonucleotide comprises a first barcode corresponding to the first portion of the analyte, and wherein the second oligonucleotide comprises a second barcode corresponding to the second portion of the analyte.
22. The method of any one of clauses 1 to 21, wherein the first oligonucleotide comprises a mosaic end (ME) transposon end to which the transposase is coupled.
23. The method of any one of clauses 1 to 22, wherein the first oligonucleotide has a different sequence than the second oligonucleotide.
24. The method of any one of clauses 1 to 23, wherein the first oligonucleotide comprises a forward primer binding site, and wherein the second oligonucleotide comprises a reverse primer binding site.
25. The method of any one of clauses 1 to 24, further comprising inhibiting activity of the transposase while specifically coupling the donor recognition probe to the first portion of the analyte and while specifically coupling the acceptor recognition probe to the second portion of the analyte.
26. The method of clause 25, wherein the activity of the transposase is inhibited using a first condition of a fluid.
27. The method of clause 26, wherein the first condition of the fluid comprises at least one of (i) presence of a sufficient amount of EDTA to inhibit activity of the transposase and (ii) absence of a sufficient amount of magnesium ions for activity of the transposase.
28. The method of clause 25, wherein the activity of the transposase is inhibited using a dsDNA quencher.
29. The method of clause 25, wherein the activity of the transposase is inhibited by associating a blocker with the transposase.
30. The method of clause 25, wherein the activity of the transposase is inhibited by the second oligonucleotide being single stranded.
31. The method of any one of clauses 25 to 30, further comprising promoting activity of the transposase before using the transposase to generate the reporter polynucleotide.
32. The method of clause 31, wherein the activity of the transposase is promoted using a second condition of the fluid.
33. The method of clause 32, wherein the second condition of the fluid comprises presence of a sufficient amount of magnesium ions for activity of the transposase.
34. The method of clause 29, wherein the activity of the transposase is promoted by degrading the blocker.
35. The method of clause 31, wherein the activity of the transposase is promoted by annealing a third oligonucleotide to the second oligonucleotide to form a double-stranded polynucleotide.
36. The method of clause 25, wherein the activity of the transposase is inhibited using a blocking group coupled to the first oligonucleotide.
37. The method of clause 36, further comprising removing the blocking group using a reagent.
38. The method of any one of clauses 1 to 37, wherein detecting the analyte comprises sequencing the reporter polynucleotide.
39. The method of clause 38, wherein the sequencing comprises performing sequencing-by-synthesis on the reporter polynucleotide.
40. The method of any one of clauses 1 to 39, wherein detecting the analyte comprises:
   attaching the reporter polynucleotide to a bead,
   hybridizing a detector probe to the reporter polynucleotide, the detector probe comprising a fluorophore, and
   detecting a signal emitted by the fluorophore.
41. The method of clause 40, wherein the bead comprises a capture probe, and
   wherein the capture probe hybridizes to the reporter polynucleotide.
42. The method of any one of clauses 1 to 41, wherein the transposase is coupled to the first recognition element via the first oligonucleotide.
43. The method of any one of clauses 1 to 42, wherein the donor recognition probe comprises two transposases, two first recognition elements, and two first oligonucleotides, wherein the two transposases form a dimer, each of the transposases being coupled to a corresponding one of the first recognition elements via a corresponding one of the first oligonucleotides.
44. The method of any one of clauses 1 to 42, wherein the donor recognition probe comprises two transposases, one first recognition element, and two first oligonucleotides, wherein the two transposases form a dimer, each of the transposases being coupled to the one first recognition element via a corresponding one of the first oligonucleotides.
45. The method of any one of clauses 1 to 42, wherein the donor recognition probe comprises two transposases, one first recognition element, and two first oligonucleotides, wherein the two transposases form a dimer, at least one of the transposases being coupled to the one first recognition element via a covalent linkage.
46. The method of any one of clauses 1 to 45, wherein the first and second oligonucleotides comprise DNA.
47. The method of any one of clauses 1 to 46, wherein the first and second oligonucleotides each comprise a unique molecular identifier.
48. The method of any one of clauses 1 to 47, wherein the transposase comprises Tn5.
49. The method of any one of clauses 1 to 48, wherein the acceptor recognition probe is coupled to a bead before the acceptor recognition probe is coupled to the second portion of the analyte, the method further comprising washing the bead after the acceptor recognition probe is coupled to the second portion of the analyte and before the donor recognition probe is coupled to the first portion of the analyte.
50. The method of any one of clauses 1 to 49, wherein the first recognition element and the first oligonucleotide are coupled to the first portion of the analyte before the transposase is coupled to the first oligonucleotide and the first recognition element.
51. A method for detecting different analytes in a mixture, the method comprising:
   coupling different analytes in a mixture to respective donor recognition probes, each of the donor recognition probes comprising a first recognition element specific to a first portion of the respective analyte, a first oligonucleotide corresponding to the first portion of that analyte, and a transposase coupled to the first recognition element and the first oligonucleotide;
   coupling different analytes in the mixture to respective acceptor recognition probes, each of the acceptor recognition probes comprising a second recognition element specific to a second portion of the respective analyte, and a second oligonucleotide corresponding to the second portion of that analyte and coupled to the second recognition element;
   for each of the analytes coupled to the respective donor recognition probe and to the respective acceptor recognition probe, using the transposase of that donor recognition probe to generate a reporter polynucleotide comprising the first and second oligonucleotides corresponding to that analyte; and
   detecting the analytes in the mixture based on the reporter polynucleotides comprising the first and second oligonucleotides corresponding to those analytes.
52. The method of clause 51, further comprising determining amounts of the detected analytes in the mixture based on amounts of the reporter polynucleotides corresponding to those analytes.
53. The method of clause 51 or clause 52, wherein, for a first one of the analytes, a first one of the donor recognition probes is specific to a first form of the first portion of that analyte.
54. The method of clause 53, wherein, for the first one of the analytes, a second one of the donor recognition probes is specific to a second form of the first portion of that analyte.
55. The method of clause 54, wherein the first and second ones of the donor recognition probes are mixed with the analytes concurrently with one another.
56. The method of clause 53, wherein, for the first one of the analytes, a second one of the donor recognition probes is specific to both the first form and to a second form of the first portion of that analyte.
57. The method of clause 56, wherein the second one of the donor recognition probes is mixed with the analytes after the first one of the donor recognition probes is mixed with the analytes.
58. The method of any one of clauses 54 to 57, wherein the analyte is a protein, wherein the first form is post-translationally modified (PTM), and wherein the second form is not PTM.
59. The method of clause 58, wherein the first form is phosphorylated, acetylated, methylated, nitrosylated, or glycosylated relative to the second form.
60. The method of any one of clauses 51 to 57, wherein the analyte is a nucleic acid, wherein the first form includes a modified nucleotide, and wherein the second form does not include a modified nucleotide.
61. The method of any one of clauses 51 to 60, further comprising determining amounts of the first and second forms of the first one of the analytes based on amounts of the reporter polynucleotides corresponding to the first and second ones of the donor recognition probes.
62. A composition, comprising:
   an analyte having first and second portions;
   a donor recognition probe coupled to the first portion of the analyte, the donor recognition probe comprising a first recognition element specific to the first portion of the analyte, a first oligonucleotide corresponding to the first portion of the analyte, and a transposase coupled to the first recognition element and the first oligonucleotide; and
   an acceptor recognition probe coupled to the second portion of the analyte, the acceptor recognition probe comprising a second recognition element specific to the second portion of the analyte and a second oligonucleotide coupled to the second recognition element and corresponding to the second portion of the analyte.
63. A kit, comprising:
   a plurality of donor recognition probes, each comprising a recognition element specific to a first portion of a respective analyte, a first oligonucleotide corresponding to the first portion of that respective analyte, and a transposase coupled to the first recognition element and the first oligonucleotide; and
   a plurality of acceptor recognition probes, each comprising a recognition element specific to a second portion of a respective analyte and a second polynucleotide coupled to the second recognition element and corresponding to the second portion of that respective analyte.
64. A method for detecting an analyte, the method comprising:
   coupling a donor recognition probe to a first portion of the analyte, the donor recognition probe comprising a first oligonucleotide corresponding to the first portion of the analyte and a transposase coupled to the first oligonucleotide;
   coupling an acceptor recognition probe to a second portion of the analyte, the acceptor recognition probe comprising a second oligonucleotide corresponding to the second portion of the analyte;
   using the transposase to generate a reporter polynucleotide comprising the first and second oligonucleotides; and
   detecting the analyte based on the reporter polynucleotide comprising the first and second oligonucleotides.
65. The method of clause 64, wherein the donor recognition probe is coupled to the first portion of the analyte via a covalent linkage, and wherein the acceptor recognition probe is coupled to the second portion of the analyte via a covalent linkage.
66. A method for detecting an analyte, the method comprising:
   coupling a first recognition probe to a first portion of the analyte, the first recognition probe comprising a first recognition element specific to the first portion of the analyte and a first oligonucleotide corresponding to the first portion of the analyte;
   coupling a second recognition probe to a second portion of the analyte, the second recognition probe comprising a second recognition element specific for the second portion of the analyte and a second oligonucleotide corresponding to the second portion of the analyte;
   coupling the first oligonucleotide to the second oligonucleotide using a splint oligonucleotide that has complementarity to both a portion of the first oligonucleotide and a portion of the second oligonucleotide to form a reporter oligonucleotide coupled to the first and second recognition probes;
   performing a sequence analysis of the reporter oligonucleotide; and
   detecting the analyte based on the sequence analysis of the reporter oligonucleotide.
67. The method of clause 66, further comprising:
   generating a double-stranded oligonucleotide comprising the reporter oligonucleotide coupled to the first and second recognition probes, and a complementary oligonucleotide hybridized to the reporter oligonucleotide.
68. The method of clause 67, further comprising excising a portion of the double-stranded oligonucleotide, wherein the sequence analysis is performed on the excised portion of the double-stranded oligonucleotide.
69. The method of clause 68, wherein the sequence analysis that is performed comprises any one or more of isothermal bead-based amplification, targeted genome amplification, and whole genome amplification.
70. The method of clause 66, wherein the first recognition probe or the second recognition probe comprises an antibody, a lectin, or an aptamer.
71. The method of clause 66, wherein the first recognition probe comprises a first antibody, a first lectin, or a first aptamer.
72. The method of clause 66, wherein the second recognition probe comprises a second antibody, a second lectin, or a second aptamer.
73. The method of clause 66, wherein the first oligonucleotide comprises a partial barcode, and the second oligonucleotide comprises a partial barcode, wherein coupling the first oligonucleotide to the second oligonucleotide results in a complete barcode that corresponds to the target analyte.
74. The method of clause 66, wherein performing the sequence analysis comprises performing a polymerase chain reaction (PCR) on the reporter oligonucleotide.
75. The method of clause 66, wherein the reporter oligonucleotide comprises a unique molecular identifier (UMI) that is amplified during the PCR.
76. A method for detecting a plurality of analytes in a sample, the method comprising:
   incubating the sample with:
      a plurality of pairs of recognition probes,
         wherein each pair of recognition probes comprises a first recognition probe and a second recognition probe,
         wherein each pair of recognition probes is specific for a respective one of the analytes, and
         wherein each first recognition probe and each second recognition probe are coupled to a respective oligonucleotide; and
      a plurality of splint oligonucleotides,
         wherein each splint oligonucleotide is complementary to portions of oligonucleotides that respectively are coupled to a first recognition probe and a second recognition probe of a pair of recognition probes which is specific to a respective one of the analytes, and
         wherein complementary binding of each splint oligonucleotide to oligonucleotides that are coupled to first recognition probes and second recognition probes results in formation of reporter oligonucleotides;
   washing the sample to remove any unbound recognition probes and any unbound splint oligonucleotides;
   performing a sequence analysis of the reporter oligonucleotides; and
   detecting the plurality of analytes based on the sequence analysis.
77. The method of clause 76, wherein incubating the sample further comprises incubation with a ligase.
78. The method of clause 76, wherein performing the sequence analysis comprises using any one or more of a microarray, a bead array, library preparation, or PCR.
79. A composition, comprising:
   a plurality of analytes;
   a plurality of pairs of recognition probes,
      wherein each pair of recognition probes comprises a first recognition probe and second recognition probe,
      wherein each pair of recognition probes is specific for a respective one of the analytes, and
      wherein each first recognition probe and each second recognition probe are coupled to a respective oligonucleotide;
      and
   a plurality of splint oligonucleotides,
      wherein each splint oligonucleotide is complementary to portions of oligonucleotides that respectively are coupled to a first recognition probe and a second recognition probe of a pair of recognition probes which is specific to a respective one of the analytes.
80. A kit, comprising;
   a plurality of pairs of recognition probes,
      wherein each pair of recognition probes comprises a first recognition probe and second recognition probe,
      wherein each pair of recognition probes is specific for a respective one of the analytes, and
      wherein each first recognition probe and each second recognition probe are coupled to a respective oligonucleotide;
      and
   a plurality of splint oligonucleotides,
      wherein each splint oligonucleotide is complementary to portions of oligonucleotides that respectively are coupled to a first recognition probe and a second recognition probe of a pair of recognition probes which is specific to a respective one of the analytes.
81. A method for detecting an analyte, the method comprising:
   coupling a first recognition probe to a first portion of the analyte, the first recognition probe comprising a first recognition element specific to the first portion of the analyte and a double-stranded oligonucleotide comprising a first barcode corresponding to the first portion of the analyte;
   coupling a second recognition probe to a second portion of the analyte, the second recognition probe comprising a second recognition element specific for the second portion of the analyte and a single-stranded oligonucleotide comprising a second barcode corresponding to the second portion of the analyte;
   hybridizing the single-stranded oligonucleotide with a single oligonucleotide strand of the double-stranded oligonucleotide to form a reporter oligonucleotide comprising the first barcode and the second barcode;
   performing a sequence analysis of the reporter oligonucleotide; and
   detecting the analyte based on the sequence analysis of the reporter oligonucleotide.
82. The method of clause 81, wherein the hybridizing step comprises strand invasion of the double-stranded oligonucleotide by the single-stranded oligonucleotide.
83. The method of clause 81, wherein the sequence analysis that is performed comprises any one or more of isothermal bead-based amplification, targeted genome amplification, and whole genome amplification.
84. The method of clause 81, wherein detecting the analyte comprises performing quantitative detection of the reporter oligonucleotide.
85. A method for detecting an analyte, the method comprising:
   coupling a first recognition probe to a first portion of the analyte, the first recognition probe comprising a first recognition element specific to the first portion of the analyte and a first oligonucleotide corresponding to the first portion of the analyte, wherein the first oligonucleotide comprises a first restriction endonuclease site;
   coupling a second recognition probe to a second portion of the analyte, the second recognition probe comprising a second recognition element specific for the second portion of the analyte and a second oligonucleotide corresponding to the second portion of the analyte, wherein the second oligonucleotide comprises a second restriction endonuclease site;
   coupling the first oligonucleotide to the second oligonucleotide;
   cutting the first oligonucleotide and the second oligonucleotide at the first and second restriction endonuclease sites to form a reporter oligonucleotide;
   performing a sequence analysis of the reporter oligonucleotide; and
   detecting the analyte based on the sequence analysis of the reporter oligonucleotide.
86. The method of clause 85, wherein the cutting step comprises using one or more restriction endonucleases.
87. The method of clause 85, wherein the sequence analysis that is performed comprises any one or more of isothermal bead-based amplification, targeted genome amplification, and whole genome amplification.
88. The method of clause 85, wherein detecting the analyte comprises performing quantitative detection of the reporter oligonucleotide.
89. A method of performing a targeted epigenetic assay, the method comprising:
   contacting a polynucleotide with a mixture of first complexes that are specific to different types of proteins coupled to respective loci of the polynucleotide,
   each of the first complexes comprising a first antibody that is specific to a corresponding type of protein, and a first transposome coupled to the first antibody and including a first oligonucleotide corresponding to that type of protein;
   respectively coupling the first complexes to proteins for which the first antibodies are specific;
   generating fragments of the polynucleotide, comprising activating the first transposomes to make first cuts in the polynucleotide and to couple the first oligonucleotides to the first cuts;
   removing the proteins and first complexes from the fragments;
   subsequently sequencing the fragments and the first oligonucleotides coupled thereto; and
   identifying the proteins that had been coupled to the fragments using the sequences of the first oligonucleotides coupled to those fragments.
90. The method of clause 89, wherein each of the first complexes comprises a plurality of first transposomes.
91. The method of clause 90, wherein each of the first complexes comprises two first transposomes.
92. The method of any one of clauses 89 to 91, wherein the first transposomes are deactivated using a first condition of a fluid.
93. The method of clause 92, wherein the first condition of the fluid comprises at least one of (i) presence of a sufficient amount of EDTA to inhibit activity of the first transposomes and (ii) absence of a sufficient amount of magnesium ions for activity of the first transposomes.
94. The method of clause 92 or clause 93, wherein the first transposomes are activated using a second condition of the fluid.
95. The method of clause 94, wherein the second condition of the fluid comprises presence of a sufficient amount of magnesium ions for activity of the first transposomes.
96. The method of any one of clauses 89 to 95, wherein the sequencing comprises performing sequencing-by-synthesis on the fragments and the oligonucleotides coupled thereto.
97. The method of any one of clauses 89 to 96, comprising using respective locations in the fragments of the first oligonucleotides to identify the respective loci of the proteins.
98. The method of any one of clauses 89 to 97, wherein the first oligonucleotides comprise primers.
99. The method of any one of clauses 89 to 98, wherein the first oligonucleotides comprise unique molecular identifiers (UMIs).
100. The method of any one of clauses 89 to 99, wherein the first oligonucleotides comprise barcodes corresponding to the proteins.
101. The method of any one of clauses 89 to 100, wherein the first oligonucleotides comprise mosaic end (ME) transposon ends.
102. The method of any one of clauses 89 to 101, wherein the first transposomes are coupled to the first antibodies via covalent linkages.
103. The method of any one of clauses 89 to 101, wherein the first transposomes are coupled to the first antibodies via non-covalent linkages.
104. The method of clause 103, wherein the first transposomes are coupled to protein A, and wherein active sites of the first antibodies are coupled to the protein A.
105. The method of any one of clauses 89 to 104, wherein the first transposomes comprise Tn5.
106. The method of any one of clauses 89 to 105, wherein each of the first complexes comprises a fusion protein comprising the first antibody and the first transposome.
107. The method of any one of clauses 89 to 106, wherein the first antibody is coupled to the first oligonucleotide, and wherein the first transposome is coupled to the first antibody via the first oligonucleotide.
108. The method of any one of clauses 89 to 107, further comprising:
   contacting the polynucleotide with a mixture of second complexes that are specific to the first complexes,
   each of the second complexes comprising a second antibody that is specific to the first antibodies, and a second transposome coupled to the second antibody and including a second oligonucleotide; and
   respectively coupling the second complexes to the first complexes;
   wherein generating fragments of the polynucleotide further comprises activating the second transposomes to make second cuts in the polynucleotide and to couple the second oligonucleotides to the second cuts; and
   wherein the second oligonucleotides are used to amplify the fragments prior to sequencing.
109. The method of any one of clauses 89 to 108, wherein the polynucleotide comprises double-stranded DNA.
110. A composition, comprising:
   a polynucleotide, having different types of proteins coupled to respective loci thereof; and
   a mixture of first complexes that are specific to different types of the proteins,
   each of the first complexes comprising a first antibody selective for a type of protein, and a first transposome coupled to the first antibody and including a first oligonucleotide corresponding to that type of protein.
111. The composition of clause 110, wherein each of the first complexes comprises a plurality of first transposomes.
112. The composition of clause 111, wherein each of the first complexes comprises two first transposomes.
113. The composition of any one of clauses 110 to 112, wherein the first transposomes are deactivated using a condition of a fluid.
114. The composition of clause 113, wherein the condition of the fluid comprises at least one of (i) presence of a sufficient amount of EDTA to inhibit activity of the first transposomes and (ii) absence of a sufficient amount of magnesium ions for activity of the first transposomes.
115. The composition of any one of clauses 110 to 114, wherein the first transposomes are activatible to cut the polynucleotide and add the first oligonucleotides to the cuts.
116. The composition of clause 115, wherein the first transposomes are activatible using a condition of a fluid.
117. The composition of clause 116, wherein the condition of the fluid comprises presence of a sufficient amount of magnesium ions for activity of the first transposomes.
118. The composition of any one of clauses 110 to 117, wherein the first oligonucleotides comprise primers.
119. The composition of any one of clauses 110 to 118, wherein the first oligonucleotides comprise unique molecular identifiers (UMIs).
120. The composition of any one of clauses 110 to 119, wherein the first oligonucleotides comprise barcodes corresponding to the proteins.
121. The composition of any one of clauses 110 to 120, wherein the first oligonucleotides comprise mosaic end (ME) transposon ends.
122. The composition of any one of clauses 110 to 121, wherein the first transposomes are coupled to the antibodies via covalent linkages.
123. The composition of any one of clauses 110 to 122, wherein the first transposomes are coupled to the antibodies via non-covalent linkages.
124. The composition of clause 123, wherein the first transposomes are coupled to protein A, and wherein active sites of the first antibodies are coupled to the protein A.
125. The composition of any one of clauses 110 to 124, wherein the first transposomes comprise Tn5.
126. The composition of any one of clauses 110 to 125, wherein each of the first complexes comprises a fusion protein comprising the first antibody and the first transposome.
127. The composition of any one of clauses 110 to 126, wherein the first antibody is coupled to the first oligonucleotide, and wherein the first transposome is coupled to the first antibody via the first oligonucleotide.
128. The composition of any one of clauses 110 to 127, further comprising:
   a mixture of second complexes that are specific to the first complexes,
   each of the second complexes comprising a second antibody that is coupled to one of the first antibodies, and a second transposome including a second oligonucleotide.
129. The composition of any one of clauses 110 to 128, wherein the polynucleotide comprises double-stranded DNA.

## Claims

1. A method of performing a targeted epigenetic assay, the method comprising:
a. contacting a polynucleotide with a mixture of first complexes that are specific to different types of proteins coupled to respective loci of the polynucleotide,
b. each of the first complexes comprising a first antibody that is specific to a corresponding type of protein, and a first transposome coupled to the first antibody and including a first oligonucleotide corresponding to that type of protein;
c. respectively coupling the first complexes to proteins for which the first antibodies are specific;
d. generating fragments of the polynucleotide, comprising activating the first transposomes to make first cuts in the polynucleotide and to couple the first oligonucleotides to the first cuts;
e. removing the proteins and first complexes from the fragments;
f. subsequently sequencing the fragments and the first oligonucleotides coupled thereto; and
g. identifying the proteins that had been coupled to the fragments using the sequences of the first oligonucleotides coupled to those fragments.

2. The method of claim 1, wherein each of the first complexes comprises a plurality of first transposomes, preferably wherein each of the first complexes comprises two first transposomes.

3. The method of any preceding claim, wherein:
the first transposomes are deactivated using a first condition of a fluid, preferably wherein the first condition of the fluid comprises at least one of (i) presence of a sufficient amount of EDTA to inhibit activity of the first transposomes and (ii) absence of a sufficient amount of magnesium ions for activity of the first transposomes; and/or
the first transposomes are activated using a second condition of the fluid, preferably wherein the second condition of the fluid comprises presence of a sufficient amount of magnesium ions for activity of the first transposomes.

4. The method of any preceding claim, wherein:
the sequencing comprises performing sequencing-by-synthesis on the fragments and the oligonucleotides coupled thereto; and/or
the method comprises using respective locations in the fragments of the first oligonucleotides to identify the respective loci of the proteins.

5. The method of any preceding claim, wherein the first oligonucleotides comprise:
primers;
unique molecular identifiers (UMIs);
barcodes corresponding to the proteins;
mosaic end (ME) transposon ends; and/or
Tn5.

6. The method of any preceding claim, wherein:
the first transposomes are coupled to the first antibodies via covalent linkages;
the first transposomes are coupled to the first antibodies via non-covalent linkages, preferably wherein the first transposomes are coupled to protein A and wherein active sites of the first antibodies are coupled to the protein A;
each of the first complexes comprises a fusion protein comprising the first antibody and the first transposome; and/or
the first antibody is coupled to the first oligonucleotide, and wherein the first transposome is coupled to the first antibody via the first oligonucleotide.

7. The method of any one preceding claim, further comprising:
a. contacting the polynucleotide with a mixture of second complexes that are specific to the first complexes,
b. each of the second complexes comprising a second antibody that is specific to the first antibodies, and a second transposome coupled to the second antibody and including a second oligonucleotide; and
c. respectively coupling the second complexes to the first complexes;
d. wherein generating fragments of the polynucleotide further comprises activating the second transposomes to make second cuts in the polynucleotide and to couple the second oligonucleotides to the second cuts; and
e. wherein the second oligonucleotides are used to amplify the fragments prior to sequencing.

8. The method of any preceding claim, wherein the polynucleotide comprises double-stranded DNA.

9. A composition, comprising:
a. a polynucleotide, having different types of proteins coupled to respective loci thereof; and
b. a mixture of first complexes that are specific to different types of the proteins,
c. each of the first complexes comprising a first antibody selective for a type of protein, and a first transposome coupled to the first antibody and including a first oligonucleotide corresponding to that type of protein.

10. The composition of claim 9, wherein each of the first complexes comprises a plurality of first transposomes, preferably wherein each of the first complexes comprises two first transposomes.

11. The composition of claim 9 or 10, wherein:
the first transposomes are deactivated using a condition of a fluid, preferably wherein the condition of the fluid comprises at least one of (i) presence of a sufficient amount of EDTA to inhibit activity of the first transposomes and (ii) absence of a sufficient amount of magnesium ions for activity of the first transposomes; and/or
the first transposomes are activatible to cut the polynucleotide and add the first oligonucleotides to the cuts, preferably wherein the first transposomes are activatible using a condition of a fluid, preferably wherein the condition of the fluid comprises presence of a sufficient amount of magnesium ions for activity of the first transposomes.

12. The composition of any one of claims 9 to 11, wherein the first oligonucleotides comprise:
primers;
unique molecular identifiers (UMIs);
barcodes corresponding to the proteins;
mosaic end (ME) transposon ends; and/or
Tn5.

13. The composition of any one of claims 9 to 12, wherein:
the first transposomes are coupled to the antibodies via covalent linkages;
the first transposomes are coupled to the antibodies via non-covalent linkages, preferably wherein the first transposomes are coupled to protein A, and wherein active sites of the first antibodies are coupled to the protein A;
each of the first complexes comprises a fusion protein comprising the first antibody and the first transposome;
the first antibody is coupled to the first oligonucleotide, and wherein the first transposome is coupled to the first antibody via the first oligonucleotide.

14. The composition of any one of claims 9 to 13, further comprising:
a. a mixture of second complexes that are specific to the first complexes,
b. each of the second complexes comprising a second antibody that is coupled to one of the first antibodies, and a second transposome including a second oligonucleotide.

15. The composition of any one of claims 9 to 14, wherein the polynucleotide comprises double-stranded DNA.
